# EUROPEAN PATENT APPLICATION

(11) **EP 2 705 854 A1**
(43) Date of publication of application: **12.03.2014**
(21) Application number: 13168106.6
(22) Date of filing: 04.01.2007
(51) Int. Cl.: A61K 39/395, A61K 31/663, A61P 19/08

(54) **Methods for preventing and treating cancer metastasis and bone loss associated with cancer metastasis**

(30) Priority: 05.01.2006 US 756944 P
(62) Divisional of application: 07716432.5
(71) Applicant: Novartis AG, 4056 Basel (CH); XOMA Technology Ltd., Berkeley, CA 94710 (US)
(72) Inventor: Kavanaugh, Michael, Emeryville, CA 94662-8097 (US); Liu, Cheng, Richmond, CA 94803 (US)
(74) Representative: Carpmaels & Ransford LLP

(57) **Abstract**

Methods for preventing and treating osteolysis, cancer metastasis and bone loss associated with cancer metastasis by administering an M-CSF-antagonist in combination with a therapeutic agent to a subject are provided.

SEQUENCE LISTING

<110> Novartis AG; XOMA TECHNOLOGY LTD.

<120> METHODS FOR PREVENTING AND TREATING CANCER METASTASIS AND BONE LOSS
ASSOCIATED WITH CANCER METASTASIS

<130> P055365EP

<140> To be assigned
<141> 2007-01-04

<150> 60/756,944
<151> 2005-01-05

<160> 88

<170> PatentIn version 3.3

<210> 1
<211> 1401
<212> DNA
<213> Mus musculus

<400> 1
atgggttggt cctgtatcat cctattcctg gtggccactg ccacaggtgt gcactccgac 60

gtgcagcttc aggagtcagg acctggcctc gtgaaacctt ctcagagtct gtccctcacc 120

tgtactgtca ctgactactc catcaccagt gattacgcct ggaactggat acggcaattc 180

ccagggaata aacttgagtg gatggggtac ataagctaca gtggtagcac ttcctacaat 240

ccatctctca aaagtcggat ctccatcact cgagacacat ccaagaacca gttcttcctg 300

cagctgaact ctgtgactac tgaggacaca gccacatatt actgtgcatc cttcgactat 360

gcccacgcca tggattactg gggccaaggg acttcggtca ctgtctcttc cgccaaaaca 420

acagccccat cggtctatcc actggcccct gtgtgtggag atacaactgg ctcctcggtg 480

actctaggat gcctggtcaa gggttatttc cctgagccag tgaccttgac ctggaactct 540

ggatccctgt ccagtggtgt gcacaccttc ccagctgtcc tgcagtctga cctctacacc 600

ctcagcagct cagtgactgt aacctcgagc acctggccca gccagtccat cacctgcaat 660

gtggcccacc cggcaagcag caccaaggtg gacaagaaaa ttgagcccag agggcccaca 720

atcaagccct gtcctccatg caaatgccca gcacctaacc tcttgggtgg accatccgtc 780

ttcatcttcc ctccaaagat caaggatgta ctcatgatct ccctgagccc catagtcaca 840

tgtgtggtgg tggatgtgag cgaggatgac ccagatgtcc agatcagctg gtttgtgaac 900

aacgtggaag tacacacagc tcagacacaa acccatagag aggattacaa cagtactctc 960

cgggtggtca gtgccctccc catccagcac caggactgga tgagtggcaa ggagttcaaa 1020

tgcaaggtca acaacaaaga cctcccagcg cccatcgaga gaaccatctc aaaacccaaa 1080

gggtcagtaa gagctccaca ggtatatgtc ttgcctccac cagaagaaga gatgactaag 1140

aaacaggtca ctctgacctg catggtcaca gacttcatgc ctgaagacat ttacgtggag 1200

tggaccaaca acgggaaaac agagctaaac tacaagaaca ctgaaccagt cctggactct 1260

gatggttctt acttcatgta cagcaagctg agagtggaaa agaagaactg ggtggaaaga 1320

aatagctact cctgttcagt ggtccacgag ggtctgcaca atcaccacac gactaagagc 1380

ttctcccgga ctccgggtaa a 1401

<210> 2
<211> 447
<212> PRT
<213> Mus musculus

<400> 2

Asp Val Gln Leu Gln Glu Ser Gly Pro Gly Leu Val Lys Pro Ser Gln
1               5                   10                  15

Ser Leu Ser Leu Thr Cys Thr Val Thr Asp Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Ser Tyr Ser Gly Ser Thr Ser Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Ser Phe Asp Tyr Ala His Ala Met Asp Tyr Trp Gly Gln Gly Thr
            100                 105                 110

Ser Val Thr Val Ser Ser Ala Lys Thr Thr Ala Pro Ser Val Tyr Pro
        115                 120                 125

Leu Ala Pro Val Cys Gly Asp Thr Thr Gly Ser Ser Val Thr Leu Gly
    130                 135                 140

Cys Leu Val Lys Gly Tyr Phe Pro Glu Pro Val Thr Leu Thr Trp Asn
145                 150                 155                 160

Ser Gly Ser Leu Ser Ser Gly Val His Thr Phe Pro Ala Val Leu Gln
                165                 170                 175

Ser Asp Leu Tyr Thr Leu Ser Ser Ser Val Thr Val Thr Ser Ser Thr

            180                 185                 190

Trp Pro Ser Gln Ser Ile Thr Cys Asn Val Ala His Pro Ala Ser Ser
        195                 200                 205

Thr Lys Val Asp Lys Lys Ile Glu Pro Arg Gly Pro Thr Ile Lys Pro
    210                 215                 220

Cys Pro Pro Cys Lys Cys Pro Ala Pro Asn Leu Leu Gly Gly Pro Ser
225                 230                 235                 240

Val Phe Ile Phe Pro Pro Lys Ile Lys Asp Val Leu Met Ile Ser Leu
                245                 250                 255

Ser Pro Ile Val Thr Cys Val Val Val Asp Val Ser Glu Asp Asp Pro
            260                 265                 270

Asp Val Gln Ile Ser Trp Phe Val Asn Asn Val Glu Val His Thr Ala
        275                 280                 285

Gln Thr Gln Thr His Arg Glu Asp Tyr Asn Ser Thr Leu Arg Val Val
    290                 295                 300

Ser Ala Leu Pro Ile Gln His Gln Asp Trp Met Ser Gly Lys Glu Phe
305                 310                 315                 320

Lys Cys Lys Val Asn Asn Lys Asp Leu Pro Ala Pro Ile Glu Arg Thr
                325                 330                 335

Ile Ser Lys Pro Lys Gly Ser Val Arg Ala Pro Gln Val Tyr Val Leu
            340                 345                 350

Pro Pro Pro Glu Glu Glu Met Thr Lys Lys Gln Val Thr Leu Thr Cys
        355                 360                 365

Met Val Thr Asp Phe Met Pro Glu Asp Ile Tyr Val Glu Trp Thr Asn
    370                 375                 380

Asn Gly Lys Thr Glu Leu Asn Tyr Lys Asn Thr Glu Pro Val Leu Asp
385                 390                 395                 400

Ser Asp Gly Ser Tyr Phe Met Tyr Ser Lys Leu Arg Val Glu Lys Lys
                405                 410                 415

Asn Trp Val Glu Arg Asn Ser Tyr Ser Cys Ser Val Val His Glu Gly
            420                 425                 430

Leu His Asn His His Thr Thr Lys Ser Phe Ser Arg Thr Pro Gly
        435                 440                 445

<210>  3
<211>  702
<212>  DNA
<213>  Mus musculus

<400>  3
atggtatcca cacctcagtt ccttgtattt ttgcttttct ggattccagc ctccagaggt      60

gacatcttgc tgactcagtc tccagccatc ctgtctgtga gtccaggaga aagagtcagt     120

ttctcctgca gggccagtca gagcattggc acaagcatac actggtatca gcaaagaaca     180

aatggttctc caaggcttct cataaagtat gcttctgagt ctatctctgg gatcccttcc     240

aggtttagtg gcagtggatc agggacagat tttactctta gcatcaacag tgtggagtct     300

gaagatattg cagattatta ctgtcaacaa attaatagct ggccaaccac gttcggcggg     360

gggacaaagt tggaaataaa acgggctgat gctgcaccaa ctgtatccat cttcccacca     420

tccagtgagc agttaacatc tggaggtgcc tcagtcgtgt gcttcttgaa caacttctac     480

cccaaagaca tcaatgtcaa gtggaagatt gatggcagtg aacgacaaaa tggcgtcctg     540

aacagttgga ctgatcagga cagcaaagac agcacctaca gcatgagcag caccctcacg     600

ttgaccaagg acgagtatga acgacataac agctatacct gtgaggccac tcacaagaca     660

tcaacttcac ccattgtcaa gagcttcaac aggaatgagt gt                        702

<210>  4
<211>  214
<212>  PRT
<213>  Mus musculus

<400>  4

Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Ser Ile Gly Thr Ser
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Ile Asn Ser Trp Pro Thr

85                  90                  95

Thr Phe Gly Gly Gly Thr Lys Leu Glu Ile Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>  5
<211>  109
<212>  PRT
<213>  Mus musculus

<400>  5

Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Ser Ile Gly Thr Ser
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Ile Asn Ser Trp Pro Thr

85                  90                  95

Thr Phe Gly Gly Gly Thr Lys Leu Glu Ile Lys Arg Ala
            100                 105

<210>  6
<211>  118
<212>  PRT
<213>  Mus musculus

<400>  6

Asp Val Gln Leu Gln Glu Ser Gly Pro Gly Leu Val Lys Pro Ser Gln
1               5                   10                  15

Ser Leu Ser Leu Thr Cys Thr Val Thr Asp Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Ser Tyr Ser Gly Ser Thr Ser Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Ser Phe Asp Tyr Ala His Ala Met Asp Tyr Trp Gly Gln Gly Thr
            100                 105                 110

Ser Val Thr Val Ser Ser
        115

<210>  7
<211>  256
<212>  PRT
<213>  ?

<400>  7

Met Thr Ala Pro Gly Ala Ala Gly Arg Cys Pro Pro Thr Thr Trp Leu
1               5                   10                  15

Gly Ser Leu Leu Leu Leu Val Cys Leu Leu Ala Ser Arg Ser Ile Thr
            20                  25                  30

Glu Glu Val Ser Glu Tyr Cys Ser His Met Ile Gly Ser Gly His Leu
        35                  40                  45

Gln Ser Leu Gln Arg Leu Ile Asp Ser Gln Met Glu Thr Ser Cys Gln
    50                  55                  60

Ile Thr Phe Glu Phe Val Asp Gln Glu Gln Leu Lys Asp Pro Val Cys
65                  70                  75                  80

Tyr Leu Lys Lys Ala Phe Leu Leu Val Gln Asp Ile Met Glu Asp Thr
                85                  90                  95

Met Arg Phe Arg Asp Asn Thr Pro Asn Ala Ile Ala Ile Val Gln Leu
            100                 105                 110

Gln Glu Leu Ser Leu Arg Leu Lys Ser Cys Phe Thr Lys Asp Tyr Glu
        115                 120                 125

Glu His Asp Lys Ala Cys Val Arg Thr Phe Tyr Glu Thr Pro Leu Gln
    130                 135                 140

Leu Leu Glu Lys Val Lys Asn Val Phe Asn Glu Thr Lys Asn Leu Leu
145                 150                 155                 160

Asp Lys Asp Trp Asn Ile Phe Ser Lys Asn Cys Asn Asn Ser Phe Ala
                165                 170                 175

Glu Cys Ser Ser Gln Gly His Glu Arg Gln Ser Glu Gly Ser Ser Ser
            180                 185                 190

Pro Gln Leu Gln Glu Ser Val Phe His Leu Leu Val Pro Ser Val Ile
        195                 200                 205

Leu Val Leu Leu Ala Val Gly Gly Leu Leu Phe Tyr Arg Trp Arg Arg
    210                 215                 220

Arg Ser His Gln Glu Pro Gln Arg Ala Asp Ser Pro Leu Glu Gln Pro
225                 230                 235                 240

Glu Gly Ser Pro Leu Thr Gln Asp Asp Arg Gln Val Glu Leu Pro Val
                245                 250                 255

<210>  8
<211>  554
<212>  PRT
<213>  ?

<400>  8

Met Thr Ala Pro Gly Ala Ala Gly Arg Cys Pro Pro Thr Thr Trp Leu
1               5                   10                  15

Gly Ser Leu Leu Leu Leu Val Cys Leu Leu Ala Ser Arg Ser Ile Thr
            20                  25                  30

Glu Glu Val Ser Glu Tyr Cys Ser His Met Ile Gly Ser Gly His Leu
        35                  40                  45

Gln Ser Leu Gln Arg Leu Ile Asp Ser Gln Met Glu Thr Ser Cys Gln
    50                  55                  60

Ile Thr Phe Glu Phe Val Asp Gln Glu Gln Leu Lys Asp Pro Val Cys
65                  70                  75                  80

Tyr Leu Lys Lys Ala Phe Leu Leu Val Gln Asp Ile Met Glu Asp Thr
                85                  90                  95

Met Arg Phe Arg Asp Asn Thr Pro Asn Ala Ile Ala Ile Val Gln Leu
            100                 105                 110

Gln Glu Leu Ser Leu Arg Leu Lys Ser Cys Phe Thr Lys Asp Tyr Glu
        115                 120                 125

Glu His Asp Lys Ala Cys Val Arg Thr Phe Tyr Glu Thr Pro Leu Gln
    130                 135                 140

Leu Leu Glu Lys Val Lys Asn Val Phe Asn Glu Thr Lys Asn Leu Leu
145                 150                 155                 160

Asp Lys Asp Trp Asn Ile Phe Ser Lys Asn Cys Asn Asn Ser Phe Ala
                165                 170                 175

Glu Cys Ser Ser Gln Asp Val Val Thr Lys Pro Asp Cys Asn Cys Leu
            180                 185                 190

Tyr Pro Lys Ala Ile Pro Ser Ser Asp Pro Ala Ser Val Ser Pro His
        195                 200                 205

Gln Pro Leu Ala Pro Ser Met Ala Pro Val Ala Gly Leu Thr Trp Glu
    210                 215                 220

Asp Ser Glu Gly Thr Glu Gly Ser Ser Leu Leu Pro Gly Glu Gln Pro
225                 230                 235                 240

Leu His Thr Val Asp Pro Gly Ser Ala Lys Gln Arg Pro Pro Arg Ser
                245                 250                 255

Thr Cys Gln Ser Phe Glu Pro Pro Glu Thr Pro Val Val Lys Asp Ser

            260                 265                 270

Thr Ile Gly Gly Ser Pro Gln Pro Arg Pro Ser Val Gly Ala Phe Asn
        275                 280                 285

Pro Gly Met Glu Asp Ile Leu Asp Ser Ala Met Gly Thr Asn Trp Val
    290                 295                 300

Pro Glu Glu Ala Ser Gly Glu Ala Ser Glu Ile Pro Val Pro Gln Gly
305                 310                 315                 320

Thr Glu Leu Ser Pro Ser Arg Pro Gly Gly Gly Ser Met Gln Thr Glu
                325                 330                 335

Pro Ala Arg Pro Ser Asn Phe Leu Ser Ala Ser Ser Pro Leu Pro Ala
            340                 345                 350

Ser Ala Lys Gly Gln Gln Pro Ala Asp Val Thr Gly Thr Ala Leu Pro
        355                 360                 365

Arg Val Gly Pro Val Arg Pro Thr Gly Gln Asp Trp Asn His Thr Pro
    370                 375                 380

Gln Lys Thr Asp His Pro Ser Ala Leu Leu Arg Asp Pro Pro Glu Pro
385                 390                 395                 400

Gly Ser Pro Arg Ile Ser Ser Leu Arg Pro Gln Gly Leu Ser Asn Pro
                405                 410                 415

Ser Thr Leu Ser Ala Gln Pro Gln Leu Ser Arg Ser His Ser Ser Gly
            420                 425                 430

Ser Val Leu Pro Leu Gly Glu Leu Glu Gly Arg Arg Ser Thr Arg Asp
        435                 440                 445

Arg Arg Ser Pro Ala Glu Pro Glu Gly Gly Pro Ala Ser Glu Gly Ala
    450                 455                 460

Ala Arg Pro Leu Pro Arg Phe Asn Ser Val Pro Leu Thr Asp Thr Gly
465                 470                 475                 480

His Glu Arg Gln Ser Glu Gly Ser Ser Ser Pro Gln Leu Gln Glu Ser
                485                 490                 495

Val Phe His Leu Leu Val Pro Ser Val Ile Leu Val Leu Leu Ala Val
            500                 505                 510

Gly Gly Leu Leu Phe Tyr Arg Trp Arg Arg Arg Ser His Gln Glu Pro
        515                 520                 525

Gln Arg Ala Asp Ser Pro Leu Glu Gln Pro Glu Gly Ser Pro Leu Thr
    530                 535                 540

Gln Asp Asp Arg Gln Val Glu Leu Pro Val
545                 550

<210>  9
<211>  438
<212>  PRT
<213>  ?

<400>  9

Met Thr Ala Pro Gly Ala Ala Gly Arg Cys Pro Pro Thr Thr Trp Leu
1               5                   10                  15

Gly Ser Leu Leu Leu Leu Val Cys Leu Leu Ala Ser Arg Ser Ile Thr
            20                  25                  30

Glu Glu Val Ser Glu Tyr Cys Ser His Met Ile Gly Ser Gly His Leu
        35                  40                  45

Gln Ser Leu Gln Arg Leu Ile Asp Ser Gln Met Glu Thr Ser Cys Gln
    50                  55                  60

Ile Thr Phe Glu Phe Val Asp Gln Glu Gln Leu Lys Asp Pro Val Cys
65                  70                  75                  80

Tyr Leu Lys Lys Ala Phe Leu Leu Val Gln Asp Ile Met Glu Asp Thr
                85                  90                  95

Met Arg Phe Arg Asp Asn Thr Pro Asn Ala Ile Ala Ile Val Gln Leu
            100                 105                 110

Gln Glu Leu Ser Leu Arg Leu Lys Ser Cys Phe Thr Lys Asp Tyr Glu
        115                 120                 125

Glu His Asp Lys Ala Cys Val Arg Thr Phe Tyr Glu Thr Pro Leu Gln
    130                 135                 140

Leu Leu Glu Lys Val Lys Asn Val Phe Asn Glu Thr Lys Asn Leu Leu
145                 150                 155                 160

Asp Lys Asp Trp Asn Ile Phe Ser Lys Asn Cys Asn Asn Ser Phe Ala
                165                 170                 175

Glu Cys Ser Ser Gln Asp Val Val Thr Lys Pro Asp Cys Asn Cys Leu
            180                 185                 190

Tyr Pro Lys Ala Ile Pro Ser Ser Asp Pro Ala Ser Val Ser Pro His
        195                 200                 205

Gln Pro Leu Ala Pro Ser Met Ala Pro Val Ala Gly Leu Thr Trp Glu
    210                 215                 220

Asp Ser Glu Gly Thr Glu Gly Ser Ser Leu Leu Pro Gly Glu Gln Pro
225                 230                 235                 240

Leu His Thr Val Asp Pro Gly Ser Ala Lys Gln Arg Pro Pro Arg Ser
                245                 250                 255

Thr Cys Gln Ser Phe Glu Pro Pro Glu Thr Pro Val Val Lys Asp Ser
            260                 265                 270

Thr Ile Gly Gly Ser Pro Gln Pro Arg Pro Ser Val Gly Ala Phe Asn
        275                 280                 285

Pro Gly Met Glu Asp Ile Leu Asp Ser Ala Met Gly Thr Asn Trp Val
    290                 295                 300

Pro Glu Glu Ala Ser Gly Glu Ala Ser Glu Ile Pro Val Pro Gln Gly
305                 310                 315                 320

Thr Glu Leu Ser Pro Ser Arg Pro Gly Gly Gly Ser Met Gln Thr Glu
                325                 330                 335

Pro Ala Arg Pro Ser Asn Phe Leu Ser Ala Ser Ser Pro Leu Pro Ala
            340                 345                 350

Ser Ala Lys Gly Gln Gln Pro Ala Asp Val Thr Gly His Glu Arg Gln
        355                 360                 365

Ser Glu Gly Ser Ser Ser Pro Gln Leu Gln Glu Ser Val Phe His Leu
    370                 375                 380

Leu Val Pro Ser Val Ile Leu Val Leu Leu Ala Val Gly Gly Leu Leu
385                 390                 395                 400

Phe Tyr Arg Trp Arg Arg Arg Ser His Gln Glu Pro Gln Arg Ala Asp
                405                 410                 415

Ser Pro Leu Glu Gln Pro Glu Gly Ser Pro Leu Thr Gln Asp Asp Arg
            420                 425                 430

Gln Val Glu Leu Pro Val
        435

<210>  10
<211>  441
<212>  PRT
<213>  Mus musculus

<400>  10

Glu Ile Gln Leu Gln Gln Ser Gly Pro Glu Leu Val Lys Thr Gly Thr
1               5                   10                  15

Ser Val Lys Ile Ser Cys Lys Ala Ser Gly Tyr Ser Phe Thr Gly Tyr
            20                  25                  30

Phe Met His Trp Val Lys Gln Ser His Gly Lys Ser Leu Glu Trp Ile
        35                  40                  45

Gly Tyr Ile Ser Cys Tyr Asn Gly Asp Thr Asn Tyr Asn Gln Asn Phe
    50                  55                  60

Lys Gly Lys Ala Thr Phe Thr Val Asp Thr Ser Ser Ser Thr Ala Tyr
65                  70                  75                  80

Met Gln Phe Asn Ser Leu Thr Ser Glu Asp Ser Ala Val Tyr Tyr Cys
                85                  90                  95

Ala Arg Glu Gly Gly Asn Tyr Pro Ala Tyr Trp Gly Gln Gly Thr Leu
            100                 105                 110

Val Thr Val Ser Ala Ala Lys Thr Thr Pro Pro Ser Val Tyr Pro Leu
        115                 120                 125

Ala Pro Gly Ser Ala Ala Gln Thr Asn Ser Met Val Thr Leu Gly Cys
    130                 135                 140

Leu Val Lys Gly Tyr Phe Pro Glu Pro Val Thr Val Thr Trp Asn Ser
145                 150                 155                 160

Gly Ser Leu Ser Ser Gly Val His Thr Phe Pro Ala Val Leu Gln Ser
                165                 170                 175

Asp Leu Tyr Thr Leu Ser Ser Ser Val Thr Val Pro Ser Ser Thr Trp
            180                 185                 190

Pro Ser Glu Thr Val Thr Cys Asn Val Ala His Pro Ala Ser Ser Thr
        195                 200                 205

Lys Val Asp Lys Lys Ile Val Pro Arg Asp Cys Gly Cys Lys Pro Cys
    210                 215                 220

Ile Cys Thr Val Pro Glu Val Ser Ser Val Phe Ile Phe Pro Pro Lys
225                 230                 235                 240

Pro Lys Asp Val Leu Thr Ile Thr Leu Thr Pro Lys Val Thr Cys Val
                245                 250                 255

Val Val Asp Ile Ser Lys Asp Asp Pro Glu Val Gln Phe Ser Trp Phe
            260                 265                 270

Val Asp Asp Val Glu Val His Thr Ala Gln Thr Gln Pro Arg Glu Glu
        275                 280                 285

Gln Phe Asn Ser Thr Phe Arg Ser Val Ser Glu Leu Pro Ile Met His
    290                 295                 300

Gln Asp Trp Leu Asn Gly Lys Glu Phe Lys Cys Arg Val Asn Ser Ala
305                 310                 315                 320

Ala Phe Pro Ala Pro Ile Glu Lys Thr Ile Ser Lys Thr Lys Gly Arg
                325                 330                 335

Pro Lys Ala Pro Gln Val Tyr Thr Ile Pro Pro Pro Lys Glu Gln Met
            340                 345                 350

Ala Lys Asp Lys Val Ser Leu Thr Cys Met Ile Thr Asp Phe Phe Pro
        355                 360                 365

Glu Asp Ile Thr Val Glu Trp Gln Trp Asn Gly Gln Pro Ala Glu Asn
    370                 375                 380

Tyr Lys Asn Thr Gln Pro Ile Met Asp Thr Asp Gly Ser Tyr Phe Val
385                 390                 395                 400

Tyr Ser Lys Leu Asn Val Gln Lys Ser Asn Trp Glu Ala Gly Asn Thr
                405                 410                 415

Phe Thr Cys Ser Val Leu His Glu Gly Leu His Asn His His Thr Glu
            420                 425                 430

Lys Ser Leu Ser His Ser Pro Gly Lys
        435                 440

<210>  11
<211>  214

<212>  PRT
<213>  Mus musculus

<400>  11

Asp Ile Val Met Thr Gln Ser His Lys Phe Met Ser Thr Ser Val Gly
1               5                   10                  15

Asp Arg Val Thr Ile Thr Cys Lys Ala Ser Gln Asn Val Gly Thr Ala
            20                  25                  30

Val Thr Trp Tyr Gln Gln Lys Pro Gly Gln Ser Pro Lys Leu Leu Ile
        35                  40                  45

Tyr Trp Thr Ser Thr Arg His Ala Gly Val Pro Asp Arg Phe Thr Gly
    50                  55                  60

Ser Gly Ser Gly Thr Asp Phe Thr Leu Thr Ile Ser Asp Val Gln Ser
65                  70                  75                  80

Glu Asp Leu Ala Asp Tyr Phe Cys Gln Gln Tyr Ser Ser Tyr Pro Leu
                85                  90                  95

Thr Phe Gly Ala Gly Thr Lys Leu Glu Leu Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>  12
<211>  449

<212>  PRT
<213>  Mus musculus

<400>  12

Glu Val Lys Leu Val Glu Ser Gly Gly Gly Leu Val Gln Pro Gly Gly
1               5                   10                  15

Ser Leu Lys Leu Ser Cys Ala Thr Ser Gly Phe Thr Phe Ser Asp Tyr
            20                  25                  30

Tyr Met Tyr Trp Val Arg Gln Thr Pro Glu Lys Arg Leu Glu Trp Val
        35                  40                  45

Ala Tyr Ile Ser Asn Gly Gly Gly Ser Thr Tyr Tyr Pro Asp Thr Val
    50                  55                  60

Lys Gly Arg Phe Thr Ile Ser Arg Asp Asn Ala Lys Asn Thr Leu Tyr
65                  70                  75                  80

Leu Gln Met Ser Arg Leu Lys Ser Glu Asp Thr Ala Met Tyr Tyr Cys
                85                  90                  95

Ala Arg Gln Gly Ser Tyr Gly Tyr Pro Phe Ala Tyr Trp Gly Gln Gly
            100                 105                 110

Thr Leu Val Thr Val Ser Ala Ala Lys Thr Thr Ala Pro Ser Val Tyr
        115                 120                 125

Pro Leu Ala Pro Val Cys Gly Asp Thr Thr Gly Ser Ser Val Thr Leu
    130                 135                 140

Gly Cys Leu Val Lys Gly Tyr Phe Pro Glu Pro Val Thr Leu Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Thr Ser Ser
            180                 185                 190

Thr Trp Pro Ser Gln Ser Ile Thr Cys Asn Val Ala His Pro Ala Ser
        195                 200                 205

Ser Thr Lys Val Asp Lys Lys Ile Glu Pro Arg Gly Pro Thr Ile Lys
    210                 215                 220

Pro Cys Pro Pro Cys Lys Cys Pro Ala Pro Asn Leu Leu Gly Gly Pro
225                 230                 235                 240

Ser Val Phe Ile Phe Pro Pro Lys Ile Lys Asp Val Leu Met Ile Ser
                245                 250                 255

Leu Ser Pro Ile Val Thr Cys Val Val Val Asp Val Ser Glu Asp Asp
            260                 265                 270

Pro Asp Val Gln Ile Ser Trp Phe Val Asn Asn Val Glu Val His Thr
        275                 280                 285

Ala Gln Thr Gln Thr His Arg Glu Asp Tyr Asn Ser Thr Leu Arg Val
    290                 295                 300

Val Ser Ala Leu Pro Ile Gln His Gln Asp Trp Met Ser Gly Lys Glu
305                 310                 315                 320

Phe Lys Cys Lys Val Asn Asn Lys Asp Leu Pro Ala Pro Ile Glu Arg
                325                 330                 335

Thr Ile Ser Lys Pro Lys Gly Ser Val Arg Ala Pro Gln Val Tyr Val
            340                 345                 350

Leu Pro Pro Pro Glu Glu Glu Met Thr Lys Lys Gln Val Thr Leu Thr
        355                 360                 365

Cys Met Val Thr Asp Phe Met Pro Glu Asp Ile Tyr Val Glu Trp Thr
    370                 375                 380

Asn Asn Gly Lys Thr Glu Leu Asn Tyr Lys Asn Thr Glu Pro Val Leu
385                 390                 395                 400

Asp Ser Asp Gly Ser Tyr Phe Met Tyr Ser Lys Leu Arg Val Glu Lys
                405                 410                 415

Lys Asn Trp Val Glu Arg Asn Ser Tyr Ser Cys Ser Val Val His Glu
            420                 425                 430

Gly Leu His Asn His His Thr Thr Lys Ser Phe Ser Arg Thr Pro Gly
        435                 440                 445

Lys

<210>  13
<211>  214
<212>  PRT
<213>  Mus musculus

<400>  13

Ala Ile Gln Met Thr Gln Thr Thr Ser Ser Leu Ser Ala Ser Leu Gly
1               5                   10                  15

Asp Arg Val Thr Ile Ser Cys Ser Ala Ser Gln Gly Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Lys Leu Pro Trp
                85                  90                  95

Thr Phe Gly Gly Gly Thr Lys Leu Glu Ile Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>  14
<211>  522
<212>  PRT
<213>  Mus musculus

<400> 14

Asp Val Gln Leu Gln Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Ser Tyr Ser Gly Ser Thr Ser Tyr Asn Pro Ser Leu
    50                  55                  60

Lys Ser Arg Ile Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Arg Leu Glu Thr Trp Leu Phe Asp Tyr Trp Gly Gln Gly Thr Thr
            100                 105                 110

Leu Thr Val Ser Ser Ala Lys Thr Thr Pro Pro Ser Val Tyr Pro Leu
        115                 120                 125

Ala Pro Gly Cys Gly Asp Thr Thr Gly Ser Ser Val Thr Leu Gly Cys
    130                 135                 140

Leu Val Lys Gly Tyr Phe Pro Glu Ser Val Thr Val Thr Trp Asn Ser
145                 150                 155                 160

Gly Ser Leu Ser Ser Ser Val His Thr Phe Pro Ala Leu Leu Gln Ser
                165                 170                 175

Gly Leu Tyr Thr Met Ser Ser Ser Val Thr Val Pro Ser Ser Thr Trp
            180                 185                 190

Pro Ser Gln Thr Val Thr Cys Ser Val Ala His Pro Ala Ser Ser Thr
        195                 200                 205

Thr Val Asp Lys Lys Leu Glu Pro Ser Gly Pro Ile Ser Thr Ile Asn
    210                 215                 220

Pro Cys Pro Pro Cys Lys Glu Cys His Lys Cys Pro Ala Pro Asn Leu
225                 230                 235                 240

Glu Gly Gly Pro Ser Val Phe Ile Phe Pro Pro Asn Ile Lys Asp Val

245                 250                 255

Leu Met Ile Ser Leu Thr Pro Lys Val Thr Cys Val Val Val Asp Val
            260                 265                 270

Ser Glu Asp Asp Pro Asp Val Gln Ile Ser Trp Phe Val Asn Asn Val
        275                 280                 285

Glu Val His Thr Ala Gln Thr Gln Thr His Arg Glu Asp Tyr Asn Ser
    290                 295                 300

Thr Ile Arg Val Val Ser Thr Leu Pro Ile Gln His Gln Asp Trp Met
305                 310                 315                 320

Ser Gly Lys Glu Phe Lys Cys Lys Val Asn Asn Lys Asp Leu Pro Ser
                325                 330                 335

Pro Ile Glu Arg Thr Ile Ser Lys Ile Lys Gly Leu Val Arg Ala Pro
            340                 345                 350

Gln Val Tyr Ile Leu Pro Pro Pro Ala Glu Gln Leu Ser Arg Lys Asp
        355                 360                 365

Val Ser Leu Thr Cys Leu Val Val Gly Phe Asn Pro Gly Asp Ile Ser
    370                 375                 380

Val Glu Trp Thr Ser Asn Gly His Thr Glu Glu Asn Tyr Lys Asp Thr
385                 390                 395                 400

Ala Pro Val Leu Asp Ser Asp Gly Ser Tyr Phe Ile Tyr Ser Lys Leu
                405                 410                 415

Asn Met Lys Thr Ser Lys Trp Glu Lys Thr Asp Ser Phe Ser Cys Asn
            420                 425                 430

Val Arg His Glu Gly Leu Lys Asn Tyr Tyr Leu Lys Lys Thr Ile Ser
        435                 440                 445

Arg Ser Pro Gly Leu Asp Leu Asp Asp Ile Cys Ala Glu Ala Lys Asp
    450                 455                 460

Gly Glu Leu Asp Gly Leu Trp Thr Thr Ile Thr Ile Phe Ile Ser Leu
465                 470                 475                 480

Phe Leu Leu Ser Val Cys Tyr Ser Ala Ser Val Thr Leu Phe Lys Val
                485                 490                 495

Lys Trp Ile Phe Ser Ser Val Val Glu Leu Lys Gln Lys Ile Ser Pro
            500                 505                 510

Asp Tyr Arg Asn Met Ile Gly Gln Gly Ala
        515                 520

<210>  15
<211>  214
<212>  PRT
<213>  Mus musculus

<400>  15

Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Ser Ile Gly Thr Ser
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Ser Asn Ser Trp Pro Thr
                85                  90                  95

Thr Phe Gly Gly Gly Thr Lys Leu Glu Ile Lys Trp Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>  16
<211>  5
<212>  PRT
<213>  Homo sapiens

<400>  16

Gly Tyr Phe Met His
1               5

<210>  17
<211>  5
<212>  PRT
<213>  Homo sapiens

<400>  17

Asp Tyr Tyr Met Tyr
1               5

<210>  18
<211>  6
<212>  PRT
<213>  Homo sapiens

<400>  18

Ser Asp Tyr Ala Trp Asn
1               5

<210>  19
<211>  17
<212>  PRT
<213>  Homo sapiens

<400>  19

Tyr Ile Ser Cys Tyr Asn Gly Asp Thr Asn Tyr Asn Gln Asn Phe Lys
1               5                   10                  15

Gly

<210>  20
<211>  17
<212>  PRT
<213>  Homo sapiens

<400>  20

Tyr Ile Ser Asn Gly Gly Gly Ser Thr Tyr Tyr Pro Asp Thr Val Lys
1               5                   10                  15

Gly

<210>  21
<211>  16
<212>  PRT
<213>  Homo sapiens

<400>  21

Tyr Ile Ser Tyr Ser Gly Ser Thr Ser Tyr Asn Pro Ser Leu Lys Ser
1               5                   10                  15

<210>  22
<211>  8
<212>  PRT
<213>  Homo sapiens

<400>  22

Glu Gly Gly Asn Tyr Pro Ala Tyr
1               5

<210>  23
<211>  10
<212>  PRT
<213>  Homo sapiens

<400>  23

Gln Gly Ser Tyr Gly Tyr Pro Phe Ala Tyr
1               5                   10

<210>  24
<211>  9
<212>  PRT
<213>  Homo sapiens

<400>  24

Phe Asp Tyr Ala His Ala Met Asp Tyr
1               5

<210>  25
<211>  8
<212>  PRT
<213>  Homo sapiens

<400>  25

Leu Glu Thr Trp Leu Phe Asp Tyr
1               5

<210>  26
<211>  7
<212>  PRT
<213>  Homo sapiens

<400>  26

Asp Tyr Gly Trp Phe Asp Tyr
1               5


<210>  27
<211>  11
<212>  PRT
<213>  Homo sapiens

<400>  27

Lys Ala Ser Gln Asn Val Gly Thr Ala Val Thr
1               5                   10


<210>  28
<211>  11
<212>  PRT
<213>  Homo sapiens

<400>  28

Ser Ala Ser Gln Gly Ile Ser Asn Tyr Leu Asn
1               5                   10


<210>  29
<211>  11
<212>  PRT
<213>  Homo sapiens

<400>  29

Arg Ala Ser Gln Ser Ile Gly Thr Ser Ile His
1               5                   10


<210>  30
<211>  7
<212>  PRT
<213>  Homo sapiens

<400>  30

Trp Thr Ser Thr Arg His Ala
1               5


<210>  31
<211>  7
<212>  PRT
<213>  Homo sapiens

<400>  31

Tyr Thr Ser Ser Leu His Ser

1               5


<210>  32
<211>  7
<212>  PRT
<213>  Homo sapiens

<400>  32

Tyr Ala Ser Glu Ser Ile Ser
1               5


<210>  33
<211>  7
<212>  PRT
<213>  Homo sapiens

<400>  33

Tyr Thr Ser Glu Ser Ile Ser
1               5


<210>  34
<211>  9
<212>  PRT
<213>  Homo sapiens

<400>  34

Gln Gln Tyr Ser Ser Tyr Pro Leu Thr
1               5


<210>  35
<211>  9
<212>  PRT
<213>  Homo sapiens

<400>  35

Gln Gln Tyr Ser Lys Leu Pro Trp Thr
1               5


<210>  36
<211>  9
<212>  PRT
<213>  Homo sapiens

<400>  36

Gln Gln Ile Asn Ser Trp Pro Thr Thr
1               5


<210>  37
<211>  9
<212>  PRT
<213>  Homo sapiens

<400>  37

Gln Gln Ser Asn Ser Trp Pro Thr Thr
1               5


<210>  38
<211>  9
<212>  PRT
<213>  Homo sapiens

<400>  38

Gln Gln Tyr Ser Ser Trp Pro Thr Thr
1               5


<210>  39
<211>  130
<212>  PRT
<213>  Homo sapiens


<220>
<221>  misc_feature
<222>  (23)..(23)
<223>  Xaa can be any naturally occurring amino acid

<220>
<221>  misc_feature
<222>  (27)..(27)
<223>  Xaa can be any naturally occurring amino acid

<220>
<221>  misc_feature
<222>  (29)..(29)
<223>  Xaa can be any naturally occurring amino acid

<220>
<221>  misc_feature
<222>  (31)..(36)
<223>  Xaa can be any naturally occurring amino acid

<220>
<221>  misc_feature
<222>  (51)..(51)
<223>  Xaa can be any naturally occurring amino acid

<220>
<221>  misc_feature
<222>  (56)..(57)
<223>  Xaa can be any naturally occurring amino acid

<220>
<221>  misc_feature
<222>  (59)..(59)
<223>  Xaa can be any naturally occurring amino acid

<220>
<221>  misc_feature
<222>  (61)..(61)
<223>  Xaa can be any naturally occurring amino acid

<220>
<221>  misc_feature
<222>  (84)..(84)
<223>  Xaa can be any naturally occurring amino acid

<220>
<221>  misc_feature
<222>  (86)..(86)
<223>  Xaa can be any naturally occurring amino acid

<220>
<221>  misc_feature
<222>  (101)..(116)
<223>  Xaa can be any naturally occurring amino acid

<220>
<221>  misc_feature
<222>  (119)..(119)
<223>  Xaa can be any naturally occurring amino acid

<220>
<221>  misc_feature
<222>  (125)..(125)
<223>  Xaa can be any naturally occurring amino acid

<400>  39

Asp Val Gln Leu Gln Glu Ser Gly Pro Gly Leu Val Lys Pro Ser Gln
1               5                   10                  15

Thr Leu Ser Leu Thr Cys Xaa Val Ser Gly Xaa Ser Xaa Ser Xaa Xaa
            20                  25                  30

Xaa Xaa Xaa Xaa Trp Ile Arg Gln Pro Pro Gly Lys Gly Leu Glu Trp
        35                  40                  45

Ile Gly Xaa Tyr Tyr Arg Ala Xaa Xaa Gly Xaa Thr Xaa Tyr Asn Pro
    50                  55                  60

Ser Leu Lys Ser Arg Val Thr Ile Ser Val Asp Thr Ser Lys Asn Gln
65                  70                  75                  80

Phe Ser Leu Xaa Leu Xaa Ser Val Thr Ala Ala Asp Thr Ala Val Tyr
                85                  90                  95

Tyr Cys Ala Arg Xaa Xaa Xaa Xaa Xaa Xaa Xaa Xaa Xaa Xaa Xaa Xaa
            100                 105                 110

Xaa Xaa Xaa Xaa Phe Asp Xaa Trp Gly Gln Gly Thr Xaa Val Thr Val
        115                 120                 125

Ser Ser
    130

<210>  40
<211>  354
<212>  DNA
<213>  Homo sapiens

<400>  40
gacgtacaac ttcaagaatc tggcccaggt ctcgtcaaac cttctcaaac tctctcactc      60

acctgcactg ttactgacta ctctattaca tccgactacg cttggaactg gatccgacaa     120

tttcctggta aaaaactcga atggatgggt tatatttctt actctggctc cacctcctac     180

aatccttctc tgaaatcacg catcacaatt tcccgcgata cctctaaaaa tcaattttca     240

ctccaactca attctgttac cgccgccgat actgccacct actactgtgc ctcttttgac     300

tacgctcacg ccatggatta ttggggacag ggtactaccg ttaccgtaag ctca           354


<210>  41
<211>  118
<212>  PRT
<213>  Homo sapiens

<400>  41

Asp Val Gln Leu Gln Glu Ser Gly Pro Gly Leu Val Lys Pro Ser Gln
1               5                   10                  15


Thr Leu Ser Leu Thr Cys Thr Val Thr Asp Tyr Ser Ile Thr Ser Asp
            20                  25                  30


Tyr Ala Trp Asn Trp Ile Arg Gln Phe Pro Gly Lys Lys Leu Glu Trp
        35                  40                  45


Met Gly Tyr Ile Ser Tyr Ser Gly Ser Thr Ser Tyr Asn Pro Ser Leu
    50                  55                  60


Lys Ser Arg Ile Thr Ile Ser Arg Asp Thr Ser Lys Asn Gln Phe Ser
65                  70                  75                  80


Leu Gln Leu Asn Ser Val Thr Ala Ala Asp Thr Ala Thr Tyr Tyr Cys
                85                  90                  95


Ala Ser Phe Asp Tyr Ala His Ala Met Asp Tyr Trp Gly Gln Gly Thr
            100                 105                 110


Thr Val Thr Val Ser Ser
        115


<210>  42
<211>  354
<212>  DNA
<213>  Homo sapiens

<400>  42
caagttcaac ttcaagaatc aggccccgga ctcgttaaac cctctcaaac tctctctctt      60

acttgcactg tatccgatta ctctattact tcagactacg cttggaactg gatcagacaa     120

tttcccggaa aaggactcga atggatggga tatatctctt actctggctc aacctcttac     180

aacccctctc tcaaatctcg aataacaatc tcacgcgata cttctaaaaa tcaattctca     240

cttcaactta actccgttac tgccgccgac actgccgttt actactgtgc ttccttcgat     300

tacgcccacg ctatggatta ttggggacaa ggaactaccg tcactgtcag ctca           354


<210>  43
<211>  118
<212>  PRT
<213>  Homo sapiens

<400>  43

Gln Val Gln Leu Gln Glu Ser Gly Pro Gly Leu Val Lys Pro Ser Gln
1               5                   10                  15


Thr Leu Ser Leu Thr Cys Thr Val Ser Asp Tyr Ser Ile Thr Ser Asp
            20                  25                  30


Tyr Ala Trp Asn Trp Ile Arg Gln Phe Pro Gly Lys Gly Leu Glu Trp
        35                  40                  45


Met Gly Tyr Ile Ser Tyr Ser Gly Ser Thr Ser Tyr Asn Pro Ser Leu
    50                  55                  60


Lys Ser Arg Ile Thr Ile Ser Arg Asp Thr Ser Lys Asn Gln Phe Ser
65                  70                  75                  80


Leu Gln Leu Asn Ser Val Thr Ala Ala Asp Thr Ala Val Tyr Tyr Cys
                85                  90                  95


Ala Ser Phe Asp Tyr Ala His Ala Met Asp Tyr Trp Gly Gln Gly Thr
            100                 105                 110


Thr Val Thr Val Ser Ser
        115


<210>  44
<211>  327
<212>  DNA
<213>  Homo sapiens

<400>  44
gaaatagttc ttactcaatc ccccggtaca ctctcagttt ccccaggcga acgcgtcact      60

ttttcttgca gagcatcaca atcaatcggc acttcaattc attggtatca acaaaaaaca     120

ggacaggccc cacgacttct tattaaatat gcatcagaac gagccacagg catcccagac 180

agattttcag gttcaggatc aggcaccgat ttcacactta caatatccag agtcgaatca 240

gaagattttg cagattacta ttgtcaacaa ataaacagct ggcccactac attcggacaa 300

ggcacaaaac tcgaaattaa acgtacg 327

<210> 45
<211> 109
<212> PRT
<213> Homo sapiens

<400> 45

Glu Ile Val Leu Thr Gln Ser Pro Gly Thr Leu Ser Val Ser Pro Gly
1               5                   10                  15

Glu Arg Val Thr Phe Ser Cys Arg Ala Ser Gln Ser Ile Gly Thr Ser
            20                  25                  30

Ile His Trp Tyr Gln Gln Lys Thr Gly Gln Ser Pro Arg Leu Leu Ile
        35                  40                  45

Lys Tyr Ala Ser Glu Arg Ile Ser Gly Ile Pro Asp Arg Phe Ser Gly
    50                  55                  60

Ser Gly Ser Gly Thr Asp Phe Thr Leu Thr Ile Ser Arg Val Glu Ser
65                  70                  75                  80

Glu Asp Phe Ala Asp Tyr Tyr Cys Gln Gln Ile Asn Ser Trp Pro Thr
                85                  90                  95

Thr Phe Gly Gln Gly Thr Lys Leu Glu Ile Lys Arg Thr
            100                 105

<210> 46
<211> 327
<212> DNA
<213> Homo sapiens

<400> 46
gaaatagttc ttactcaatc ccccggtaca ctctcagttt ccccaggcga acgcgtcact 60

ttttcttgca gagcatcaca atcaatcggc acttcaattc attggtatca acaaaaaaca 120

ggacaggccc cacgacttct tattaaatat gcatcagaac gagccacagg catcccagac 180

agattttcag gttcaggatc aggcaccgat ttcacactta caatatccag agtcgaatca 240

gaagattttg cagattacta ttgtcaacaa ataaacagct ggcccactac attcggacaa 300

ggcacaaaac tcgaaattaa acgtacg 327

<210>  47
<211>  109
<212>  PRT
<213>  Homo sapiens

<400>  47

Glu Ile Val Leu Thr Gln Ser Pro Gly Thr Leu Ser Val Ser Pro Gly
1               5                   10                  15

Glu Arg Val Thr Phe Ser Cys Arg Ala Ser Gln Ser Ile Gly Thr Ser
            20                  25                  30

Ile His Trp Tyr Gln Gln Lys Thr Gly Gln Ala Pro Arg Leu Leu Ile
        35                  40                  45

Lys Tyr Ala Ser Glu Arg Ala Thr Gly Ile Pro Asp Arg Phe Ser Gly
    50                  55                  60

Ser Gly Ser Gly Thr Asp Phe Thr Leu Thr Ile Ser Arg Val Glu Ser
65                  70                  75                  80

Glu Asp Phe Ala Asp Tyr Tyr Cys Gln Gln Ile Asn Ser Trp Pro Thr
                85                  90                  95

Thr Phe Gly Gln Gly Thr Lys Leu Glu Ile Lys Arg Thr
            100                 105

<210>  48
<211>  109
<212>  PRT
<213>  Homo sapiens

<400>  48

Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Ser Ile Gly Thr Ser
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Asp Arg Phe Ser Gly
    50                  55                  60

Ser Gly Ser Gly Thr Asp Phe Thr Leu Thr Ile Ser Arg Val Glu Ser
65                  70                  75                  80

Glu Asp Phe Ala Asp Tyr Tyr Cys Gln Gln Ile Asn Ser Trp Pro Thr

85                  90                  95

Thr Phe Gly Gln Gly Thr Lys Leu Glu Ile Lys Arg Thr
            100                 105

<210>  49
<211>  111
<212>  PRT
<213>  Homo sapiens

<220>
<221>  misc_feature
<222>  (98)..(98)
<223>  Xaa can be any naturally occurring amino acid

<400>  49

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Pro Xaa Thr Phe Gly Gln Gly Thr Lys Val Glu Ile Lys Arg Thr
            100                 105                 110

<210>  50
<211>  108
<212>  PRT
<213>  Homo sapiens

<400>  50

Asp Val Val Met Thr Gln Ser Pro Ala Phe Leu Ser Val Thr Pro Gly
1               5                   10                  15

Glu Lys Val Thr Ile Thr Cys Gln Ala Ser Glu Gly Ile Gly Asn Tyr
            20                  25                  30

Leu Tyr Trp Tyr Gln Gln Lys Pro Asp Gln Ala Lys Leu Leu Ile Lys
        35                  40                  45

Tyr Ala Ser Gln Ser Ile Ser Gly Val Pro Ser Arg Phe Ser Gly Ser
    50                  55                  60

Gly Ser Gly Thr Asp Phe Thr Phe Thr Ile Ser Ser Leu Glu Ala Glu
65                  70                  75                  80

Asp Ala Ala Thr Tyr Tyr Cys Gln Gln Gly Asn Lys His Pro Leu Thr
                85                  90                  95

Phe Gly Gly Gly Thr Lys Val Glu Ile Lys Arg Thr
            100                 105

<210>  51
<211>  109
<212>  PRT
<213>  Artificial sequence

<220>
<223>  Low Risk Light Chain vs. VK6 Subgroup 2-1-(1) A14:

<400>  51

Asp Ile Val Leu Thr Gln Ser Pro Ala Phe Leu Ser Val Thr Pro Gly
1               5                   10                  15

Glu Lys Val Thr Phe Thr Cys Gln Ala Ser Gln Ser Ile Gly Thr Ser
            20                  25                  30

Ile His Trp Tyr Gln Gln Lys Thr Asp Gln Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Thr Ile Ser Ser Val Glu Ala
65                  70                  75                  80

Glu Asp Ala Ala Asp Tyr Tyr Cys Gln Gln Ile Asn Ser Trp Pro Thr
                85                  90                  95

Thr Phe Gly Gly Gly Thr Lys Leu Glu Ile Lys Arg Thr
            100                 105

<210>  52
<211>  327
<212>  DNA
<213>  Artificial sequence

<220>
<223>  Low Risk + Moderate Risk Light Chain vs. VK6 Subgroup 2-1-(1)
      A14: DNA SEQ

<400>  52
gacatagttc tcacacaatc accagcattc ctctcagtta cacccggcga aaaagtaacc      60

tttacctgtc aggcttctca atctatcggc acttctattc actggtatca acaaaaaacc     120

gatcaagctc ctaaactcct cataaaatac gcatccgaat ccatctccgg tatcccctcc     180

agattttcag gctccggctc cggcacagat ttcaccctta ccattagctc agttgaagcc     240

gaagacgcag ctgattacta ctgtcaacaa ataaactcat ggcccactac tttcggcggc     300

ggcactaaac tcgaaataaa acgtacg                                         327


<210>  53
<211>  109
<212>  PRT
<213>  Artificial seqeunce

<220>
<223>  Low Risk + Moderate Risk Light Chain vs. VK6 Subgroup 2-1-(1)
      A14:

<400>  53

Asp Ile Val Leu Thr Gln Ser Pro Ala Phe Leu Ser Val Thr Pro Gly
1               5                   10                  15


Glu Lys Val Thr Phe Thr Cys Gln Ala Ser Gln Ser Ile Gly Thr Ser
            20                  25                  30


Ile His Trp Tyr Gln Gln Lys Thr Asp Gln Ala Pro Lys Leu Leu Ile
        35                  40                  45


Lys Tyr Ala Ser Glu Ser Ile Ser Gly Ile Pro Ser Arg Phe Ser Gly
    50                  55                  60


Ser Gly Ser Gly Thr Asp Phe Thr Leu Thr Ile Ser Ser Val Glu Ala
65                  70                  75                  80


Glu Asp Ala Ala Asp Tyr Tyr Cys Gln Gln Ile Asn Ser Trp Pro Thr
                85                  90                  95


Thr Phe Gly Gly Gly Thr Lys Leu Glu Ile Lys Arg Thr
            100                 105


<210>  54
<211>  100
<212>  PRT
<213>  Homo sapiens

<400>  54

Gln Ile Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Val Gly Val Gly Trp Ile Arg Gln Pro Pro Gly Lys Ala Leu Glu
        35                  40                  45

Trp Leu Ala Leu Ile Tyr Trp Asn Asp Asp Lys Arg Tyr Ser Pro Ser
    50                  55                  60

Leu Lys Ser Arg Leu Thr Ile Thr Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala His Arg
            100

<210>  55
<211>  98
<212>  PRT
<213>  Homo sapiens

<400>  55

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg

<210> 56
<211> 98
<212> PRT
<213> Homo sapiens

<400> 56

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg

<210> 57
<211> 98
<212> PRT
<213> Homo sapiens

<400> 57

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg

<210> 58
<211> 101
<212> PRT
<213> Homo sapiens

<400> 58

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg
            100

<210> 59
<211> 98
<212> PRT
<213> Homo sapiens

<400> 59

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Ser Tyr
            20                  25                  30

Ala Met Asn Trp Val Arg Gln Ala Pro Gly Gln Gly Leu Glu Trp Met
        35                  40                  45

Gly Trp Ile Asn Thr Asn Thr Gly Asn Pro Thr Tyr Ala Gln Gly Phe

50                  55                  60

Thr Gly Arg Phe Val Phe Ser Leu Asp Thr Ser Val Ser Thr Ala Tyr
65                  70                  75                  80

Leu Gln Ile Cys Ser Leu Lys Ala Glu Asp Thr Ala Val Tyr Tyr Cys
                85                  90                  95

Ala Arg

<210> 60
<211> 58
<212> PRT
<213> Mus musculus

<400> 60

Asp Val Gln Leu Gln Glu Ser Gly Pro Gly Leu Val Lys Pro Ser Gln
1               5                   10                  15

Ser Leu Ser Leu Thr Cys Thr Val Thr Asp Tyr Ser Ile Thr Ser Asp
            20                  25                  30

Tyr Ala Trp Asn Trp Ile Arg Gln Phe Pro Gly Asn Lys Leu Glu Trp
        35                  40                  45

Met Gly Tyr Ile Ser Tyr Ser Gly Ser Thr
    50                  55

<210> 61
<211> 59
<212> PRT
<213> Homo sapiens

<220>
<221> misc_feature
<222> (1)..(1)
<223> Xaa can be any naturally occurring amino acid

<220>
<221> misc_feature
<222> (16)..(16)
<223> Xaa can be any naturally occurring amino acid

<220>
<221> misc_feature
<222> (30)..(30)
<223> Xaa can be any naturally occurring amino acid

<220>
<221> misc_feature
<222> (33)..(33)
<223> Xaa can be any naturally occurring amino acid

<220>
<221>  misc_feature
<222>  (35)..(35)
<223>  Xaa can be any naturally occurring amino acid

<220>
<221>  misc_feature
<222>  (50)..(50)
<223>  Xaa can be any naturally occurring amino acid

<220>
<221>  misc_feature
<222>  (52)..(52)
<223>  Xaa can be any naturally occurring amino acid

<220>
<221>  misc_feature
<222>  (55)..(56)
<223>  Xaa can be any naturally occurring amino acid

<220>
<221>  misc_feature
<222>  (58)..(58)
<223>  Xaa can be any naturally occurring amino acid

<400> 61

Xaa Val Gln Leu Val Gln Ser Gly Ala Glu Val Lys Lys Pro Gly Xaa
1               5                   10                  15

Ser Val Lys Val Ser Cys Lys Ala Ser Gly Tyr Thr Phe Xaa Ser Tyr
            20                  25                  30

Xaa Ile Xaa Trp Val Arg Gln Ala Pro Gly Gln Gly Leu Glu Trp Met
        35                  40                  45

Gly Xaa Ile Xaa Pro Tyr Xaa Xaa Gly Xaa Thr
    50                  55

<210> 62
<211>  62
<212>  PRT
<213>  Homo sapiens

<220>
<221>  misc_feature
<222>  (23)..(23)
<223>  Xaa can be any naturally occurring amino acid

<220>
<221>  misc_feature
<222>  (27)..(27)
<223>  Xaa can be any naturally occurring amino acid

<220>
<221>  misc_feature
<222>  (29)..(29)

<223>  Xaa can be any naturally occurring amino acid

<220>
<221>  misc_feature
<222>  (32)..(37)
<223>  Xaa can be any naturally occurring amino acid

<220>
<221>  misc_feature
<222>  (52)..(52)
<223>  Xaa can be any naturally occurring amino acid

<220>
<221>  misc_feature
<222>  (58)..(59)
<223>  Xaa can be any naturally occurring amino acid

<220>
<221>  misc_feature
<222>  (61)..(61)
<223>  Xaa can be any naturally occurring amino acid

<400> 62

Gln Val Gln Leu Gln Glu Ser Gly Pro Gly Leu Val Lys Pro Ser Gln
1               5                   10                  15

Thr Leu Ser Leu Thr Cys Xaa Val Ser Gly Xaa Ser Xaa Ser Ser Xaa
            20                  25                  30

Xaa Xaa Xaa Xaa Xaa Trp Ile Arg Gln Pro Pro Gly Lys Gly Leu Glu
        35                  40                  45

Trp Ile Gly Xaa Ile Tyr Tyr Arg Ala Xaa Xaa Gly Xaa Thr
    50                  55                  60

<210> 63
<211>  60
<212>  PRT
<213>  Homo sapiens

<220>
<221>  misc_feature
<222>  (31)..(31)
<223>  Xaa can be any naturally occurring amino acid

<220>
<221>  misc_feature
<222>  (33)..(33)
<223>  Xaa can be any naturally occurring amino acid

<220>
<221>  misc_feature
<222>  (35)..(35)
<223>  Xaa can be any naturally occurring amino acid

<220>
<221>  misc_feature

<222>  (49)..(50)
<223>  Xaa can be any naturally occurring amino acid

<220>
<221>  misc_feature
<222>  (52)..(53)
<223>  Xaa can be any naturally occurring amino acid

<220>
<221>  misc_feature
<222>  (55)..(56)
<223>  Xaa can be any naturally occurring amino acid

<220>
<221>  misc_feature
<222>  (58)..(59)
<223>  Xaa can be any naturally occurring amino acid

<400> 63

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Xaa Tyr
            20                  25                  30

Xaa Met Xaa Trp Val Arg Gln Ala Pro Gly Lys Gly Leu Glu Trp Val
        35                  40                  45

Xaa Xaa Ile Xaa Xaa Lys Xaa Xaa Gly Xaa Xaa Thr
    50                  55                  60

<210> 64
<211>  58
<212>  PRT
<213>  Homo sapiens

<400> 64

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr
    50                  55

<210> 65
<211>  59
<212>  PRT
<213>  Homo sapiens

<400> 65

Gln Ile Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Val Gly Val Gly Trp Ile Arg Gln Pro Pro Gly Lys Ala Leu Glu
        35                  40                  45

Trp Leu Ala Leu Ile Tyr Trp Asn Asp Asp Lys
    50                  55

<210> 66
<211> 58
<212> PRT
<213> Homo sapiens

<400> 66

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys
    50                  55

<210> 67
<211> 58
<212> PRT
<213> Homo sapiens

<400> 67

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr
    50                  55

<210> 68
<211> 58
<212> PRT
<213> Homo sapiens

<400> 68

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Met Pro Gly Lys Gly Leu Glu Trp Met
        35                  40                  45

Gly Ile Ile Tyr Pro Gly Asp Ser Asp Thr
    50                  55

<210> 69
<211> 61
<212> PRT
<213> Homo sapiens

<400> 69

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn
    50                  55                  60

<210> 70
<211> 58
<212> PRT
<213> Homo sapiens

<400> 70

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Ser Tyr
            20                  25                  30

Ala Met Asn Trp Val Arg Gln Ala Pro Gly Gln Gly Leu Glu Trp Met
        35                  40                  45

Gly Trp Ile Asn Thr Asn Thr Gly Asn Pro
    50                  55

<210> 71
<211>  60
<212>  PRT
<213>  Mus musculus

<400> 71

Ser Tyr Asn Pro Ser Leu Lys Ser Arg Ile Ser Ile Thr Arg Asp Thr
1               5                   10                  15

Ser Lys Asn Gln Phe Phe Leu Gln Leu Asn Ser Val Thr Thr Glu Asp
            20                  25                  30

Thr Ala Thr Tyr Tyr Cys Ala Ser Phe Asp Tyr Ala His Ala Met Asp
        35                  40                  45

Tyr Trp Gly Gln Gly Thr Ser Val Thr Val Ser Ser
    50                  55                  60

<210> 72
<211>  70
<212>  PRT
<213>  Homo sapiens

<220>
<221>  misc_feature
<222>  (14)..(14)
<223>  Xaa can be any naturally occurring amino acid

<220>
<221>  misc_feature
<222>  (16)..(16)
<223>  Xaa can be any naturally occurring amino acid

<220>
<221>  misc_feature
<222>  (31)..(31)
<223>  Xaa can be any naturally occurring amino acid

<220>
<221>  misc_feature
<222>  (41)..(53)
<223>  Xaa can be any naturally occurring amino acid

<220>
<221>  misc_feature
<222>  (55)..(56)
<223>  Xaa can be any naturally occurring amino acid

<220>

<221>  misc_feature
<222>  (59)..(59)
<223>  Xaa can be any naturally occurring amino acid

<400> 72

Asn Tyr Ala Gln Lys Phe Gln Gly Arg Val Thr Ile Thr Xaa Asp Xaa
1               5                   10                  15

Ser Thr Ser Thr Ala Tyr Met Glu Leu Ser Ser Leu Arg Ser Xaa Asp
            20                  25                  30

Thr Ala Val Tyr Tyr Cys Ala Arg Xaa Xaa Xaa Xaa Xaa Xaa Xaa Xaa
        35                  40                  45

Xaa Xaa Xaa Xaa Xaa Asp Xaa Xaa Phe Asp Xaa Trp Gly Gln Gly Thr
    50                  55                  60

Leu Val Thr Val Ser Ser
65                  70

<210> 73
<211>  70
<212>  PRT
<213>  Homo sapiens

<220>
<221>  misc_feature
<222>  (1)..(1)
<223>  Xaa can be any naturally occurring amino acid

<220>
<221>  misc_feature
<222>  (24)..(24)
<223>  Xaa can be any naturally occurring amino acid

<220>
<221>  misc_feature
<222>  (26)..(26)
<223>  Xaa can be any naturally occurring amino acid

<220>
<221>  misc_feature
<222>  (41)..(56)
<223>  Xaa can be any naturally occurring amino acid

<220>
<221>  misc_feature
<222>  (59)..(59)
<223>  Xaa can be any naturally occurring amino acid

<220>
<221>  misc_feature
<222>  (65)..(65)
<223>  Xaa can be any naturally occurring amino acid

<400> 73

Xaa Tyr Asn Pro Ser Leu Lys Ser Arg Val Thr Ile Ser Val Asp Thr
1               5                   10                  15

Ser Lys Asn Gln Phe Ser Leu Xaa Leu Xaa Ser Val Thr Ala Ala Asp
            20                  25                  30

Thr Ala Val Tyr Tyr Cys Ala Arg Xaa Xaa Xaa Xaa Xaa Xaa Xaa Xaa
        35                  40                  45

Xaa Xaa Xaa Xaa Xaa Xaa Xaa Xaa Phe Asp Xaa Trp Gly Gln Gly Thr
    50                  55                  60

Xaa Val Thr Val Ser Ser
65                  70

<210> 74
<211> 70
<212> PRT
<213> Homo sapiens

<220>
<221> misc_feature
<222> (40)..(52)
<223> Xaa can be any naturally occurring amino acid

<220>
<221> misc_feature
<222> (55)..(56)
<223> Xaa can be any naturally occurring amino acid

<220>
<221> misc_feature
<222> (59)..(59)
<223> Xaa can be any naturally occurring amino acid

<400> 74

Tyr Tyr Ala Asp Ser Val Lys Gly Arg Phe Thr Ile Ser Arg Asp Asn
1               5                   10                  15

Ser Lys Asn Thr Leu Tyr Leu Gln Met Asn Ser Leu Arg Ala Glu Asp
            20                  25                  30

Thr Ala Val Tyr Tyr Cys Ala Xaa Xaa Xaa Xaa Xaa Xaa Xaa Xaa Xaa
        35                  40                  45

Xaa Xaa Xaa Xaa Tyr Tyr Xaa Xaa Phe Asp Xaa Trp Gly Gln Gly Thr
    50                  55                  60

Leu Val Thr Val Ser Ser
65                  70

<210> 75
<211> 70
<212> PRT
<213> Homo sapiens

<220>
<221> misc_feature
<222> (41)..(55)
<223> Xaa can be any naturally occurring amino acid

<400> 75

Asn Tyr Ala Gln Lys Phe Gln Gly Arg Val Thr Met Thr Arg Asp Thr
1               5                   10                  15

Ser Ile Ser Thr Ala Tyr Met Glu Leu Ser Arg Leu Arg Ser Asp Asp
            20                  25                  30

Thr Ala Val Tyr Tyr Cys Ala Arg Xaa Xaa Xaa Xaa Xaa Xaa Xaa Xaa
        35                  40                  45

Xaa Xaa Xaa Xaa Xaa Xaa Xaa Tyr Phe Asp Tyr Trp Gly Gln Gly Thr
    50                  55                  60

Leu Val Thr Val Ser Ser
65                  70

<210> 76
<211> 70
<212> PRT
<213> Homo sapiens

<220>
<221> misc_feature
<222> (42)..(55)
<223> Xaa can be any naturally occurring amino acid

<400> 76

Arg Tyr Ser Pro Ser Leu Lys Ser Arg Leu Thr Ile Thr Lys Asp Thr
1               5                   10                  15

Ser Lys Asn Gln Val Val Leu Thr Met Thr Asn Met Asp Pro Val Asp
            20                  25                  30

Thr Ala Thr Tyr Tyr Cys Ala His Arg Xaa Xaa Xaa Xaa Xaa Xaa Xaa
        35                  40                  45

Xaa Xaa Xaa Xaa Xaa Xaa Xaa Tyr Phe Asp Tyr Trp Gly Gln Gly Thr
    50                  55                  60

Leu Val Thr Val Ser Ser
65                  70

<210> 77
<211>  70
<212>  PRT
<213>  Homo sapiens

<220>
<221>  misc_feature
<222>  (41)..(55)
<223>  Xaa can be any naturally occurring amino acid

<400> 77

Tyr Tyr Val Asp Ser Val Lys Gly Arg Phe Thr Ile Ser Arg Asp Asn
1               5                   10                  15

Ala Lys Asn Ser Leu Tyr Leu Gln Met Asn Ser Leu Arg Ala Glu Asp
            20                  25                  30

Thr Ala Val Tyr Tyr Cys Ala Arg Xaa Xaa Xaa Xaa Xaa Xaa Xaa Xaa
        35                  40                  45

Xaa Xaa Xaa Xaa Xaa Xaa Xaa Tyr Phe Asp Tyr Trp Gly Gln Gly Thr
    50                  55                  60

Leu Val Thr Val Ser Ser
65                  70

<210> 78
<211>  70
<212>  PRT
<213>  Homo sapiens

<220>
<221>  misc_feature
<222>  (41)..(55)
<223>  Xaa can be any naturally occurring amino acid

<400> 78

Asn Tyr Asn Pro Ser Leu Lys Ser Arg Val Thr Ile Ser Val Asp Lys
1               5                   10                  15

Ser Lys Asn Gln Phe Ser Leu Lys Leu Ser Ser Val Thr Ala Ala Asp
            20                  25                  30

Thr Ala Val Tyr Tyr Cys Ala Arg Xaa Xaa Xaa Xaa Xaa Xaa Xaa Xaa
        35                  40                  45

Xaa Xaa Xaa Xaa Xaa Xaa Xaa Tyr Phe Asp Tyr Trp Gly Gln Gly Thr

50                  55                  60

Leu Val Thr Val Ser Ser
65                  70

<210> 79
<211>  70
<212>  PRT
<213>  Homo sapiens

<220>
<221>  misc_feature
<222>  (41)..(55)
<223>  Xaa can be any naturally occurring amino acid

<400> 79

Arg Tyr Ser Pro Ser Phe Gln Gly Gln Val Thr Ile Ser Ala Asp Lys
1               5                   10                  15

Ser Ile Ser Thr Ala Tyr Leu Gln Trp Ser Ser Leu Lys Ala Ser Asp
            20                  25                  30

Thr Ala Met Tyr Tyr Cys Ala Arg Xaa Xaa Xaa Xaa Xaa Xaa Xaa Xaa
        35                  40                  45

Xaa Xaa Xaa Xaa Xaa Xaa Xaa Tyr Phe Asp Tyr Trp Gly Gln Gly Thr
    50                  55                  60

Leu Val Thr Val Ser Ser
65                  70

<210> 80
<211>  70
<212>  PRT
<213>  Homo sapiens

<220>
<221>  misc_feature
<222>  (41)..(55)
<223>  Xaa can be any naturally occurring amino acid

<400> 80

Asp Tyr Ala Val Ser Val Lys Ser Arg Ile Thr Ile Asn Pro Asp Thr
1               5                   10                  15

Ser Lys Asn Gln Phe Ser Leu Gln Leu Asn Ser Val Thr Pro Glu Asp
            20                  25                  30

Thr Ala Val Tyr Tyr Cys Ala Arg Xaa Xaa Xaa Xaa Xaa Xaa Xaa Xaa
        35                  40                  45

Xaa Xaa Xaa Xaa Xaa Xaa Xaa Tyr Phe Asp Tyr Trp Gly Gln Gly Thr
    50                  55                  60

Leu Val Thr Val Ser Ser
65                  70

<210> 81
<211>  70
<212>  PRT
<213>  Homo sapiens

<220>
<221>  misc_feature
<222>  (41)..(55)
<223>  Xaa can be any naturally occurring amino acid

<400> 81

Thr Tyr Ala Gln Gly Phe Thr Gly Arg Phe Val Phe Ser Leu Asp Thr
1               5                   10                  15

Ser Val Ser Thr Ala Tyr Leu Gln Ile Cys Ser Leu Lys Ala Glu Asp
            20                  25                  30

Thr Ala Val Tyr Tyr Cys Ala Arg Xaa Xaa Xaa Xaa Xaa Xaa Xaa Xaa
        35                  40                  45

Xaa Xaa Xaa Xaa Xaa Xaa Xaa Tyr Phe Asp Tyr Trp Gly Gln Gly Thr
    50                  55                  60

Leu Val Thr Val Ser Ser
65                  70

<210> 82
<211>  1404
<212>  DNA
<213>  Homo sapiens

<400> 82
atgggatgga gttgcattat acttttcctc gttgccaccg ccactggagt tcactctgac      60

gtacaacttc aagaatctgg cccaggtctc gtcaaacctt ctcaaactct ctcactcacc     120

tgcactgtta ctgactactc tattacatcc gactacgctt ggaactggat ccgacaattt     180

cctggtaaaa aactcgaatg gatgggttat atttcttact ctggctccac ctcctacaat     240

ccttctctga aatcacgcat cacaatttcc cgcgatacct ctaaaaatca attttcactc     300

caactcaatt ctgttaccgc cgccgatact gccacctact actgtgcctc ttttgactac     360

gctcacgcca tggattattg gggacagggt actaccgtta ccgtaagctc agccagcaca     420

aagggcccat cggtcttccc cctggcaccc tcctccaaga gcacctctgg gggcacagcg 480

gccctgggct gcctggtcaa ggactacttc cccgaaccgg tgacggtgtc gtggaactca 540

ggcgccctga ccagcggcgt gcacaccttc ccggctgtcc tacagtcctc aggactctac 600

tccctcagca gcgtggtgac cgtgccctcc agcagcttgg gcacccagac ctacatctgc 660

aacgtgaatc acaagcccag caacaccaag gtggacaaga gagttgagcc caaatcttgt 720

gacaaaactc acacatgtcc accgtgccca gcacctgaac tcctgggggg accgtcagtc 780

ttcctcttcc ccccaaaacc caaggacacc ctcatgatct cccggacccc tgaggtcaca 840

tgcgtggtgg tggacgtgag ccacgaagac cctgaggtca agttcaactg gtacgtggac 900

ggcgtggagg tgcataatgc caagacaaag ccgcgggagg agcagtacaa cagcacgtac 960

cgtgtggtca gcgtcctcac cgtcctgcac caggactggc tgaatggcaa ggagtacaag 1020

tgcaaggtct ccaacaaagc cctcccagcc cccatcgaga aaaccatctc caaagccaaa 1080

gggcagcccc gagaaccaca ggtgtacacc ctgcccccat cccgggagga gatgaccaag 1140

aaccaggtca gcctgacctg cctggtcaaa ggcttctatc ccagcgacat cgccgtggag 1200

tgggagagca atgggcagcc ggagaacaac tacaagacca cgcctcccgt gctggactcc 1260

gacggctcct tcttcctcta tagcaagctc accgtggaca agagcaggtg gcagcagggg 1320

aacgtcttct catgctccgt gatgcatgag gctctgcaca accactacac gcagaagagc 1380

ctctccctgt ccccgggtaa atga 1404

<210> 83
<211> 467
<212> PRT
<213> Homo sapiens

<400> 83

Met Gly Trp Ser Cys Ile Ile Leu Phe Leu Val Ala Thr Ala Thr Gly
1               5                   10                  15

Val His Ser Asp Val Gln Leu Gln Glu Ser Gly Pro Gly Leu Val Lys
            20                  25                  30

Pro Ser Gln Thr Leu Ser Leu Thr Cys Thr Val Thr Asp Tyr Ser Ile
        35                  40                  45

Thr Ser Asp Tyr Ala Trp Asn Trp Ile Arg Gln Phe Pro Gly Lys Lys
    50                  55                  60

Leu Glu Trp Met Gly Tyr Ile Ser Tyr Ser Gly Ser Thr Ser Tyr Asn
65                  70                  75                  80

Pro Ser Leu Lys Ser Arg Ile Thr Ile Ser Arg Asp Thr Ser Lys Asn

85                  90                  95

Gln Phe Ser Leu Gln Leu Asn Ser Val Thr Ala Ala Asp Thr Ala Thr
            100                 105                 110

Tyr Tyr Cys Ala Ser Phe Asp Tyr Ala His Ala Met Asp Tyr Trp Gly
        115                 120                 125

Gln Gly Thr Thr Val Thr Val Ser Ser Ala Ser Thr Lys Gly Pro Ser
    130                 135                 140

Val Phe Pro Leu Ala Pro Ser Ser Lys Ser Thr Ser Gly Gly Thr Ala
145                 150                 155                 160

Ala Leu Gly Cys Leu Val Lys Asp Tyr Phe Pro Glu Pro Val Thr Val
                165                 170                 175

Ser Trp Asn Ser Gly Ala Leu Thr Ser Gly Val His Thr Phe Pro Ala
            180                 185                 190

Val Leu Gln Ser Ser Gly Leu Tyr Ser Leu Ser Ser Val Val Thr Val
        195                 200                 205

Pro Ser Ser Ser Leu Gly Thr Gln Thr Tyr Ile Cys Asn Val Asn His
    210                 215                 220

Lys Pro Ser Asn Thr Lys Val Asp Lys Arg Val Glu Pro Lys Ser Cys
225                 230                 235                 240

Asp Lys Thr His Thr Cys Pro Pro Cys Pro Ala Pro Glu Leu Leu Gly
                245                 250                 255

Gly Pro Ser Val Phe Leu Phe Pro Pro Lys Pro Lys Asp Thr Leu Met
            260                 265                 270

Ile Ser Arg Thr Pro Glu Val Thr Cys Val Val Val Asp Val Ser His
        275                 280                 285

Glu Asp Pro Glu Val Lys Phe Asn Trp Tyr Val Asp Gly Val Glu Val
    290                 295                 300

His Asn Ala Lys Thr Lys Pro Arg Glu Glu Gln Tyr Asn Ser Thr Tyr
305                 310                 315                 320

Arg Val Val Ser Val Leu Thr Val Leu His Gln Asp Trp Leu Asn Gly
                325                 330                 335

Lys Glu Tyr Lys Cys Lys Val Ser Asn Lys Ala Leu Pro Ala Pro Ile
            340                 345                 350

Glu Lys Thr Ile Ser Lys Ala Lys Gly Gln Pro Arg Glu Pro Gln Val
        355                 360                 365

Tyr Thr Leu Pro Pro Ser Arg Glu Glu Met Thr Lys Asn Gln Val Ser
    370                 375                 380

Leu Thr Cys Leu Val Lys Gly Phe Tyr Pro Ser Asp Ile Ala Val Glu
385                 390                 395                 400

Trp Glu Ser Asn Gly Gln Pro Glu Asn Asn Tyr Lys Thr Thr Pro Pro
                405                 410                 415

Val Leu Asp Ser Asp Gly Ser Phe Phe Leu Tyr Ser Lys Leu Thr Val
            420                 425                 430

Asp Lys Ser Arg Trp Gln Gln Gly Asn Val Phe Ser Cys Ser Val Met
        435                 440                 445

His Glu Ala Leu His Asn His Tyr Thr Gln Lys Ser Leu Ser Leu Ser
    450                 455                 460

Pro Gly Lys
465

<210> 84
<211> 3985
<212> DNA
<213> Homo sapiens

<220>
<221> CDS
<222> (293)..(3211)

<220>
<221> sig_peptide
<222> (293)..(349)

<220>
<221> mat_peptide
<222> (350)..(3208)

<220>
<221> misc_feature
<222> (374)..(598)
<223> Immunoglobulin

<220>
<221> misc_feature
<222> (917)..(1177)
<223> Immunoglobulin

<220>
<221>  misc_feature
<222>  (2516)..(3022)
<223>  Tyrosine Kinase, Catalytic Domain

<400> 84
gaagggcaga cagagtgtcc aaaagcgtga gagcacgaag tgaggagaag gtggagaaga      60
gagaagagga agaggaagag gaagagagga agcggaggga actgcggcca ggctaaaagg     120
ggaagaagag gatcagccca aggaggagga agaggaaaac aagacaaaca gccagtgcag     180
aggagaggaa cgtgtgtcca gtgtcccgat ccctgcggag ctagtagctg agagctctgt     240
gccctgggca ccttgcagcc ctgcacctgc ctgccacttc cccaccgagg cc atg ggc     298
cca gga gtt ctg ctg ctc ctg ctg gtg gcc aca gct tgg cat ggt cag       346
gga atc cca gtg ata gag ccc agt gtc cct gag ctg gtc gtg aag cca       394
gga gca acg gtg acc ttg cga tgt gtg ggc aat ggc agc gtg gaa tgg       442
gat ggc ccc cca tca cct cac tgg acc ctg tac tct gat ggc tcc agc       490
agc atc ctc agc acc aac aac gct acc ttc caa aac acg ggg acc tat       538
cgc tgc act gag cct gga gac ccc ctg gga ggc agc gcc gcc atc cac       586
ctc tat gtc aaa gac cct gcc cgg ccc tgg aac gtg cta gca cag gag       634
gtg gtc gtg ttc gag gac cag gac gca cta ctg ccc tgt ctg ctc aca       682
gac ccg gtg ctg gaa gca ggc gtc tcg ctg gtg cgt gtg cgt ggc cgg       730
ccc ctc atg cgc cac acc aac tac tcc ttc tcg ccc tgg cat ggc ttc       778
acc atc cac agg gcc aag ttc att cag agc cag gac tat caa tgc agt       826
gcc ctg atg ggt ggc agg aag gtg atg tcc atc agc atc cgg ctg aaa 874
gtg cag aaa gtc atc cca ggg ccc cca gcc ttg aca ctg gtg cct gca 922
gag ctg gtg cgg att cga ggg gag gct gcc cag atc gtg tgc tca gcc 970
agc agc gtt gat gtt aac ttt gat gtc ttc ctc caa cac aac aac acc 1018
aag ctc gca atc cct caa caa tct gac ttt cat aat aac cgt tac caa 1066
aaa gtc ctg acc ctc aac ctc gat caa gta gat ttc caa cat gcc ggc 1114
aac tac tcc tgc gtg gcc agc aac gtg cag ggc aag cac tcc acc tcc 1162
atg ttc ttc cgg gtg gta gag agt gcc tac ttg aac ttg agc tct gag 1210
cag aac ctc atc cag gag gtg acc gtg ggg gag ggg ctc aac ctc aaa 1258
gtc atg gtg gag gcc tac cca ggc ctg caa ggt ttt aac tgg acc tac 1306
ctg gga ccc ttt tct gac cac cag cct gag ccc aag ctt gct aat gct 1354
acc acc aag gac aca tac agg cac acc ttc acc ctc tct ctg ccc cgc 1402
ctg aag ccc tct gag gct ggc cgc tac tcc ttc ctg gcc aga aac cca 1450
gga ggc tgg aga gct ctg acg ttt gag ctc acc ctt cga tac ccc cca 1498
gag gta agc gtc ata tgg aca ttc atc aac ggc tct ggc acc ctt ttg 1546
tgt gct gcc tct ggg tac ccc cag ccc aac gtg aca tgg ctg cag tgc 1594
agt ggc cac act gat agg tgt gat gag gcc caa gtg ctg cag gtc tgg      1642
gat gac cca tac cct gag gtc ctg agc cag gag ccc ttc cac aag gtg      1690
acg gtg cag agc ctg ctg act gtt gag acc tta gag cac aac caa acc      1738
tac gag tgc agg gcc cac aac agc gtg ggg agt ggc tcc tgg gcc ttc      1786
ata ccc atc tct gca gga gcc cac acg cat ccc ccg gat gag ttc ctc      1834
ttc aca cca gtg gtg gtc gcc tgc atg tcc atc atg gcc ttg ctg ctg      1882
ctg ctg ctc ctg ctg cta ttg tac aag tat aag cag aag ccc aag tac      1930
cag gtc cgc tgg aag atc atc gag agc tat gag ggc aac agt tat act      1978
ttc atc gac ccc acg cag ctg cct tac aac gag aag tgg gag ttc ccc      2026
cgg aac aac ctg cag ttt ggt aag acc ctc gga gct gga gcc ttt ggg      2074
aag gtg gtg gag gcc acg gcc ttt ggt ctg ggc aag gag gat gct gtc      2122
ctg aag gtg gct gtg aag atg ctg aag tcc acg gcc cat gct gat gag      2170
aag gag gcc ctc atg tcc gag ctg aag atc atg agc cac ctg ggc cag      2218
cac gag aac atc gtc aac ctt ctg gga gcc tgt acc cat gga ggc cct      2266
gta ctg gtc atc acg gag tac tgt tgc tat ggc gac ctg ctc aac ttt      2314
ctg cga agg aag gct gag gcc atg ctg gga ccc agc ctg agc ccc ggc      2362
cag gac ccc gag gga ggc gtc gac tat aag aac atc cac ctc gag aag      2410
aaa tat gtc cgc agg gac agt ggc ttc tcc agc cag ggt gtg gac acc      2458
tat gtg gag atg agg cct gtc tcc act tct tca aat gac tcc ttc tct      2506
gag caa gac ctg gac aag gag gat gga cgg ccc ctg gag ctc cgg gac      2554
ctg ctt cac ttc tcc agc caa gta gcc cag ggc atg gcc ttc ctc gct      2602
tcc aag aat tgc atc cac cgg gac gtg gca gcg cgt aac gtg ctg ttg      2650
acc aat ggt cat gtg gcc aag att ggg gac ttc ggg ctg gct agg gac      2698
atc atg aat gac tcc aac tac att gtc aag ggc aat gcc cgc ctg cct      2746
gtg aag tgg atg gcc cca gag agc atc ttt gac tgt gtc tac acg gtt      2794
cag agc gac gtc tgg tcc tat ggc atc ctc ctc tgg gag atc ttc tca      2842
ctt ggg ctg aat ccc tac cct ggc atc ctg gtg aac agc aag ttc tat      2890
aaa ctg gtg aag gat gga tac caa atg gcc cag cct gca ttt gcc cca      2938
aag aat ata tac agc atc atg cag gcc tgc tgg gcc ttg gag ccc acc      2986
cac aga ccc acc ttc cag cag atc tgc tcc ttc ctt cag gag cag gcc      3034
caa gag gac agg aga gag cgg gac tat acc aat ctg ccg agc agc agc      3082
aga agc ggt ggc agc ggc agc agc agc agt gag ctg gag gag gag agc     3130
tct agt gag cac ctg acc tgc tgc gag caa ggg gat atc gcc cag ccc     3178
ttg ctg cag ccc aac aac tat cag ttc tgc tga ggagttgacg acagggagta     3231
ccactctccc ctcctccaaa cttcaactcc tccatggatg gggcgacacg gggagaacat     3291
acaaactctg ccttcggtca tttcactcaa cagctcggcc cagctctgaa acttgggaag     3351
gtgagggatt caggggaggt cagaggatcc cacttcctga gcatgggcca tcactgccag     3411
tcaggggctg ggggctgagc cctcaccccc ccctccccta ctgttctcat ggtgttggcc     3471
tcgtgtttgc tatgccaact agtagaacct tctttcctaa tccccttatc ttcatggaaa     3531
tggactgact ttatgcctat gaagtcccca ggagctacac tgatactgag aaaaccaggc     3591
tctttggggc tagacagact ggcagagagt gagatctccc tctctgagag gagcagcaga     3651
tgctcacaga ccacactcag ctcaggcccc ttggagcagg atggctcctc taagaatctc     3711
acaggacctc ttagtctctg ccctatacgc cgccttcact ccacagcctc acccctccca     3771
cccccatact ggtactgctg taatgagcca agtggcagct aaaagttggg ggtgttctgc     3831
ccagtcccgt cattctgggc tagaaggcag gggaccttgg catgtggctg gccacaccaa     3891
gcaggaagca caaactcccc caagctgact catcctaact aacagtcacg ccgtgggatg     3951
tctctgtcca cattaaacta acagcattaa tgca                                 3985

<210>  85
<211>  972
<212>  PRT
<213>  Homo sapiens

<400>  85

<210> 86
<211>  327
<212>  DNA
<213>  Homo sapiens

<400> 86
gaaatagtcc ttacccaatc tcccggaacc ctctcagtat ctcccggcga acgagtaacc      60

ttttcatgta gagcatccca atccatcggc acttcaattc actggtatca gcagaaaaca     120

ggtcaatccc cacggcttct tataaaatat gcatcagaat caatttctgg catcccagac     180

agattttcag gttcaggatc aggcaccgat ttcacactta caatatccag agtcgaatca     240

gaagattttg cagattacta ttgtcaacaa ataaacagct ggcccactac attcggacaa     300

ggcacaaaac tcgaaattaa acgtacg                                         327

<210> 87
<211>  109
<212>  PRT
<213>  Homo sapiens

<400> 87

Glu Ile Val Leu Thr Gln Ser Pro Gly Thr Leu Ser Val Ser Pro Gly
1               5                   10                  15

Glu Arg Val Thr Phe Ser Cys Arg Ala Ser Gln Ser Ile Gly Thr Ser
            20                  25                  30

Ile His Trp Tyr Gln Gln Lys Thr Gly Gln Ser Pro Arg Leu Leu Ile
        35                  40                  45

Lys Tyr Ala Ser Glu Ser Ile Ser Gly Ile Pro Asp Arg Phe Ser Gly
    50                  55                  60

Ser Gly Ser Gly Thr Asp Phe Thr Leu Thr Ile Ser Arg Val Glu Ser
65                  70                  75                  80

Glu Asp Phe Ala Asp Tyr Tyr Cys Gln Gln Ile Asn Ser Trp Pro Thr
                85                  90                  95

Thr Phe Gly Gln Gly Thr Lys Leu Glu Ile Lys Arg Thr
            100                 105

<210> 88
<211> 327
<212> DNA
<213> Homo sapiens

<400> 88
gaaatagttc ttactcaatc ccccggtaca ctctcagttt ccccaggcga acgcgtcact      60

ttttcttgca gagcatcaca atcaatcggc acttcaattc attggtatca acaaaaaaca     120

ggacaggccc cacgacttct tattaaatat gcatcagaat caatttctgg catcccagac     180

agattttcag gttcaggatc aggcaccgat ttcacactta caatatccag agtcgaatca     240

gaagattttg cagattacta ttgtcaacaa ataaacagct ggcccactac attcggacaa     300

ggcacaaaac tcgaaattaa acgtacg                                         327

## Description

### TECHNICAL FIELD

This invention relates to methods for preventing and treating osteolysitic diseases, including cancer metastasis and bone loss associated with cancer metastasis by administering an M-CSF-antagonist in combination with another therapeutic agent to a subject.

### BACKGROUND OF THE INVENTION

Osteoclasts, which mediate bone resorption, are involved in normal and abnormal bone remodeling processes, including osteolytic diseases. Osteoclasts are multinucleated cells differentiating from haemopoietic cells. It is generally accepted that osteoclasts are formed by the fusion of mononuclear precursors derived from haemopoietic stem cells in the bone marrow, rather than incomplete cell divisions (Chambers, Bone and Mineral Research, 6: 1- 25, 1989; Göthling et al., Clin Orthop Relat R. 120: 201-228, 1976; Kahn et al., Nature 258: 325-327, 1975, Suda et al., Endocr Rev 13: 66-80, 1992; Walker, Science 180: 875, 1973; Walker, Science 190: 785-787, 1975; Walker, Science 190: 784-785, 1975). They share a common stem cell with monocyte-macrophage lineage cells (Ash et al., Nature 283: 669-670, 1980, Kerby et al., J. Bone Miner Res 7: 353-62, 1992). The differentiation of osteoclast precursors into mature multinucleated osteoclasts requires different factors including hormonal and local stimuli (Athanasou et al., Bone Miner 3: 317-333,1988; Feldman et al., Endocrinology 107: 1137-1143, 1980; Walker, Science 190: 784-785, 1975; Zheng et al., Histochem J 23: 180-188, 1991) and living bone and bone cells have been shown to play a critical role in osteoclast development (Hagenaars et al., Bone Miner 6: 179-189, 1989). Osteoblastic or bone marrow stromal cells are also required for osteoclast differentiation. One of the factors produced by these cells that supports osteoclast formation is macrophage-colony stimulating factor, M-CSF (Wiktor-Jedrzejczak et al., Proc Natl Acad Sci USA 87: 4828-4832, 1990; Yoshida et al., Nature 345: 442-444, 1990). Receptor activator for NF-κ B ligand (RANKL, also known as TRANCE, ODF and OPGL) is another signal (Suda et al., Endocr Rev 13: 66-80, 1992) through which osteoblastic/stromal cells stimulate osteoclast formation and resorption via a receptor, RANK (TRANCER), located on osteoclasts and osteoclast precursors (Lacey et al., Cell 93: 165-176, 1998; Tsuda et al., Biochem Biophys Res Co 234: 137-142, 1997; Wong et al., J Exp Med 186: 2075-2080, 1997; Wong et al., J Biol. Chem 272: 25190-25194, 1997; Yasuda et al., Endocrinology 139: 1329-1337, 1998; Yasuda et al., Proc Natl Acad'Sci US 95: 3597-3602, 1998). Osteoblasts also secrete a protein that strongly inhibits osteoclast formation called osteoprotegerin (OPG, also known as OCIF), which acts as a decoy receptor for the RANKL thus inhibiting the positive signal between osteoclasts and osteoblasts via RANK and RANKL.

Osteoclasts are responsible for dissolving both the mineral and organic bone matrix (Blair et al., J Cell Biol 102: 1164-1172, 1986). Osteoclasts represent terminally differentiated cells expressing a unique polarized morphology with specialized membrane areas and several membrane and cytoplasmic markers, such as tartrate resistant acid phosphatase (TRAP) (Anderson et al. 1979), carbonic anhydrase II (Väänänen et al., Histochemistry 78: 481-485, 1983), calcitonin receptor (Warshafsky et al., Bone 6: 179-185, 1985) and vitronectin receptor (Davies et al., J Cell Biol 109: 1817-1826, 1989). Multinucleated osteoclasts usually contain less than 10 nuclei, but they may contain up to 100 nuclei being between 10 and 100 µm in diameter (Göthling et al., Clin Orthop Relat R 120: 201-228, 1976). This makes them relatively easy to identify by light microscopy. They are highly vacuolated when in the active state, and also contain many mitochondria, indicative of a high metabolic rate (Mundy, in Primer on the metabolic bone diseases and disorders of mineral metabolism, pages 18-22, 1990). Since osteoclasts play a major role in osteolytic bone metastases, there is a need in the art for new agents and methods for preventing osteoclast stimulation and function.

Cancer metastasis is the primary cause of post-operation or post-therapy recurrence in cancer patients. Despite intensive efforts to develop treatments, cancer metastasis remains substantially refractory to therapy. Bone is one of the most common sites of metastasis of various types of human cancers (e.g., breast, lung, prostate and thyroid cancers). The occurrence of osteolytic bone metastases causes serious morbidity due to intractable pain, high susceptibility to fracture, nerve compression and hypercalcemia. Despite the importance of these clinical problems, there are few available treatments for bone loss associated with cancer metastasis.
Several therapeutic strategies targeting osteolytic disease are currently being used or under development, where efforts have mainly focused on the development of drugs to block bone resorption through inhibiting the formation or activity of osteoclasts. The bisphosphonates (BPs), pyrophosphate analogs that concentrate in bone, are to date the most effective inhibitor of bone resorption. BPs are taken up by osteoclasts, inhibiting their activity and causing the cells to undergo apoptosis, thereby inhibiting bone resorption. Alendronate was the first BP inhibitor of bone resorption to show a significant reduction in spine/hip fractures, and is approved for treatment of osteoporosis. The latest generation BP, Zometa, is approved for treatment of hypercalcemia and bone disease in solid tumors and multiple myeloma and is under investigation for possible treatment of Paget's disease and bone metastasis resulting from solid tumors and multiple myeloma. Zometa acts at very low doses, and is given as a15 minute i.v. infusion once a month, but also affects osteoblasts and may cause side effects such as renal toxicity and osteonecrosis of the jaw (Fromigue and Body, J, Endocrinol. Invest. 25:39-46, 2002; Ibrahim, A. et al., Clin. Canc. Res. 9:2394-99, 2003; Body, J.J., The Breast. S2:S37-44, 2003; Yaccoby, S. et al., Brit. J. Hemat., 116:278-80, 2002; Corey, E. et al., Clin. Canc. Res. 9: 295-306, 2003; Coleman, R.E., Sem. Oncol., 29(6): 43-49, 2002; Coleman, R.E., Eur. Soc. Med. Oncol. 16:687-95, 2005; Bamias et al., J Clin Oncol 13: 8580-8587, 2005. Thus, there remains a need in the art to identify new agents and methods for preventing or treating osteolytic diseases and/or cancer metastasis, including osteolytic bone metastases.

### SUMMARY OF THE INVENTION

The compositions and methods of the present invention fulfill the aforementioned and other related needs in the art. In one embodiment of the invention, a method is provided for treating a subject suffering from or at risk of an osteolytic disorder comprising administering to the subject a monotherapeutically amount of a M-CSF antagonist and a monotherapeutically effective amount of a second anti-osteoclast agent for a transition period of about 1 day to a year, during which the M-CSF antagonist reduces the number of active osteoclasts to a therapeutically desirable level. Exemplary M-CSF antagonists include M-CSF antibodies and exemplary second anti-osteoclast agents include bisphosphonates and RANKL inhibitors, including anti-RANKL antibodies. The methods and/or uses involving anti-M-CSF antibody and osteoclast inhibitor herein optionally exclude the use of RX1, 5H4, MC1 and MC3-derived antibodies disclosed in International Publication No. WO 2005/068503. The duration of the transition period may be, for example, at least one day up to one year, and may be monitored, e.g., by relevant markers of osteoclast growth or activity. Alternatively, they may be given simultaneously.

By way of example, markers of bone formation include but are not limited to calcium, and total and bone-specific alkaline phosphatase (BAP), osteocalcin (OC, bone gla-protein), Procollagen type I C propeptide (PICP), Procollagen type I N propeptide (PINP), and markers of bone resorption include but are not limited to NTX (N-terminal cross-linking telopeptide of bone collagen) and CTX (C-terminal cross-linking telopeptide of bone collagen), pyridinium crosslinks (pyridinoline and deoxypyridinoline [DPD]) and associated peptides, bone type I collagen degradation products hydroxyproline and hydroxylysine glycosides, tartrate-resistant acid phosphatase(TRACP), and bone sialoprotein (BSP). See Fohr et al., J. Clin. Endocrinol. Metab., November 2003, 88(11):5059-5075.

In related embodiments, the aforementioned methods are provided wherein the second anti-osteoclast agent is discontinued after the transition period. In other related embodiments, the aforementioned methods are provided wherein the amount of the second anti-osteoclast agent is reduced after the transition period. In further related embodiments, the aforementioned methods are provided wherein the amount of M-CSF antagonist is reduced after the transition period.

It is contemplated that the methods of the instant invention achieve their therapeutic potential by inhibiting the interaction between M-CSF and its receptor (M-CSFR). It is further contemplated that the inhibition of M-CSF/M-CSFR interaction inhibits osteoclast proliferation and/or differentiation. In any of the methods or compositions of the invention, the M-CSF antagonist may be a polypeptide comprising an anti-M-CSF antibody; a polypeptide comprising an anti-M-CSFR antibody; a soluble polypeptide comprising an M-CSF mutein or derivative thereof; or a soluble polypeptide comprising an M-CSFR mutein or derivative thereof; or a nucleic acid molecule that inhibits the expression of M-CSF or M-CSFR. The identification, production and modification of various M-CSF antagonists is described in Int'l Publication No. WO 2005/068503, hereby incorporated by reference in its entirety.

The M-CSF antibody may be a polyclonal antibody; a monoclonal antibody; a humanized antibody; a human antibody; a human engineered antibody; a chimeric antibody; Fab, F(ab')₂ or Fᵥ antibody fragment; or a mutein of any one of the aforementioned antibodies.

M-CSF antibodies of the instant invention that inhibit osteolysis are described in Int'l Publication No. WO 2005/068503, which is hereby incorporated by reference in its entirety for its teaching with respect to M-CSF antibodies.

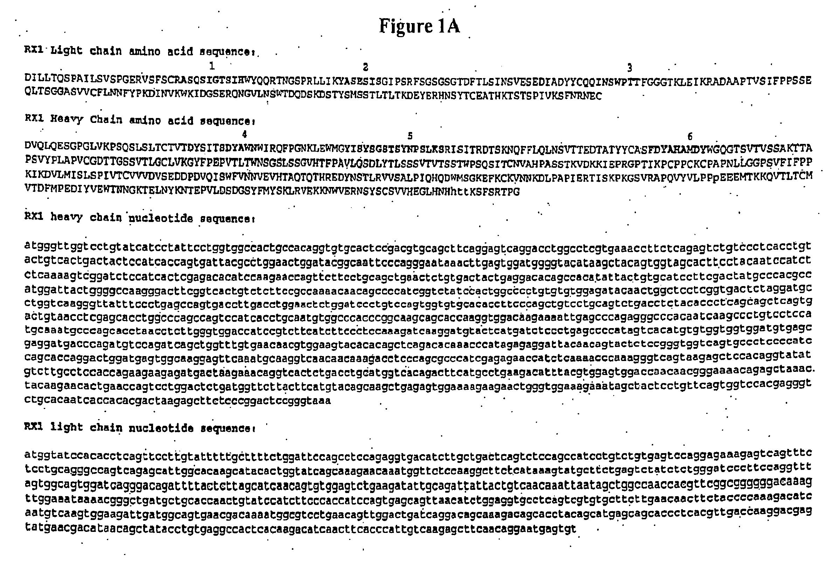

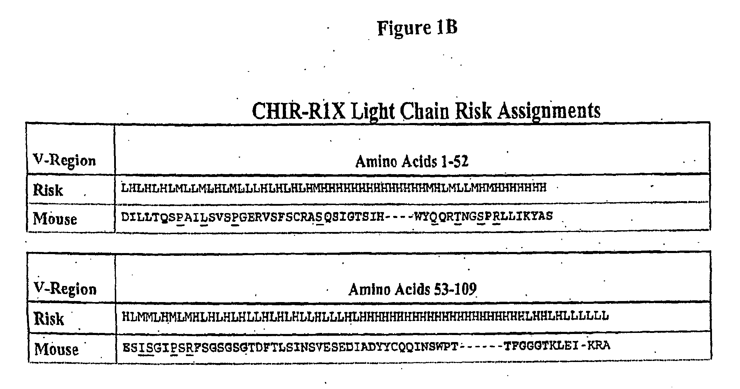

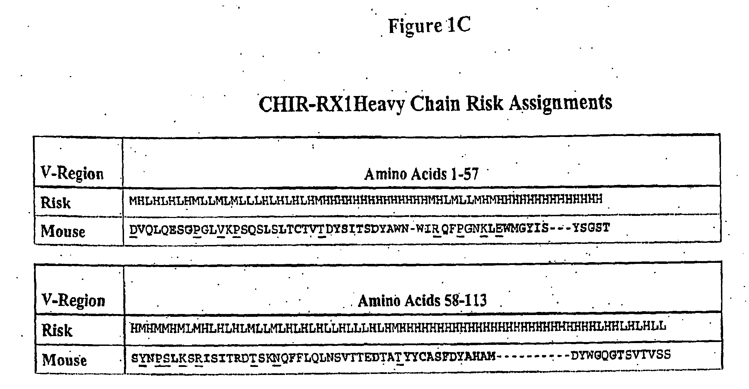

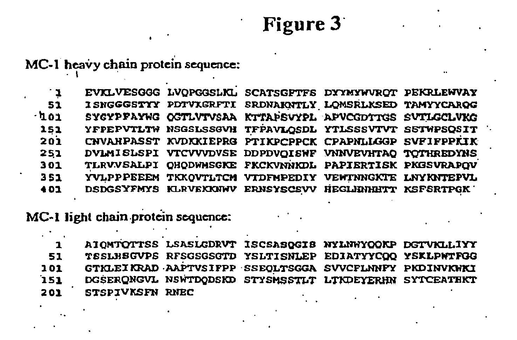

In one embodiment of the invention, a non-murine monoclonal antibody is provided, including functional fragment, that specifically binds to the same epitope of M-CSF as anyone of murine monoclonal antibody RX1, MC1, or M C3 having the amino acid sequences set forth in Figures 1, 3, and 4, respectively. In a related embodiment, an aformentioned antibody is provided wherein the antibody is selected from the group consisting of a polyclonal antibody; a monoclonal antibody including a Human Engineered™ antibody; a humanized antibody; a human antibody; a chimeric antibody; Fab, F(ab')2; Fv; Sc Fv or SCA antibody fragment; a diabody; linear antibody; or a mutein of any one of these antibodies, that preferably retain binding affinity of at least 10⁻⁷, 10⁻⁸ or 10⁻⁹ or higher. A non-murine monoclonal antibody, including functional fragment, that competes with monoclonal antibody RX1, MC1, and/or MC3 having the amino acid sequence set forth in Figure 1 for binding to M-CSF by more than 75%, is also contemplated.

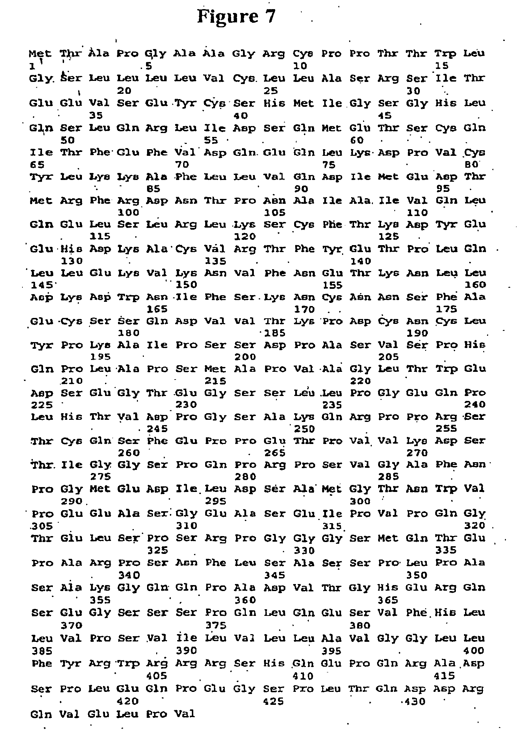

In another embodiment, a non-murine monoclonal antibody, including functional fragment, wherein said non-murine monoclonal antibody or functional fragment thereofbinds an epitope of M-CSF that includes at least 4, 5, 6, 7 or 8 contiguous residues of amino acids 98-105 of Figure 7 is provided.

In another embodiment, the invention provides a non-murine monoclonal antibody, including functional fragment, wherein said non-murine monoclonal antibody or functional fragment thereofbinds an epitope ofM-CSF that includes at least 4, 5, 6, 7 or 8 contiguous residues of amino acids 65-73 or 138-144 of Figure 7 (corresponding to M-CSF epitopes recognized by 5H4 or MC3).

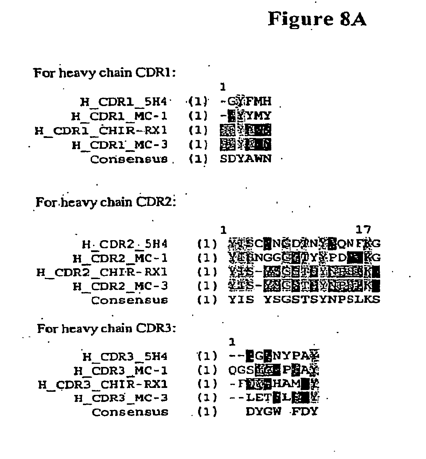

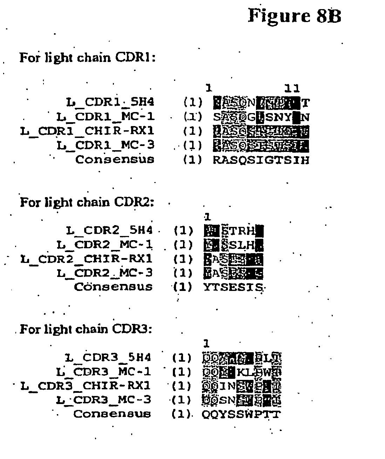

In yet another embodiment, the aforementioned antibody or fragment that binds an epitope of M-CSF that includes amino acids 98-105 of Figure 7 is provided. In a related embodiment, the aforementioned antibody is provided comprising CDR3 of Figure 1A. In another embodiment, the antibody is provided comprising at least 1, 2, 3, 4, 5, or 6 CDRs of murine antibody RX1 set forth in Figure 1A. Such an antibody that comprises at least 1, 2, 3, 4, or 5 CDRs of murine antibody RX1 may also comprise at least 1, 2, 3, 4, or 5 CDRs of any of the 6 CDRs of antibody 5H4 set forth in Figure 8A-B. Alternatively, the antibody that comprises at least 1, 2, 3, 4, or 5 CDRs of murine antibody RX1 may also comprise at least 1, 2, 3, 4, or 5 CDRs of any of the 6 CDRs of antibody MC1 set forth in Figure 8A-B. In yet another alternative, the aforementioned antibody may also comprise at least 1, 2, 3, 4, or 5 CDRs of any of the 6 CDRs of antibody MC3 set forth in Figure 8A-B. In a related embodiment, the antibody that comprises at least 1, 2, 3, 4, or 5 CDRs of murine antibody RX1 may comprise at least 1, 2, 3, 4 or 5 CDRs of the consensus CDRs set forth in Figure 8A-B is provided. In still another related embodiment, in the aforementioned antibody one or more residues of the consensus CDR(s) is substituted by the corresponding residue of any of the CDRs of antibody murine RX1, 5H4, MC1 or MC3. The desired binding affinity may be retained even though one or more of the amino acids in the antibody have been mutated, e.g. by conservative substitutions in the CDRs, and/or conservative or non-conservative changes in the low and moderate risk residues.

In another embodiment of the invention, variants of the aforementioned antibody are provided, comprising a variable heavy chain amino acid sequence which is at least 60, 65, 70, 75, 80, 85, 90, 91, 92, 93, 94, 95, 96, 97, 98, or 99% homologous to the amino acid sequence set forth in Figures 1A, 2, 3, or 4. In a related embodiment, the antibody comprises a variable light chain amino acid sequence which is at least 60,-65, 70, 75, 80, 85, 90, 91, 92, 93, 94, 95, 96, 97, 98, or 99% homologous to the amino acid sequence set forth in Figures 1A, 2, 3, or 4.

In yet another embodiment, the antibody comprises a constant region and one or more heavy and light chain variable framework regions of a human antibody sequence. In a related embodiment, the antibody comprises a modified or unmodified constant region of a human IgG1, IgG2, IgG3 or IgG4. In a preferred embodiment, the constant region is human IgG1 or IgG4, which may optionally be modified to enhance or decrease certain properties. In the case of IgG1, modifications to the constant region, particularly the hinge or CH2 region, may increase or decrease effector function, including ADCC and/or CDC activity. In other embodiments, an IgG2 constant region is modified to decrease antibody-antigen aggregate formation. In the case of IgG4, modifications to the constant region, particularly the hinge region, may reduce the formation of half-antibodies.

In one embodiment of the invention, a non-murine monoclonal antibody is provided that specifically binds to the same epitope of M-CSF as any one of the murine antibodies RX1, 5H4, MC1 or MC3 as described in Int'l Publication No. WO 2005/068503, or competes with any one of the aforementioned murine antibodies for binding to M-CSF by more than 10%, more preferably by more than 25%, still more preferably by more than 50%, even more preferably by more than 75%, and most preferably more than 90%. Antibodies derived from the sequences of such murine antibodies, including chimeric, human, humanized, human engineered antibodies, or fragments or muteins or chemically derivatized versions thereof, are described in WO 2005/068503.

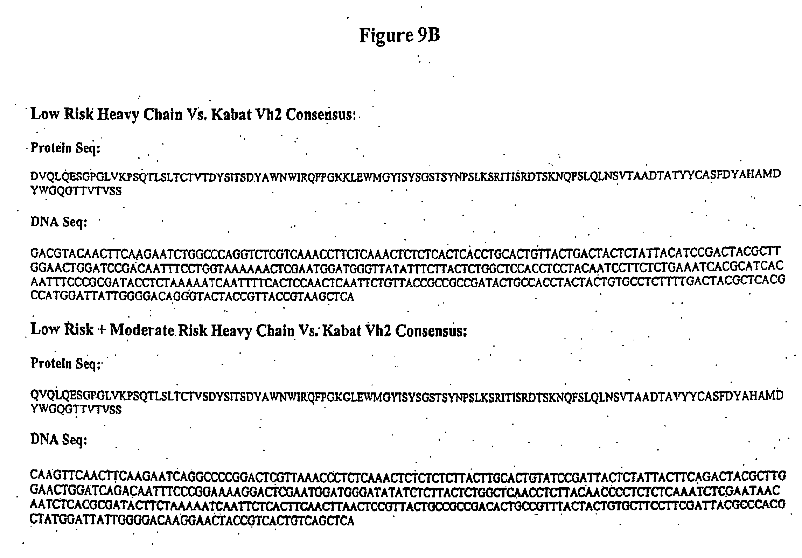

The term "RX 1-derived antibody" includes any one of the following:
1) an amino acid variant of murine antibody RX1 having the amino acid sequence set out in Figure 1, including variants comprising a variable heavy chain amino acid sequence which is at least 60, 65, 70, 75, 80, 85, 90, 91, 92, 93, 94, 95, 96, 97, 98, or 99% homologous to the amino acid sequence as set forth in Figure 1, and/or comprising a variable light chain amino acid sequence which is at least 60, 65, 70, 75, 80, 85, 90, 91, 92, 93, 94, 95, 96, 97, 98, or 99% homologous to the amino acid sequence as set forth in Figure 1, taking into account similar amino acids for the homology determination;
2) M-CSF-binding polypeptides (excluding murine antibody RX 1) comprising one or more complementary determining regions (CDRs) of murine antibody RX 1 having the amino acid sequence set out in Figure 1, preferably comprising at least CDR3 of the RX 1 heavy chain, and preferably comprising two or more, or three or more, or four or more, or five or more, or all six CDRs;
3) Human Engineered™ antibodies having the heavy and light chain amino acid sequences set out in Figures 9B through 12B or variants thereof comprising a heavy or light chain having at least 60% amino acid sequence identity with the original Human Engineered™ heavy or the light chain of Figures 9B through 12B, more preferably at least 80%, more preferably at least 85%, more preferably at least 90%, and most preferably ai least 95%, including for example, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, and 100% identical;
4) M-CSF-binding polypeptides (excluding murine antibody RX1) comprising the high risk residues of one or more CDRs of the Human Engineered™ antibodies of Figures 9B through 12B, and preferably comprising high risk residues of two or more, or three or more, or four or more, or five or more, or all six CDRs;
5) Human Engineered™ antibodies or variants retaining the high risk amino acid residues set out in Figure 1B, and comprising one or more changes at the low or moderate risk residues set out in Figure 1B;
   for example, comprising one or more changes at a low risk residue and conservative substitutions at a moderate risk residue set out in Figure 1B, or
   for example, retaining the moderate and high risk amino acid residues set out in Figure 1B and comprising one or more changes at a low risk residue,
   where changes include insertions, deletions or substitutions and maybe conservative substitutions or may cause the engineered antibody to be closer in sequence to a human light chain or heavy chain sequence, a human germline light chain or heavy chain sequence, a consensus human light chain or heavy chain sequence, or a consensus human germline light chain or heavy chain sequence;
   that retain ability to bind M-CSF. Such antibodies preferably bind to M-CSF with an affinity of at least 10⁻⁷, 10⁻⁸ or 10⁻⁹ or higher and preferably neutralize the osteoclastogenesis inducing activity of M-CSF.

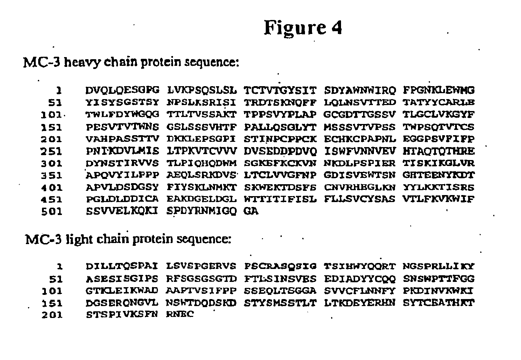

Similarly, the term "MC3-derived antibody" includes any one of the following:
1) an amino acid variant of murine antibody MC3 having the amino acid sequence set out in Figure 4, including variants comprising a variable heavy chain amino acid sequence which is at least 60, 65, 70, 75, 80, 85, 90, 91, 92, 93, 94, 95, 96, 97, 98, or 99% homologous to the amino acid sequence as set forth in Figure 4, and/or comprising a variable light chain amino acid sequence which is at least 60, 65, 70, 75, 80, 85, 90, 91, 92, 93, 94, 95, 96, 97, 98, or 99% homologous to the amino acid sequence as set forth in Figure 4, taking into account similar amino acids for the homology determination;
2) M-CSF-binding polypeptides (optionally including or excluding murine antibody MC3) comprising one or more complementary determining regions (CDRs) of murine antibody MC3 having the amino acid sequence set out in Figure 4, preferably comprising at least CDR3 of the MC3 heavy chain, and preferably comprising two or more, or three or more, or four or more, or five or more, or all six CDRs;
3) Human Engineered™ antibodies generated by altering the murine sequence according to the methods set forth in Studnicka et al., U.S. Patent No. 5,766,886 and Example 4A herein, using the Kabat numbering set forth in Figures 13C-13E to identify low, moderate and high risk residues; such antibodies comprising at least one of the following heavy chains and at least one of the following light chains: (a) a heavy chain in which all of the low risk residues have been modified, if necessary, to be the same residues as a human reference immunoglobulin sequence or (b) a heavy chain in which all of the low and moderate risk residues have been modified, if necessary, to be the same residues as a human reference immunoglobulin sequence, (c) a light chain in which all of the low risk residues have been modified, if necessary, to be the same residues as a human reference immunoglobulin sequence or (b) a light chain in which all of the low and moderate risk residues have been modified, if necessary, to be the same residues as a human reference immunoglobulin sequence
4) variants of the aforementioned antibodies in preceding paragraph (3) comprising a heavy or light chain having at least 60% amino acid sequence identity with the original Human Engineered™ heavy or the light chain, more preferably at least 80%, more preferably at least 85%, more preferably at least 90%, and most preferably at least 95%, including for example, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, and 100% identical;
5) M-CSF-binding polypeptides (optionally including or excluding murine antibody MC3) comprising the high risk residues of one or more CDRs of the murine MC3 antibody of Figure 4, and preferably comprising high risk residues of two or more, or three or more, or four or more, or five or more, or all six CDRs;
6) Human Engineered™ antibodies or variants retaining the high risk amino acid residues of murine MC3 antibody, and comprising one or more changes at the low or moderate risk residues;
   for example, comprising one or more changes at a low risk residue and conservative substitutions at a moderate risk residue, or
   for example, retaining the moderate and high risk amino acid residues and comprising one or more changes at a low risk residue,
   where changes include insertions, deletions or substitutions and may be conservative substitutions or may cause the engineered antibody to be close in sequence to a human light chain or heavy chain sequence, a human germline light chain or heavy chain sequence, a consensus human light chain or heavy chain sequence, or a consensus human germline light chain or heavy chain sequence;
that retain ability to bind M-CSF. Such antibodies preferably bind to M-CSF with an affinity of at least 10⁻⁷, 10⁻⁸ or 10⁻⁹ or higher and preferably neutralize the osteoclastogenesis inducing activity of M-CSF.

The term "5H4-derived antibody" or "MC1-derived antibody" is similarly defined according to the above description.

As described in detail herein, RX1, 5H4, MC1 or MC3-derived antibodies, including Human Engineered™ antibodies or variants, may be of different isotypes, such as IgG, IgA, IgM or IgE. Antibodies of the IgG class may include a different constant region, e.g. an IgG2 antibody may be modified to display an IgG1 or IgG4 constant region. In preferred embodiments, the invention provides Human Engineered™ antibodies or variants comprising a modified or unmodified IgG1 or IgG4 constant region. In the case of IgG1, modifications to the constant region, particularly the hinge or CH2 region, may increase or decrease effector function, including ADCC and/or CDC activity. In other embodiments, an IgG2 constant region is modified to decrease antibody-antigen aggregate formation. In the case of IgG4, modifications to the constant region, particularly the hinge region, may reduce the formation of half-antibodies. In specific exemplary embodiments, mutating the IgG4 hinge sequence Cys-Pro-Ser-Cys to the IgG1 hinge sequence Cys-Pro-Pro-Cys is provided.

A pharmaceutical composition comprising any one of the aforementioned M-CSF antagonists or M-CSF antibodies and a pharmaceutically acceptable carrier, excipient or diluent may be administered according to the present invention.

It may be further advantageous to mix two or more M-CSF antagonists together or to co-administer an M-CSF antagonist and a second anti-osteoclast agent to provide improved efficacy against osteolytic disorders of the invention, including cancer metastasis and/or bone loss associated with cancer metastasis.

In exemplary embodiments of the invention, the aforementioned methods are provided wherein the second anti-osteoclast agent is a bisphosphonate. In a further embodiment, the bisphophonate is zoledronate, pamidronate, clodronate, etidronate, tiludronate, alendronate, ibandronate or risedronate. Exemplary other anti-osteoclast agents include bisphosphonates, PTHrP neutralizing agents (e.g., antibody, antisense, siRNA), cathepsin K inhibitors, MIP-1-α antagonists, RANK/RANKL neutralizing agents (e.g., anti-RANK antibody, anti-RANKL antibody, or antisense, soluble RANKL receptor or muteins thereof), RANKL vaccine, osteoprotegrin (OPG), platelet-derived growth factors (PDGF), src kinase inhibitors, gallium maltolate, and matrix metalloproteinase (MMP) inhibitors.

The therapeutic methods of the present invention may be combined with yet a third therapeutic agent such as a cancer chemotherapeutic agent or with radiation treatment or surgery. Cancer chemotherapeutic agents include, without limitation, alkylating agents, such as carboplatin and cisplatin; nitrogen mustard alkylating agents; nitro'sourea alkylating agents, such as carmustine (BCNU); antimetabolites, such as methotrexate; purine analog antimetabolites, mercaptopurine; pyrimidine analog antimetabolites, such as fluorouracil (5-FU) and gemcitabine; hormonal antineoplastics, such as goserelin, leuprolide, and tamoxifen; natural antineoplastics, such as aldesleukin, interleukin-2, docetaxel, etoposide (VP-16), interferon alfa, paclitaxel, and tretinoin (ATRA); antibiotic natural antineoplastics, such as bleomycin, dactinomycin, daunorubicin, doxorubicin, and mitomycin; and vinca alkaloid natural antineoplastics, such as vinblastine, vincristine, vindesine; hydroxyurea; aceglatone, adriamycin, ifosfamide, enocitabine, epitiostanol, aclarubicin, ancitabine, nimustine, procarbazine hydrochloride, carboquone, carboplatin, carmofur, chromomycin A3, antitumor polysaccharides, antitumor platelet factors, cyclophosphamide, Schizophyllan, cytarabine, dacarbazine, thioinosine, thiotepa, tegafur, , neocarzinostatin, OK-432, bleomycin, furtulon, broxuridine, busulfan, honvan, peplomycin, , Bestatin (ubenimex), interferon-β, mepitiostane, mitobronitol, merphalan, laminin peptides, lentinan, Coriolus versicolor extract, tegafur/uracil, estramustine (estrogen/mechlorethamine).

Further, additional agents used as adjunctive therapy for cancer patients include EPO, G-CSF, ganciclovir; antibiotics, leuprolide; meperidine; zidovudine (AZT); interleukins 1 through 18, including mutants and analogues; interferons or cytokines, such as interferons α, β, and γ.; hormones, such as luteinizing hormone releasing hormone (LHRH) and analogues and, gonadotropin releasing hormone (GnRH); growth factors, such as transforming growth factor- β (TGF-β), fibroblast growth factor (FGF), nerve growth factor (NGF), growth hormone releasing factor (GHRF), epidermal growth factor (EGF), fibroblast growth factor homologous factor (FGFHF), hepatocyte growth factor (HGF), and insulin growth factor (IGF); tumor necrosis factor- a. & β (TNF- a & β); invasion inhibiting factor-2 (IIF-2); bone morphogenetic proteins 1-7 (B-MP 1-7); somatostatin; thymosin- a -1; γ-globulin; superoxide dismutase (SOD); complement factors; anti-angiogenesis factors; antigenic materials; pro-drugs; growth factor receptor kinase inhibitors; anti-Her2 anitbody; and VEGF neutralizing antibody.

Subsequent to the transition period, the amount of M-CSF antagonist or amount of second anti-osteoclast agent required to achieve a therapeutic effect may be reduced. Thus, after such time period, an M-CSF antagonist may improve efficacy of the second anti-osteoclast agent, or reduce side effects associated with administration of the second anti-osteoclast agent, or improve the safety of the second anti-osteoclast agent. An M-CSF antagonist may also improve efficacy, reduce side effects of, or improve safety of a third therapeutic modality such as cancer chemotherapy, other adjunctive therapy, surgery or radiation therapy. In another embodiment of the invention, a package, vial or container is provided comprising a medicament comprising an M-CSF antagonist and instructions that the medicament should be used in combination with a second and/or third therapeutic agent and/or with surgery or radiation therapy.

Numerous osteolytic disorders are contemplated to be amenable to treatment according to the present invention. As used herein, an "osteolytic disorder" is any condition resulting from increased osteoclast activity. A subject at risk of an osteolytic disorder may be a subject in a group predisposed to develop an osteolytic disorder, or a subject suffering from a disease that causes or contributes to increased osteoclastic activity. In exemplary embodiments of the invention, the osteolytic disorder maybe a metabolic bone disease associated with relatively increased osteoclast activity, including an endocrinopathy (hypercortisolism, hypogonadism, primary or secondary hyperparathyroidism, hyperthyroidism), hypercalcemia, deficiency state (rickets/osteomalacia, scurvy, malnutrition), chronic disease (malabsorption syndromes, chronic renal failure (renal osteodystrophy), chronic liver disease (hepatic osteodystrophy)), drugs (glucocorticoids (glucocorticoid-induced osteoporosis), heparin, alcohol), or hereditary disease (osteogenesis imperfecta, homocystinuria), cancer, osteoporosis, osteopetrosis, inflammation of bone associated with arthritis and rheumatoid arthritis, periodontal disease, fibrous dysplasia, and/or Paget's disease.

In other exemplary embodiments, the osteolytic disorder may be a metastatic cancer to bone, wherein the metastatic cancer is breast, lung, renal, multiple myeloma, thyroid, prostate, adenocarcinoma, blood cell malignancy, including leukemia and lymphoma; head and neck cancer; gastrointestinal cancer, including esophageal cancer, stomach cancer, colon cancer, intestinal cancer, colorectal cancer, rectal cancer, pancreatic cancer, liver cancer, cancer of the bile duct or gall bladder; malignancy of the female genital tract, including ovarian carcinoma, uterine endometrial cancer, vaginal cancer, or cervical cancer; bladder cancer; brain cancer, including neuroblastoma; sarcoma, osteosarcoma; or skin cancer, including malignant melanoma or squamous cell cancer.

In exemplary embodiments of the invention, any of the foregoing methods may prevent or reduce bone loss or preventing or reducing bone metastases or severity of bone loss associated with the disease.

M-CSF antibody administered according to the present invention may be given at a dose between about 2 µg/kg to 30 mg/kg, 0.1 mg/kg to 30 mg/kg or 0.1 mg/kg to 10 mg/kg body weight.

### BRIEF DESCRIPTION OF THE DRAWINGS

Figure 1A shows the amino acid sequence of M-CSF-specific murine antibody RX1 (SEQ ID NOs: 2 and 4) (encoded by the cDNA insert of the plasmid deposited with the American Type Culture Collection, Manassas, VA, USA, under ATCC deposit number PTA-6113) and a corresponding nucleic acid sequence (SEQ ID NOs: 1 and 3). The CDR regions are numbered and shown in bold.
Figures 1B and 1C show the amino acid sequences of M-CSF specific murine antibody RX1 light (SEQ ID NO: 5) and heavy chains (SEQ ID NO: 6), respectively, with high risk (bold), moderate risk (underline), and low risk residues identified according to Studnicka et al., WO93/11794.
Figures 2, 3, and 4 show the amino acid sequences of MCSF-specific murine antibodies 5H4 (SEQ ID NOs: 10 and 11), MC1 (SEQ ID NOs: 12 and 13) (produced by the hybridoma deposited under ATCC deposit number PTA-6263) and MC3 (SEQ ID NOs: 14 and 15) (produced by the hybridoma deposited under ATCC deposit number PTA-6264), respectively.
Figure 5 is the amino acid sequence of M-CSFα (SEQ ID NO: 7).

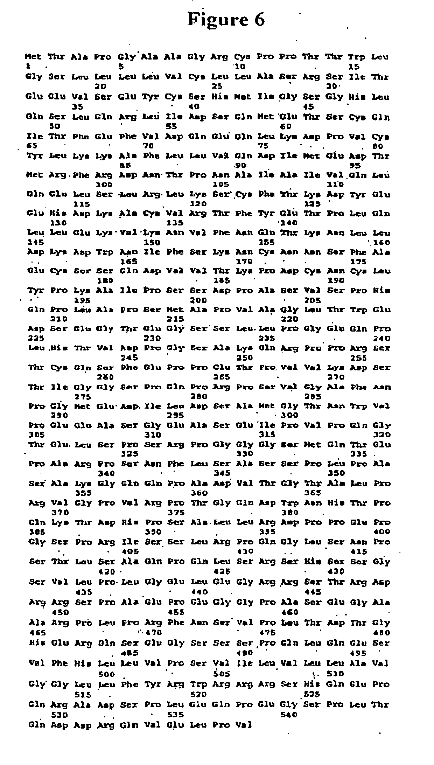

Figure 6 is the amino acid sequence of M-CSFβ (SEQ ID NO: 8).
Figure 7 is the amino acid sequence of M-CSFγ (SEQ ID NO: 9). A number of polymorphisms in the DNA sequence may result in amino acid differences. For example, a common polymorphism provides an Ala rather than Pro at position 104.
Figures 8A and B are an alignment of CDR regions of the heavy and light chain amino acid sequences of human M-CSF specific murine antibodies RX1; 5H4; MC1; and MC3 (SEQ ID NOs: 16-38).

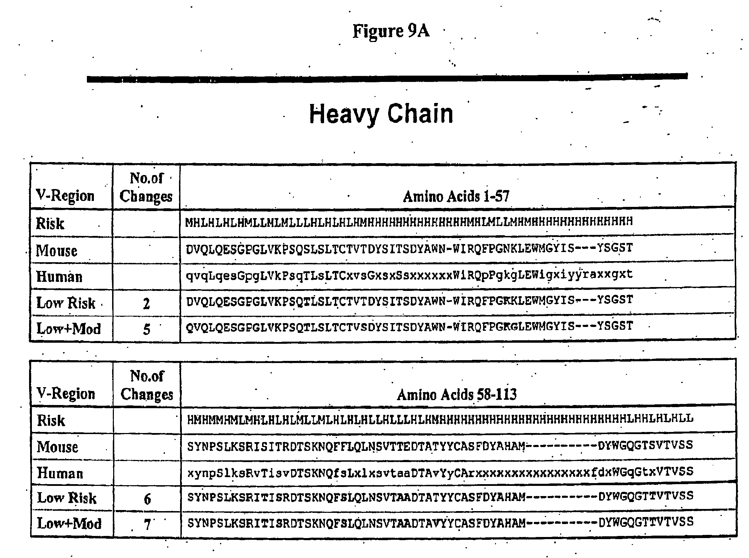

Figure 9A shows (a) the risk line for the murine RX1 heavy chain (H=high risk, M=moderate risk, L=low risk), (b) the RX1 heavy chain amino acid sequence (SEQ ID NO: 6), (c) the amino acid sequence of the closest human consensus sequence, Kabat Vh2 consensus, aligned to RX1 (SEQ ID NO: 39) and (d) changes that were made to produce two exemplary Human Engineered™ sequences (SEQ ID NOs: 41 and 43). Figure 9B shows the amino acid sequences of the two exemplary heavy chain Human Engineered™ sequences (SEQ ID NOs: 41 and 43), designated "low risk" and "low+moderate risk" as well as corresponding nucleic acid sequences (SEQ ID NOs: 40 and 42).

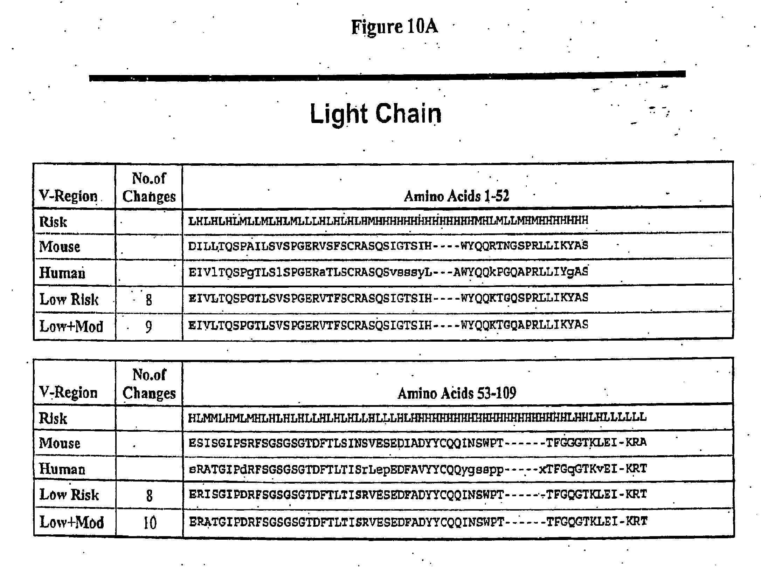

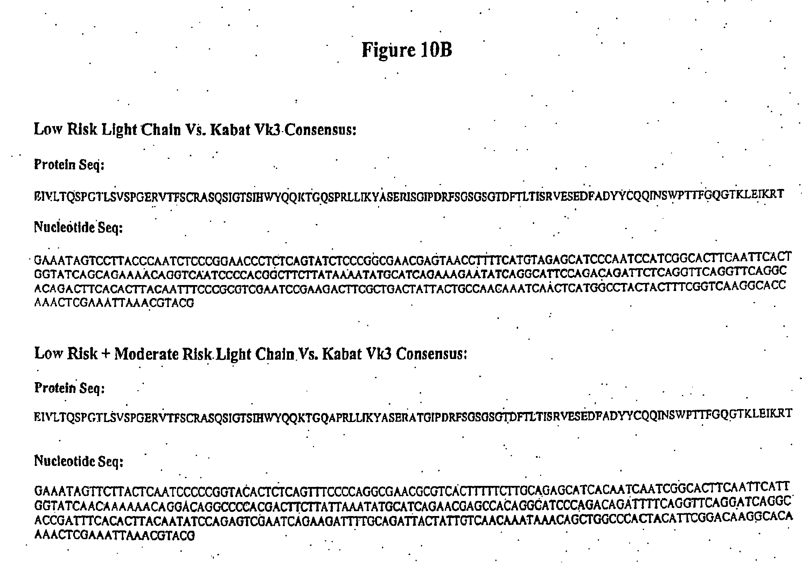

Figure 10A shows (a) the risk line for the murine RX1 light chain (H=high risk, M=moderate risk, L=low risk), (b) the RX1 light chain amino acid sequence (SEQ ID NO: 5), (c) the amino acid sequence of the closest human consensus sequence, Kabat Vk3 consensus, aligned to RX1 (SEQ ID NO: 49) and (d) changes that were made to produce two exemplary Human Engineered™ sequences (SEQ ID NOs: 45 and 47). Figure 10B shows the amino acid sequences of the two exemplary light chain Human Engineered™ sequences (SEQ ID NOs: 45 and 47), designated "low risk" and "low+moderate risk" as well as corresponding nucleic acid sequences (SEQ ID NOs: 44 and 46).

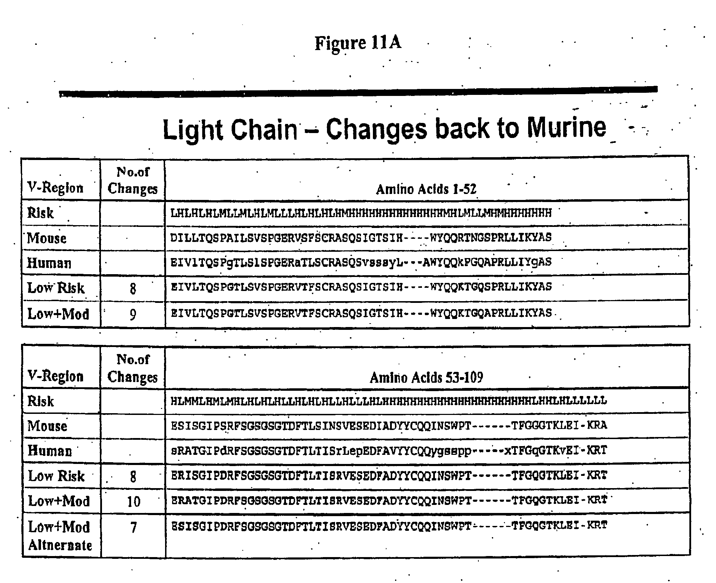

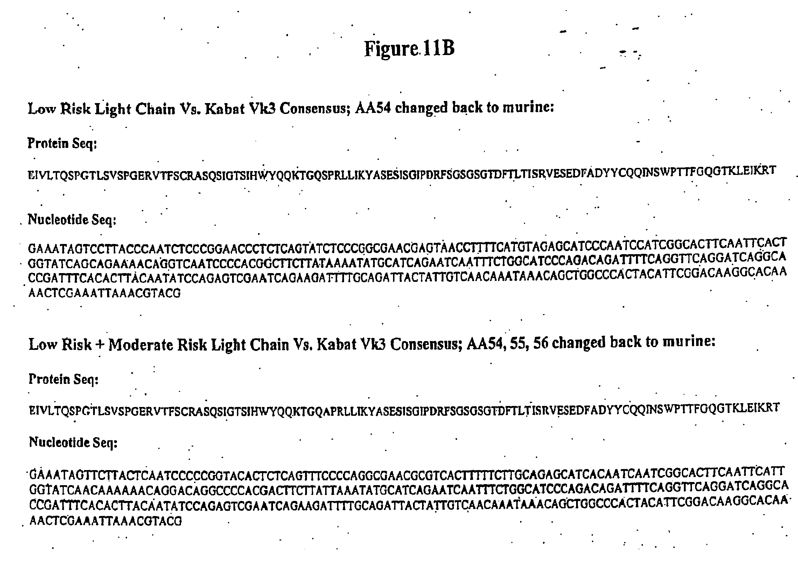

Figure 11A shows (a) the risk line for the murine RX1 light chain (H=high risk, M=moderate risk, L=low risk), (b) the RX1 light chain amino acid sequence (SEQ ID NO: 5), (c) the amino acid sequence of the closest human consensus sequence, Kabat Vk3 consensus, aligned to RX1 (SEQ ID NO: 49) and (d) an alternate exemplary amino acid sequence in which positions 54-56 were not changed (i.e. remained the murine sequence) (SEQ ID NO: 48). Figure 11B shows the amino acid sequences of two exemplary alternate light chain Human Engineered™ sequences (SEQ ID NOs: 48, 87), as well as corresponding nucleic acid sequences (SEQ ID NOs: 88 and 86).

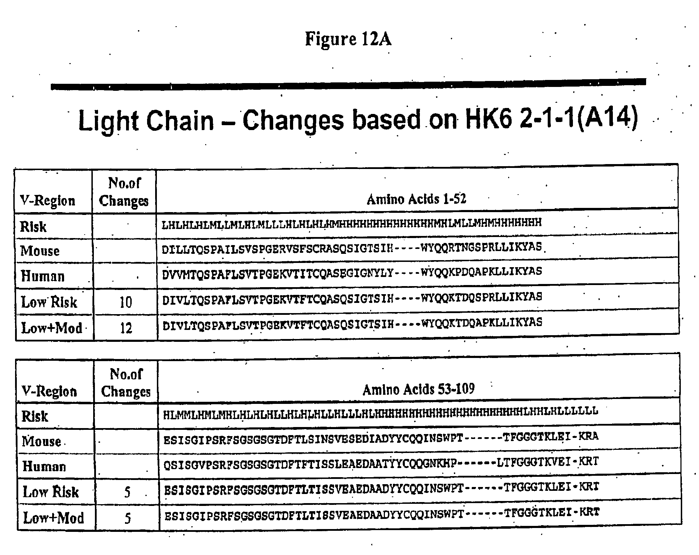

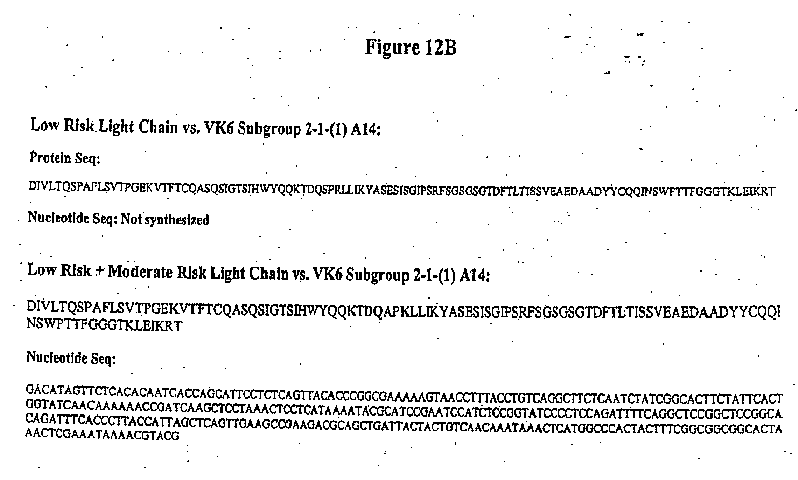

Figure 12A shows (a) the risk line for the murine RX1 light chain (H=high risk, M=moderate risk, L=low risk), (b).the RX1 light chain amino acid sequence (SEQ ID NO: 5), (c) the amino acid sequence of the closest human consensus germline sequence, Vk6 Subgroup 2-1-(1) A14, aligned to RX1 (SEQ ID NO: 50)and (d) changes that were made to produce two exemplary Human Engineered™ sequences (SEQ ID NOs: 51 and 53). Figure 12B shows the amino acid sequences of the two exemplary light chain Human Engineered™ sequences (SEQ ID NOs: 51 and 53), designated "low risk" and "low+moderate risk" as well as the corresponding nucleic acid sequence (SEQ ID NO: 52).

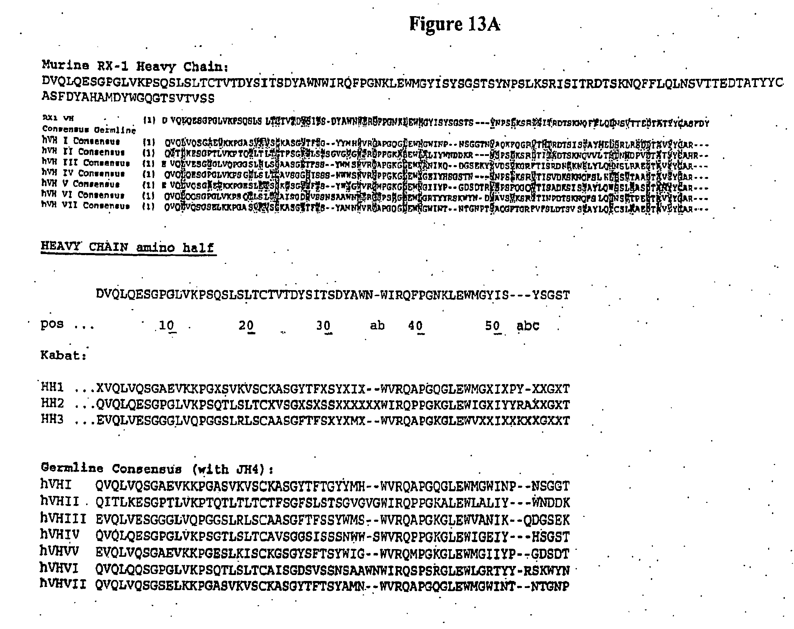

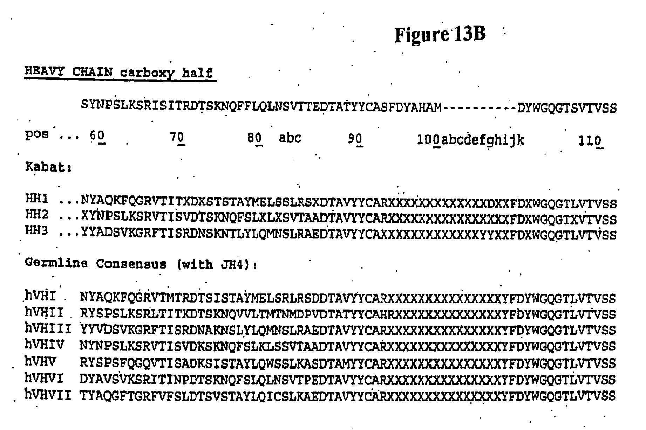

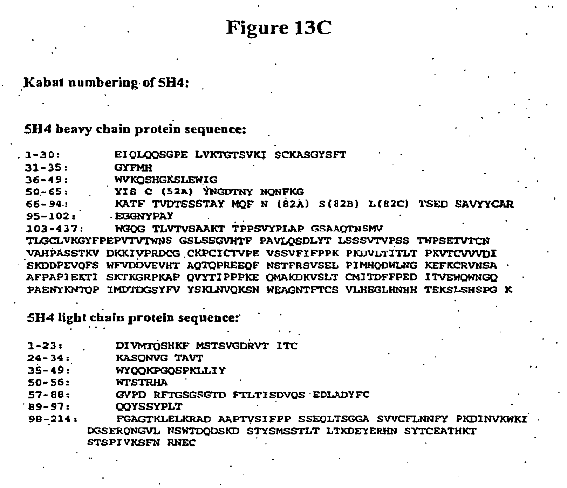

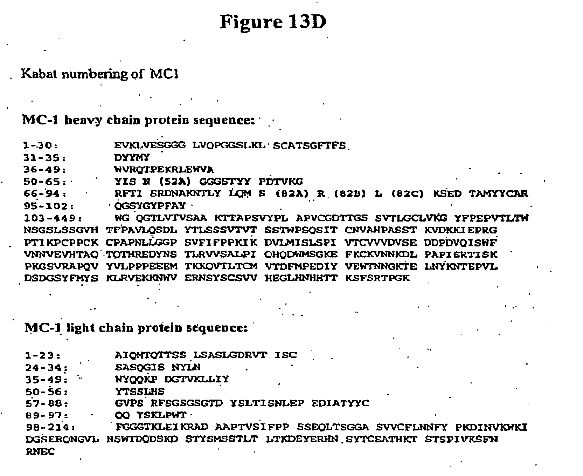

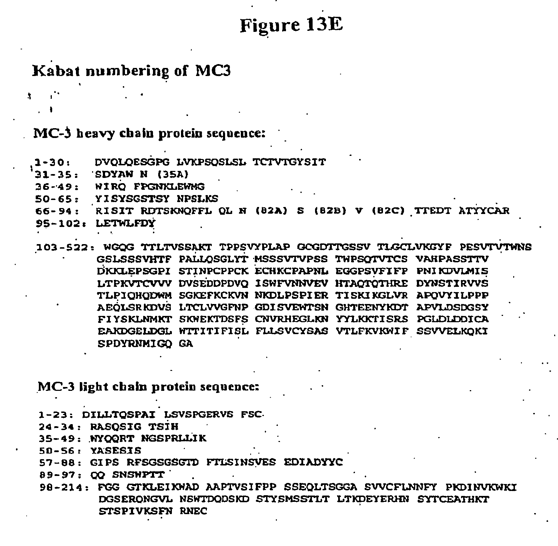

Figures 13A and 24B show the alignment of murine RX1 heavy chain amino acid sequence (SEQ ID NO: 54) with various human consensus and human germline consensus sequences using the Kabat numbering system (amino acid numbering indicated in line designated "POS") (SEQ ID NOs: 55-83). Figures 13C-13E show how the amino acid residues of antibodies 5H4, MC1 and MC3 correspond to the Kabat numbering system (SEQ ID NOs: 10 and 11; SEQ ID NOs: 12 and 13; SEQ ID NOs: 14 and 15, respectively).
Figure 14 shows the anti-resorptive effects of Zometa in an animal model.
Figure 15 shows the percent of animals in each group with detectable osteolysis.
Figure 16 shows the mean osteolysis scores based on x-ray image analysis on the last day of the study.
Figure 17 shows representative Faxitron x-ray images of tibias (tumor inoculation site) on the final day of the study. Arrows point to sites of osteolysis.
Figure 18 shows the effect of RX1 on osteoclast activity.
Figure 19 shows inhibition of osteclast activity by Zometa.
Figure 20 shows the results of a pharmacokinetic study with RX1 in primates.
Figure 21 shows the results of a pharmacokinetic study with RX1 in primates.

### DETAILED DESCRIPTION

Colony stimulating factor (CSF-1), also known as macrophage colony stimulating factor (M-CSF), has been found crucial for osteoclast formation. In addition, M-CSF has been shown to modulate the osteoclastic functions of mature osteoclasts, their migration and their survival in cooperation with other soluble factors and cell to cell interactions provided by osteoblasts and fibroblasts (Fixe and Praloran, Cytokine 10: 3-7, 1998; Martin et al., Critical Rev. in Eukaryotic Gene Expression 8: 107-23 (1998)).

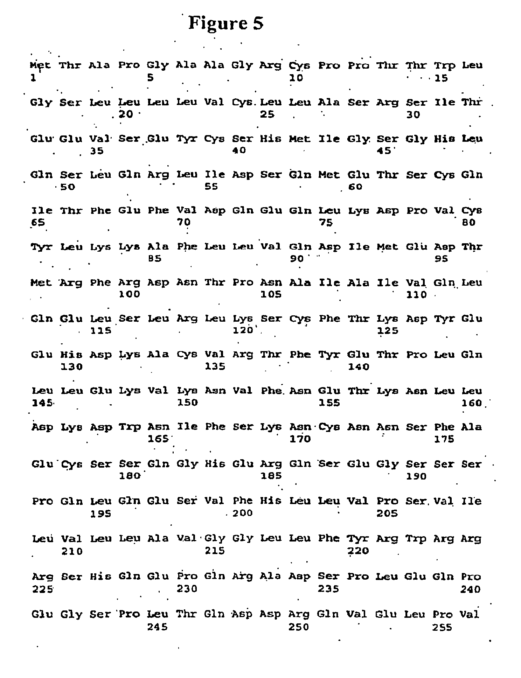

The full-length human M-CSF mRNA encodes a precursor protein of 554 amino acids. Through alternative mRNA splicing and differential post-translational proteolytic processing, M-CSF can either be secreted into the circulation as a glycoprotein or chondroitin sulfate containing proteoglycan or be expressed as a membrane-spanning glycoprotein on the surface of M-CSF producing cells. The three-dimensional structure of the bacterially expressed amino terminal 150 amino acids of human M-CSF, the minimal sequence required for full in vitro biological activity, indicates that this protein is a-disulfide linked dimer with each monomer consisting of four alpha helical bundles and an anti-parallel beta sheet (Pandit et al., Science 258: 1358-62 (1992)). Three distinct M-CSF species are produced through alternative mRNA splicing. The three polypeptide precursors are M-CFSα of 256 amino acids, M-CSFβ of 554 amino acids, and M-CSFγ of 438 amino acids. M-CSFβ is a secreted protein that does not occur in a membrane-bound form. M-CSFα is expressed as an integral membrane protein that is slowly released by proteolytic cleavage. M-CSFα is cleaved at amino acids 191-197 of the sequence set out in Figure 5. The membrane-bound form of M-CSF can interact with receptors on nearby cells and therefore mediates specific cell-to-cell contacts. The term "M-CSF" may also inlcude amino acids 36-438 of Figure 7.

Various forms of M-CSF function by binding to its receptor M-CSFR on target cells. M-CSFR is a membrane spanning molecule with five extracellular immunoglobulin-like domains, a transmembrane domain and an intracellular interrupted Src related tyrosine kinase domain. M-CSFR is encoded by the *c-fms* proto-oncogene. Binding of M-CSF to the extracellular domain of M-CSFR leads to dimerization of the receptor, which activates the cytoplasmic kinase domain, leading to autophosphorylation and phosphorylation of other cellular proteins (Hamilton J. A., J Leukoc Biol.,62(2):145-55, 1997; Hamilton J, A., Immuno Today., 18(7): 313-7, 1997).

Phosphorylated cellular proteins induce a cascade of biochemical events leading to cellular responses: mitosis, secretion of cytokines, membrane ruffling, and regulation of transcription of its own receptor (Fixe and Praloran, Cytokine 10: 32-37, 1998).

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma,; lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma; kidney cancer, liver cancer, vulval cancer, thyroid cancer, hepatic carcinoma and various types of head and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor (e.g., cancer) treatment, a therapeutic agent may directly decrease the pathology of tumor cells, or render the tumor cells more susceptible to treatment by other therapeutic agents, e.g., radiation and/or chemotherapy. The "pathology" of cancer includes all phenomena that compromise the well being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

"Mammal" for purposes of treatment refers to any animal classified as a mammal, including humans, domestic and farm animals, and zoo, sports, or pet animals, such as dogs, horses, cats, cows, etc. Preferably, the mammal is human.

As used herein, the phrase "metastatic cancer" is defined as cancers that have potential to spread to other areas of the body, particularly bone. A variety of cancers can metastasize to the bone, but the most common metastasizing cancers are breast, lung, renal, multiple myeloma, thyroid and prostate. By way of example, other cancers that have the potential to metastasize to bone include but are not limited to adenocarcinoma, blood cell malignancies, including leukemia and lymphoma; head and neck cancers; gastrointestinal cancers, including esophageal cancer, stomach cancer, colon cancer, intestinal cancer, colorectal cancer, rectal cancer, pancreatic cancer, liver cancer, cancer of the bile duct or gall bladder; malignancies of the female genital tract, including ovarian carcinoma, uterine endometrial cancers, vaginal cancer, and cervical cancer; bladder cancer; brain cancer, including neuroblastoma; sarcoma, osteosarcoma; and skin cancer, including malignant melanoma and squamous cell cancer. The present invention especially contemplates prevention and treatment of tumor-induced osteolytic lesions in bone.

As used herein, the phrase "therapeutically effective amount" refers to is meant to refer to an amount of therapeutic or prophylactic M-CSF antagonist, such as M-CSF antibody, that would be appropriate for an embodiment of the present invention, that will elicit the desired therapeutic or prophylactic effect or response when administered in accordance with the desired treatment regimen.

Human "M-CSF" as used herein refers to a human polypeptide having substantially the same amino acid sequence as the mature human M-CSFα, M-CSFβ, or M-CSFγ polypeptides described in Kawasaki et al., Science 230:291 (1985), Cerretti et al., Molecular Immunology, 25:761 (1988), or Ladner et al., EMBO Journal 6:2693 (1957), each of which are incorporated herein by reference. Such terminology reflects the understanding that the three mature M-CSFs have different amino acid sequences, as described above, and that the active form of M-CSF is a disulfide bonded dimer; thus, when the term "M-CSF" refers to the biologically active form, the dimeric form is intended. "M-CSF dimer" refers to two M-CSF polypeptide monomers that have dimerized and includes both homodimers (consisting of two of the same type of M-CSF monomer) and heterodimers (consisting of two different monomers). M-CSF monomers may be converted to M-CSF dimers in vitro as described in U.S. Pat. No. 4,929,700, which is incorporated herein by reference.

### I. Antagonists

As used herein, the term "antagonist" generally refers to the property of a molecule, compound or other agent to, for example, interfere with the binding of one molecule with another molecule or the stimulation of one cell by another cell either through steric hindrance, conformational alterations or other biochemical mechanisms. In one regard, the term antagonist relates to the property of an agent to prevent the binding of a receptor to its ligand, e.g., the binding of M-CSF with M-CSFR, thereby inhibiting the signal transduction pathway triggered by M-CSF. The term antagonist is not limited by any specific action mechanism, but, rather, refers generally to the functional property presently defined. Antagonists of the present invention include, but are not limited to: M-CSF antibodies and fragments and muteins and modifications thereof, soluble M-CSF and fragments and muteins and modifications thereof, M-CSFR antibodies and fragments and muteins and modifications thereof, soluble M-CSFR and fragments and muteins and modifications thereof, and peptides as well as other chemical compounds and molecules that bind to M-CSF or M-CSFR and nucleic acid molecules such as antisense or RNAi compounds that inhibit expression of M-CSF and M-CSFR. Any of the antagonists of the present invention can be administered in any manner known in the art. For example, M-CSF muteins, M-CSFR muteins or antibody fragments that bind to M-CSF or M-CSFR can be administered via gene therapy.

M-CSF antagonists of the present invention include, where applicable, functional equivalents. For example, molecules may differ in length, structure, components, etc., but may still retain one or more of the defined functions. More particularly, functional equivalents of the antibodies, antibody fragments or peptides of the present invention may include mimetic compounds, i.e., constructs designed to mimic the proper configuration and/or orientation for antigen binding.

Preferred M-CSF antagonists may optionally be modified by addition of side groups, etc., e.g., by amino terminal acylation, carboxy terminal amidation or by coupling of additional groups to amino acid side chains. Antagonists may also comprise one or more conservative amino acid substitutions. By "conservative amino acid substitutions" is meant those changes in amino acid sequence that preserve the general charge, hydrophobicity/hydrophilicity and/or steric bulk of the amino acid substituted. For example, substitutions between the following groups are conservative: Gly/Ala, Val/Ile/Leu, Asp/Glu, Lys/Arg, Asn/Gln, Ser/Cys/Thr, and Phe/Trp/Tyr. Such modifications will not substantially diminish the efficacy of the M-CSF antagonists and may impart such desired properties as, for example, increased in vivo half life or decreased toxicity.

The invention is also intended to include polypeptides bearing modifications other than the insertion, deletion, or substitution of amino acid residues. By way of example, the modifications may be covalent in nature, and include for example, chemical bonding with polymers, lipids, other organic, and inorganic moieties. Such derivatives may be prepared to increase circulating half-life of a polypeptide, or may be designed to improve targeting capacity for the polypeptide to desired cells, tissues, or organs. Similarly, the invention further embraces M-CSF or M-CSFR polypeptides that have been covalently modified to include one or more water soluble polymer attachments such as polyethylene glycol, polyoxyethylene glycol, or polypropylene glycol.

### A. M-CSF Antibodies

The term "antibody" is used in the broadest sense and includes fully assembled antibodies, monoclonal antibodies, polyclonal antibodies, multispecific antibodies (e.g., bispecific antibodies), antibody fragments that can bind antigen (e.g., Fab', F'(ab)2, Fv, single chain antibodies, diabodies), and recombinant peptides comprising the forgoing as long as they exhibit the desired biological activity.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that are typically include different antibodies directed against different determinants (epitopes), each monoclonal antibody is directed against a single determinant on the antigen. In addition to their specificity, the monoclonal antibodies are advantageous in that they are synthesized by the homogeneous culture, uncontaminated by other immunoglobulins with different specificities and characteristics.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 [19751, or may be made by recombinant DNA methods (see, e.g., U.S. Patent No. 4,816,567). The "monoclonal antibodies" may also be isolated from phage antibody libraries using the techniques described inClackson et al.,Nature,352:624628[1991] end Marks et al., J. Mol. Biol., 222:581-597 (1991), for example.

Depending on the amino acid sequence of the constant domain of their heavy chains, immunoglobulins can be assigned to different classes. There are five major classes, IgA, IgD, IgE, IgG and IgM, and several of these may be further divided into subclasses or isotypes, e.g. IgG1, IgG2, IgG3, IgG4, IgA1 and IgA2. The heavy-chain constant domains that correspond to the different classes of immunoglobulins are called alpha, delta, epsilon, gamma and mu respectively. The subunit structures and three-dimensional configurations of different classes of immunoglobulins are well known. Different isotypes have different effector functions; for example, IgG1 and IgG3 isotypes have ADCC activity.

"Antibody fragments" comprise a portion of an intact full length antibody, preferably the antigen binding or variable region of the intact antibody. Examples of antibody fragments include Fab, Fab', F(ab')2, and Fv fragments; diabodies; linear antibodies (Zapata et al., Protein Eng.,B(10):1057-1062 (1995)); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize 35 readily. Pepsin treatment yields an F(ab')2 fragment that has two "Single-chain Fv" or "sFv" antibody fragments comprise the VH and VL domains of antibody, wherein these domains are present in a single polypeptide chain. Preferably, the Fv polypeptide further comprises a polypeptide linker between the VH and VL domains that enables the Fv to form the desired structure for antigen binding. For a review of sFv see Pluckthun in The Pharmacology of Monoclonal Antibodies, vol. 113, Rosenburg and Moore eds., Springer-Verlag, New York, pp. 269-315 (1994).

The term "hypervariable" region refers to the amino acid residues of an antibody which are responsible for antigen-binding. The hypervariable region comprises amino acid residues from a complementarity determining region or CDR [i.e., residues 24-34 (L1), 50-56 (L2) and 89-97 (L3) in the light chain variable domain and 31-35 (H1), 50-65 (H2) and 95-102 (H3) in the heavy chain variable domain as described by Kabat et al., Sequences of Proteins of Immunological Interest, 5^{th} Ed. Public Health Service, National Institutes of Health, Bethesda, Md. (1991)] and/or those residues from a hypervariable loop (i.e., residues 26-32 (L1), 50-52 (L2) and 91-96 (L3) in the light chain variable domain and 26-32 (H1), 53-55 (H2) and 96-101 (H3) in the heavy chain variable domain as described by [Chothia et al., J. Mol.Biol. 196: 901-917 (1987)].

"Framework" or FR residues are those variable domain residues other than the hypervariable region residues.

The term "diabodies" refers to small antibody fragments with-two antigen-binding sites, which fragments comprise a heavy-chain variable domain (VH) connected to a light-chain variable domain (VL) in the same polypeptide chain (VH VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and 30 Hollinger et al., Proc. Natl. Acad. Sci. USA, 90:6444-6448 (1993).

In some embodiments, it may be desirable to generate multispecific (e.g. bispecific) monoclonal antibody including monoclonal, human, humanized, Human Engineered™ or variant anti-M-CSF antibodies having binding specificities for at least two different epitopes. Exemplary bispecific antibodies may bind to two different epitopes of M-CSF. Alternatively, an anti-M-CSF arm may be combined with an arm which binds to a triggering molecule on a leukocyte such as a T-cell receptor molecule (e.g., CD2 or CD3), or Fc receptors for I gG (FcγR), such as FcγRI (CD64), FcγRI] (CD32) and FcγRIII (CD16) so as to focus cellular defense mechanisms to the M-CSF-expressing cell. Bispecific antibodies may also be used to localize cytotoxic agents to cells which express M-CSF. These antibodies possess an M-CSF-binding arm and an arm which binds the cytotoxic agent (e.g., saporin, anti-interferon-60, vinca alkaloid, ricin A chain, methotrexate or radioactive isotope hapten). Bispecific antibodies can be prepared as full length antibodies or antibody fragments (e.g., F(ab').sub.2 bispecific antibodies).

According to another approach for making bispecific antibodies, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers. See WO96/270 published September 6, 1996.

Bispecific antibodies include cross-linked or "heteroconjugate" antibodies. For example, one of the antibodies in the heteroconjugate can be coupled to avidin, the other to biotin. Heteroconjugate antibodies may be made using any convenient cross-linking methods. Suitable cross-linking agents are well known in the art, and are disclosed in U.S. Pat. No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes. In yet a further embodiment, Fab'-SH fragments directly recovered from E. coli can be chemically coupled in vitro to form bispecific antibodies. (Shalaby et al., J. Exp. Med. 175:217-225 (1992))

Shalaby et al., J. Exp. Med. 175:217-225 (1992) describe the production of a fully humanized bispecific antibody F(ab')2 molecule. Each Fab' fragment was separately secreted from *E.coli* and subjected to directed chemical coupling in vitro to form the bispecfic antibody. The bispecific antibody thus formed was able to bind to cells overexpressing the HER2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. (Kostelny et al., J. Immunol. 148(5):1547-1553 (1992)) The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments.

The fragments comprise a heavy chain variable region (V_{H}) connected to a light-chain variable region (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol. 152: 5368 (1994).

Alternatively, the bispecific antibody may be a "linear antibody" produced as described in Zapata et al. Protein Eng. 8(10):1057-1062 (1995). Briefly, these antibodies comprise a pair oftandem Fd segments (V_{H} -C_{H}1-V^{H} -C_{H}1) which form a pair of antigen binding regions. Linear antibodies can be bispeci'fic or monospecific.

Antibodies with more than two valencies are also contemplated. For example, trispecific antibodies can be prepared. (Tutt et al., J. Immunol. 147:60 (1991))

In certain embodiments, the monoclonal, human, humanized, Human Engineered™ or variant anti-M-CSF antibody is an antibody fragment, such as an RX1, 5H4, MC1, or MC3 antibody fragment. Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992) and Brennan et al., Science 229:81 (1985)). However, these fragments can now be produced directly by recombinant host cells. Better et al., Science 240: 1041-1043 (1988) disclose secretion of functional antibody fragments from bacteria *(see, e.g.,* Better et al., Skerra et al. Science 240: 1038-1041 (1988)). For example, Fab'-SH fragments can be directly recovered from E. coli and chemically coupled to form F(ab')2 fragments (Carter et al., Bio/Technology 10:163-167 (1992)). In another embodiment, the F(ab')2 is formed using the leucine zipper GCN4 to promote assembly of the F(ab')2 molecule. According to another approach, Fv, Fab or F(ab')2 fragments can be isolated directly from recombinant host cell culture. Other techniques for the production of antibody fragments will be apparent to the skilled practitioner.

An "isolated" antibody is one that has been identified and separated and for recovered from a component of its natural environment. Contaminant components of its natural environment are materials that would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

For a detailed description of the structure and generation of antibodies, see Roth, D.B., and Craig, N.L., Cell, 94:411-414 (1998), and United States Patent No. 6,255,458, herein incorporated by reference in its entirety. Briefly, the process for generating DNA encoding the heavy and light chain immunoglobulin genes occurs primarily in developing B-cells. Prior to the rearranging and joining of various immunoglobulin gene segments, the V, D, J and constant (C) gene segments are found generally in relatively close proximity on a single chromosome. During B-cell-differentiation, one of each of the appropriate family members of the V, D, J (or only V and J in the case of light chain genes) gene segments are recombined to form functionally rearranged heavy and light immunoglobulin genes. This gene segment rearrangement process appears to be sequential. First, heavy chain D-to-J joints are made, followed by heavy chain V-to-DJ joints and light chain V-to-J joints.

The recombination of variable region gene segments to form functional heavy and light chain variable regions is mediated by recombination signal sequences (RSS's) that flank recombinationally competent V, D and J segments. RSS's necessary and sufficient to direct recombination, comprise a dyad-symmetric heptamer, an AT-rich nonamer and an intervening spacer region of either 12 or 23 base pairs. These signals are conserved among the different loci and species that carry out D-J (or V-J) recombination and are functionally interchangeable. See Oettinger, et al. (1990), Science, 248, 1517-1523 and references cited therein. The heptamer comprises the sequence CACAGTG or its analogue followed by a spacer of unconserved sequence and then a nonamer having the sequence ACAAAAACC or its analogue. These sequences are found on the J, or downstream side, of each V and D gene segment. Immediately preceding the germline D and J segments are again two recombination signal sequences, first the nonamer and then the heptamer again separated by an unconserved sequence. The heptameric and nonameric sequences following a V_{L}, V_{H} or D segment are complementary to those preceding the J_{L}, D or J_{H} segments with which they recombine. The spacers between the heptameric and nonameric sequences are either 12 base pairs long or between 22 and 24 base pairs long.

In addition to the rearrangement of V, D and J segments, further diversity is generated in the primary repertoire of immunoglobulin heavy and light chain by way of variable recombination at the locations where the V and J segments in the light chain are joined and where the D and J segments of the heavy chain are joined. Such variation in the light chain typically occurs within the last codon of the V gene segment and the first codon of the J segment. Similar imprecision in joining occurs on the heavy chain chromosome between the D and J_{H} segments and may extend over as many as 10 nucleotides. Furthermore, several nucleotides may be inserted between the D and J_{H} and between the V_{H} and D gene segments which are not encoded by genomic DNA. The addition of these nucleotides is known as N-region diversity

The net effect of such rearrangements in the variable region gene segments and the variable recombination which may occur during such joining is the production of a primary antibody repertoire.

"Fv" is the minimum antibody fragment that contains a complete antigen recognition and binding site. This region consists of a dimer of one heavy- and one light-chain variable domain in tight, non-covalent association. It is in this configuration that the three CDRs of each variable domain interact to define an antigen binding site on the surface of the VH VI dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them.

By "neutralizing antibody" is meant an antibody molecule that is able to eliminate or significantly reduce an effecter function of a target antigen to which is binds. Accordingly, a "neutralizing" anti-target antibody is capable of eliminating or significantly reducing an effecter function, such as enzyme activity, ligand binding, or intracellular signaling.

As provided herein, the compositions for and methods of treating cancer metastasis and/or bone loss associated with cancer metastasis may utilize one or more antibody used singularly or in combination with other therapeutics to achieve the desired effects. Antibodies according to the present invention may be isolated from an animal producing the antibody as a result of either direct contact with an environmental antigen or immunization with the antigen. Alternatively, antibodies may be produced by recombinant DNA methodology using one of the antibody expression systems well known in the art (See, e.g., Harlow and Lane, Antibodies: A Laboratory Manual, Cold Spring Harbor Laboratory (1988)). Such antibodies may include recombinant IgGs, chimeric fusion-proteins having immunoglobulin derived sequences or "Human Engineered™" antibodies that may all be used for the treatment of cancer metastasis and/or bone loss associated with cancer metastasis according to the present invention. In addition to intact, full-length molecules, the term "antibody" also refers to fragments thereof (such as, e.g., scFv, Fv, Fd, Fab, Fab' and F(ab)'2 fragments) or multimers or aggregates of intact molecules and/or fragments that bind to M-CSF (or M-CSFR). These antibody fragments bind antigen and may be derivatized to exhibit structural features that facilitate clearance and uptake, e.g., by incorporation of galactose residues.

In one embodiment of the present invention, M-CSF monoclonal antibodies may be prepared essentially as described in Halenbeck et al. U.S. Pat. No. 5,491,065 (1997), incorporated herein by reference. Exemplary M-CSF monoclonal antibodies include those that bind to an apparent conformational epitope associated with recombinant or native dimeric M-CSF with concomitant neutralization of biological activity. These antibodies are substantially unreactive with biologically inactive forms of M-CSF including monomeric and chemically derivatized dimeric M-CSF.

In other embodiments of the present invention, Human Engineered™ anti-M-CSF monoclonal antibodies are provided. The phrase "Human Engineered™ antibody" refers to an antibody derived from a non-human antibody, typically a mouse monoclonal antibody. Alternatively, a Human Engineered™ antibody may be derived from a chimeric antibody that retains or substantially retains the antigen binding properties of the parental, non-human, antibody but which exhibits diminished immunogenicity as compared to the parental antibody when administered to humans. The phrase "chimeric antibody," as used herein, refers to an antibody containing sequence derived from two different antibodies (see, e.g., U.S. Patent No. 4,816,567) which typically originate from different species. Most typically, chimeric antibodies comprise human and murine antibody fragments, generally human constant and mouse variable regions.

The phrase "complementarity determining region" or the term "CDR" refers to amino acid sequences which together define the binding affinity and specificity of the natural Fv region of a native immunoglobulin binding site (See, e.g., Chothia et al., J. Mol. Biol. 196:901 917 (1987); Kabat et al., U.S. Dept. of Health and Human Services NIH Publication No. 91 3242 (1991)). The phrase "constant region" refers to the portion of the antibody molecule that confers effector functions. In the present invention, mouse constant regions are preferably substituted by human constant regions. The constant regions of the subject antibodies are derived from human immunoglobulins. The heavy chain constant region can be selected from any of the five isotypes: alpha, delta, epsilon, gamma or mu.

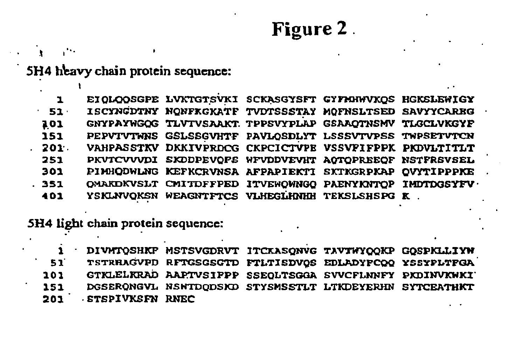

The antibodies of the present invention are said to be immunospecific or specifically binding if they bind to antigen with a Kₐ of greater than or equal to about 10⁶M⁻¹ preferably greater than or equal to about 10⁷M⁻¹, more preferably greater than or equal to about 10⁸M⁻¹ , and most preferably greater than or equal to about 10⁹M⁻¹, 10¹⁰M⁻¹, 10¹¹M⁻¹ or 10¹²M⁻¹. The anti-M-CSF antibodies may bind to different naturally occurring forms of M-CSF, including those expressed by the host's/subject's tissues as well as that expressed by the tumor. The monoclonal antibodies disclosed herein, such as RX1, 5H4, MC1, or MC3 antibody, have affinity for M-CSF and are characterized by a dissociation equilibrium constant (Kd) of at least 10⁻⁴ M, preferably at least about 10⁻⁷ M to about 10⁻⁸ M, more preferably at least about 10^{-δ}M, 10-¹⁰M, 10-¹¹M or 10-¹²M. Such affinities may be readily determined using conventional techniques, such as by equilibrium dialysis; by using the BIAcore 2000 instrument using general procedures outlined by the manufacturer; by radioimmunoassay using ¹²⁵I labeled M-CSF; or by another method known to the skilled artisan. The affinity data may be analyzed, for example, by the method of Scatchard et al., Ann N.Y. Acad. Sci., 51:660 (1949). Thus, it will be apparent that preferred M-CSF antibodies will exhibit a high degree of specificity for M-CSF and will bind with substantially lower affinity to other molecules. Preferred antibodies bind M-CSF with a similar affinity as murine RX1 of Figure 4 binds to M-CSF, exhibit low immunogenicity, and inhibit metastasis of cancer cells when tested in metastatic disease animal models. Other exemplary antibodies bind M-CSF with a similar affinity as murine 5H4, MC1 or MC3 of Figure 2, 3 or 4, respectively, binds to M-CSF.

The antigen to be used for production of antibodies may be, e.g., intact M-CSF or a fragment of M-CSF that retains the desired epitope, optionally fused to another polypeptide that allows the epitope to be displayed in its native conformation. Alternatively, cells expressing M-CSF at their cell surface can be used to generate antibodies. Such cells can be transformed to express M-CSF or may be other naturally occurring cells that express M-CSF. Other forms of M-CSF useful for generating antibodies will be apparent to those skilled in the art.

### i. Polyclonal Antibodies

Polyclonal antibodies are preferably raised in animals by multiple subcutaneous (sc) or intraperitoneal (ip) injections of the relevant antigen and an adjuvant. An improved antibody response may be obtained by conjugating the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride or other agents known in the art.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later, the animals are boosted with 1/5 to {fraction (1/10)} the original amount of peptide or conjugate in Freund's complete adjuvant by subcutaneous injection at multiple sites. At 7-14 days post-booster injection, the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### ii Monoclonal Antibodies

Monoclonal antibodies may be made using the hybridoma method first described by Kohler et al., Nature, 256:495 (1975), or may be made by recombinant DNA methods.

In the hybridoma method, a mouse or other appropriate host animal, such as a hamster or macaque monkey, is immunized as herein described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium. Human myeloma and mouse-human heteromyeloma cell lines also have been described for the production of human monoclonal antibodies (Kozbor, J. Immunol., 133: 3001 (1984) ;Brodeur et al., Monoclonal Antibody Production Techniques and Applications, pp. 51-63 (Marcel Dekker, Inc., New York, 1987)). Exemplary murine myeloma lines include those derived from MOP-21 and M.C.-11 mouse tumors available from the Salk Institute Cell Distribution Center, San Diego, Calif. USA, and SP-2 or X63-Ag8-653 cells available from the American Type Culture Collection, Rockville, Md. USA.

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The binding affinity of the monoclonal antibody can, for example, be determined by Scatchard analysis (Munson et al., Anal. Biochem., 107:220 (1980)).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

The antibodies of the present invention are said to be immunospecific or specifically binding if they bind to antigen with a Kₐ of greater than or equal to about 1.0⁶M⁻¹ preferably greater than or equal to about 10⁷M⁻¹, more preferably greater than or equal to about 10⁸M⁻¹, and most preferably greater than or equal to about 10⁹M⁻¹, 10⁻¹⁰M⁻¹, 10¹¹M⁻¹ or 10¹²M⁻¹. The anti-M-CSF antibodies may bind to different naturally occurring forms of M-CSF, including those expressed by the host's/subject's tissues as well as that expressed by the tumor. The monoclonal antibodies disclosed herein, such as RX1, 5H4, MC1, or MC3 antibody, have affinity for M-CSF and are characterized by a dissociation equilibrium constant (Kd) of at least 10⁻⁴ M, preferably at least about 10⁻⁷ M to about 10⁻⁸ M, more preferably at least about 10⁻⁸ M, 10⁻¹⁰M, 10⁻¹¹M or 10⁻¹²M. Such affinities may be readily determined using conventional techniques, such as by equilibrium dialysis; by using the BIAcore 2000 instrument, using general procedures outlined by the manufacturer; by radioimmunoassay using ¹²⁵I labeled M-CSF; or by another method known to the skilled artisan. The affinity data may be analyzed, for example, by the method of Scatchard et al., Ann N.Y. Acad. Sci., 51:660 (1949). Thus, it will be apparent that preferred M-CSF antibodies will exhibit a high degree of specificity for M-CSF and will bind with substantially lower affinity to other molecules. Preferred antibodies bind M-CSF with a similar affinity as murine RX1 of Figure 1 binds to M-CSF, exhibit low immunogenicity, and inhibit metastasis of cancer cells when tested in metastatic disease animal models. Other exemplary antibodies bind M-CSF with a similar affinity as murine 5H4, MC1 or MC3 of Figure 2, 3 or 4, respectively, binds to M-CSF.

Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened.

**TABLE 1**

| **Original Exemplary Preferred Residue Substitutions** | |
|---|---|
| Ala (A) val; leu; ile val | Arg (R) lys; gln; asn lys |
| Asn (N) gln; his; asp, lys; gln arg | Asp (D) glu; asn glu |
| Cys (C) ser; ala ser | Gln (Q) asn; glu asn |
| Glu (E) asp; gln asp | Gly (G) ala |
| His (H) asn; gln; lys; arg | Ile (I) leu; val; met; ala; leu phe; norleucine |
| Leu (L) norleucine; ile; val; ile met; ala; phe | |
| Lys (K) arg; gln; asn arg | Met (M) leu; phe; ile leu |
| Phe (F) leu; val; ile; ala; tyr | Pro (P) ala |
| Ser (S) thr | Thr (T) ser ser |
| Trp (W) tyr; phe tyr | Tyr (Y) trp; phe; thr; ser phe |
| Val (V) ile; leu; met; phe; leu ala; norleucine | |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into group based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions involve replacing a member of one of these classes with a member of another class.

Any cysteine residue not involved in maintaining the proper conformation of the humanized or variant antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

### B. M-CSF Muteins

The invention further provides M-CSF muteins that may be used as MCSF antagonists according to the methods of the invention.

"Fragment" as used herein means a portion of the intact native molecule; for example, a fragment polypeptide is a fragment of the native polypeptide in which one or more amino acids from either the N-terminal or C-terminal have been deleted.

"Mutein" as used herein with respect to polypeptides means a variant of the intact native molecule or a variant of a fragment of the native molecule, in which one or more amino acids have been substituted, inserted or deleted. Such substitutions, insertions or deletions can be at the N-terminus, C-terminus or internal to the molecule. Thus the term "muteins" includes within its scope fragments of the native molecule. Insertional muteins include fusions at the N- or C-terminus, e.g. fusion to the Fc portion of an immunoglobulin to increase half-life

Preferred muteins according to the invention exhibit at least about 65%, 70%. 75%, 80%, 85%, 90%, 95%, 97% or more sequence identity (homology) to the native polypeptide, as determined by the Smith-Waterman homology search algorithm (Meth. Mol. Biol. 70:173-187 (1997)) as implemented in the MSPRCH program (Oxford Molecular) using an affine gap search with the following search parameters: gap open penalty of 12, and gap extension penalty of 1. Other well-known and routinely used homology/identity scanning algorithm programs include Pearson and Lipman, PNAS USA, 85:2444-2448 (1988); Lipman and Pearson, Science, 222:1435 (1985); Devereaux et al., Nuc. Acids Res., 12:387-395 (1984); or the BLASTP, BLASTN or BLASTX algorithms of Altschul, et al., Mol. Biol., 215:403-410 (1990). Computerized programs using these algorithms are also available and include, but are not limited to: GAP, BESTFIT, BLAST, FASTA and TFASTA, which are commercially available from the Genetics Computing Group (GCG) package, Version 8, Madison Wis., USA; and CLUSTAL in the PC/Gene program by Intellegenetics, Mountain View Galif. Preferably, the percentage of sequence identity is determined by using the default parameters determined by the program.

"Modification" as used herein means any modification of the native polypeptide, fragment or mutein, such as glycosylation, phosphorylation, polymer conjugation (such as with polyethylene glycol), or other addition of foreign moieties, so long as the desired activity (agonist or antagonist) is retained.

U.S. Patent No. 6,025,146, and Koths, Mol. Reprod. Dev. 1997 Jan;46(1):31-38 both of which are incorporated herein by reference in their entirety, describe the crystallization of M-CSF alone and M-CSF complexed to MCSF-R, and characterize the three-dimensional structure of M-CSF as well as residues involved in receptor-binding. U.S. Patent No. 6,025,146 also describes methods for selecting candidate amino acid substitutions in M-CSF, based on structural information. The overall topology of this form of M-CSF is that of an antiparallel four alpha-helical bundle, in which the helices run up-up-down-down, unlike the more commonly observed up-down-up-down connectivity of most four helical bundles. A long crossover connection links helix A to helix B and a similar connection is found between helices C and D. In the disulfide-linked dimeric form, the bundles are linked end-to-end, forming an extremely flat, elongated structure (approximate dimensions 85 x 35 x 25). The re are three intramolecular disulfide bonds in each monomer (Cys7-Cys90, Cys48-Cys139, Cys102-Cys146) all of which are at the distal end of the molecule. One interchain disulfide bond (Cys31--Cys31) is located at the dimer interface with the noncrystallographic two-fold symmetry axis passing through it as shown in FIG. 2. Mutation experiments indicate that all of the cysteine residues in this form of M-CSF may be necessary for full biological activity. The structure described herein suggests that their role is primarily structural rather than being related to receptor recognition. U.S. Patent No. 6,025,146 provides the three-dimensional structure of the truncated recombinant M-CSF α dimer as identified by the alpha-carbon positions of the amino acid residues in the sequence.

Specific residues in helices A, C, and D appear to be involved in the specificity of the receptor-binding interaction. Since M-CSFβ has intrachain disulfide bonds involving cysteines 157 and/or 159, the C-terminal region of M-CSF likely extends from the "rear" of the structure, providing a variable-length "tether" for membrane-bound forms ofM-CSF. Thus, the "front" or receptor-binding region of M-CSF is on the opposite side of the molecules, consisting of solvent-accessible residues in or near helices A, C, and D, including residues from about 6 to 26, 71 to 90, and 110 to 130, respectively, of native M-CSF. Altering solvent accessible residues in these regions by site directed mutagenesis to increase or decrease side-chain interactions with the receptor may generate M-CSF agonists or antagonists. Residues having a solvent accessible surface area of greater than about 0.25 and preferably greater than about 0.4 are preferred based on normalization of the surface area of the amino acid accessible when in the trypeptide gly-x-gly (Kabsch, W. et al., Biopolymers 22:2577 (1983))- Preferably residues are chosen which do not interact with other parts of the protein such as the dimer interface in order to maintain the relative orientation of monomers and to avoid disturbing the process of protein folding. An optional additional consideration is selecting residues not conserved between human and mouse M-CSF, which does not recognize the human M-CSF receptor. Candidate amino acids are preferably selected for substitution with non-conservative amino acids, so as to disrupt hydrogen bonding and/or hydrophobic interactions with MCSF-R residues. For example, changing one or more histidines to non-hydrogen-donor amino acids of similar size may create an M-CSF with altered receptor binding ability. Preferred amino acids for substitution include but are not limited to: H15; Q79; R86; E115; E41; K93; D99; L55; S18; Q20; I75; V78; L85; D69; N70; H9; N63; and T34. M-CSF residues important in receptor signaling are believed to be composed of discontinuous regions of M-CSF. To minimize the likelihood of antibody formation to potentially administered M-CSF-based proteinaceous drugs, it is desirable to retain the solvent-accessible parental M-CSF residues (to resemble the native molecule) whenever possible.

Mutagenesis of amino acids H15 and H9 in the N-terminal/A helix region resulted in muteins with significantly lower biological activity and significantly lower MCSF-R binding ability. These results indicated that the reduced biological activity was due to decreased receptor binding affinity; thus, these histidine amino acids represent contacts that are important for M-CSF receptor binding affinity and should be left unchanged if full receptor-binding ability is desired. Nearby solvent accessible residues such as Y6 and S13 and others may also represent M-CSF receptor contact residues. A double mutant of M-CSF (Q20A, V78K) was constructed to test the importance of solvent accessible residues in the central portion of helices A and C. This double mutein had slightly lower (8-10 fold) biological activity and correspondingly lower receptor-binding activity. Mutagenesis of residues Q17, R21, E115 and E119 changed side chain properties of solvent-accessible amino acids in the areas of interest but did not affect biological specific activity, suggesting that these residues need not be altered in muteins designed to have antagonist activity.

In one embodiment, the invention contemplates use of M-CSF muteins in which residues of helices A and/or C and/or D involved in receptor-binding (for example, amino acids 6 to 26, 71 to 90 and/or 110 to 130) have been mutated non-conservatively. Such muteins preferably retain at least 65%, 70%, 75%, 80%, 85% or 90% similarity (i.e. amino acids that are identical or have similar properties) to the native sequence within helices A, C or D, but have higher similarity to the native sequence in the remainder of the polypeptide, e.g. , at least 95%, 98% or 99% similarity. In addition, residues that support the three-dimensional confirmation of the receptor-binding site may be mutated non-conservatively.

In another embodiment, the M-CSF mutein is a monomeric form of M-CSF. The dimeric form of M-CSF is the biologically active form, and monomeric forms of M=CSF are generally not active. Disulfide bonding of the monomers appears to occur through the Cys31-Cys31 interchain linkage. Thus, it is contemplated that monomeric forms of M-CSF may be suitable for use as antagonists. Such forms include muteins comprising cysteine deletions and/or cysteine replacements (e.g., cysteine to alanine substitutions) of Cys31 and/or other cysteines, or muteins in which the cysteine(s), particularly Cys31, have been chemically modified so that they are not available for disulfide bonding.

In yet another embodiment, the M-CSF mutein comprises one or more of helices A, C or D, or portions thereof involved in receptor-binding, alone or fused to other polypeptides that allow display of the fragments in proper three-dimensional conformation.

Muteins containing any desired conservative and/or non-conservative muteins are readily prepared using techniques well known in the art, including recombinant production or chemical synthesis.

Conservative substitutions, particularly substitutions outside of regions directly involved in ligand-receptor binding, are not expected to significantly change the binding properties of the M-CSF muteins (or M-CSFR muteins). Amino acids can be classified according to physical properties and contribution to secondary and tertiary protein structure. A conservative substitution is recognized in the art as a substitution of one amino acid for another amino acid that has similar properties. Exemplary conservative substitutions are set out in Table 2 (from WO 97/09433, page 10, published March 13, 1997 (PCT/GB96/02197, filed 9/6/96), immediately below.

**Table 2**

| | **Conservative Substitutions I** | | |
|---|---|---|---|
| | SIDE CHAIN | | |
| CHARACTERISTIC | | | AMINO ACID |
| | Aliphatic | | |
| | | Non-polar | G A P I L V |
| | | Polar-uncharged | C S T M N Q |
| | | Polar-charged | D E K R |
| | Aromatic | | H F W Y |
| | Other | | N Q D E |

Alternatively, conservative amino acids can be grouped as described in Lehninger, (Biochemistry, Second Edition; Worth Puhlishers, Inc. NY:NY (1975), pp.71-77) as set out in Table 3, immediately below.

**Table 3**

| **Conservative Substitutions II** | | | |
|---|---|---|---|
| | | SIDE CHAIN | |
| | CHARACTERISTIC | | AMINO ACID |
| Non-polar (hydrophobic) | | | |
| | A. | Aliphatic: | A L I V P |
| | B. | Aromatic: | F W |
| | C. | Sulfur-containing: | M |
| | D. | Borderline: | G |
| Uncharged-polar | | | |
| | A. | Hydroxyl: | STY |
| | B. | Amides: | N Q |
| | C. | Sulfhydryl: | C |
| | D. | Borderline: | G |
| Positively Charged (Basic): | | | K R H |
| Negatively Charged (Acidic): | | | DE |

As still an another alternative, exemplary conservative substitutions are set out in Table 4, immediately below.

**Table 4**

| **Conservative Substitutions III** | |
|---|---|
| Original Residue | Exemplary Substitution |
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| Gln (Q) | Asn |
| Glu (E) | Asp |
| His (H) | Asn, Gln, Lys, Arg |
| Ile (I) | Leu, Val, Met, Ala, Phe, |
| Leu (L) | Ile, Val, Met, Ala, Phe |
| Lys (K) | Arg, Gln, Asn |
| Met (M) | Leu, Phe, Ile |
| Phe (F) | Leu, Val, Ile, Ala |
| Pro (P) | Gly |
| Ser (S) | Thr |
| Thr (T) | Ser |
| Trp (W) | Tyr |
| Tyr (Y) | Trp, Phe, Thr, Ser |
| Val (V) | Ile, Leu, Met, Phe, Ala |

The availability of a DNA sequence encoding M-CSF permits the use of various expression systems to produce the desired polypeptides. Construction of expression vectors and recombinant production from the appropriate DNA sequences are performed by methods well known in the art. These techniques and various other techniques are generally performed according to Sambrook et al., Molecular Cloning--A Laboratory Manual, Cold Spring Harbor Laboratory, Cold Spring Harbor, N.Y. (1989), and Kriegler, M., Gene Transfer and Expression, A Laboratory Manual, Stockton Press, New York (1990), both of which are incorporated herein by reference.

Certain modifications to the primary sequence of M-CSF can be made by deletion, addition, or alteration of the amino acids encoded by the DNA sequence without destroying the desired structure (e.g., the receptor binding ability of M-CSF) in accordance with well-known recombinant DNA techniques. Further, a skilled artisan will appreciate that individual amino acids may be substituted or modified by oxidation, reduction or other modification, and the polypeptide may be cleaved to obtain fragments that retain the active binding site and structural information. Such substitutions and alterations result in polypeptides having an amino acid sequence which falls within the definition of polypeptide "having substantially the same amino acid sequence as the mature M-CSFα (SEQ ID NO: 7), M-CSFβ (SEQ ID NO: 8), and M-CSFγ (SEQ ID NO: 9)polypeptides."

Polypeptides may be produced by chemical synthesis or recombinant production techniques known in the art.

The relatedness of proteins can also be characterized through the relatedness of their encoding nucleic acids. Methods to determine identity and/or similarity of polynucleotide sequences are described above. In addition, methods to determine similarity of polynucleotide sequences through testing their ability to hybridize un der moderately or highly stringent conditions may be determined as follows. Exemplary moderately stringent hybridization conditions are as follows: hybridization at 42°C in a hybridization solution comprising 50% formamide, 1% SDS, 1 M NaCl, 10% Dextran sulfate, and washing twice for 30 minutes at 60°C in a wash solution comprising 0.1x SSC and 1% SDS. Highly stringent conditions include washes at 68°C in a wash solution comprising 0.1x SSC and 1% SDS. It is understood in the art that conditions of equivalent stringency can be achieved through variation of temperature and buffer, or salt concentration as described in the art (Ausubel, et al. (Eds.), Protocols in Molecular Biology, John Wiley & Sons (1994), pp. 6.0.3 to 6.4.10). Modifications in hybridization conditions can be empirically determined or precisely calculated based on the length and the percentage of guanosine/cytosine (GC)base pairing of the probe. The hybridization conditions can be calculated as described in Sambrook et al., (Eds.), Molecular Cloning: A Laboratory Manual, Cold Spring Harbor Laboratory Press: Cold Spring Harbor, New York (1989), pp. 9.47 to 9.51.

### C. Soluble M-CSFR

Exemplary M-CSFR fragments according to the invention may comprise one or more, or two or more, of domains involved in M-CSF/receptor binding (believed to be domains 1, 2 and 3). Preferred M-CSFR fragments comprises all three of domains 1, 2 and 3 of M-CSFR. Additional mutations and/or modifications to such fragments or to the entire extracellular domain of M-CSFR are contemplated and may be produced as described above in the section on M-CSF muteins.

M-CSFR (SEQ ID NOs: 84 and 85) is a membrane spanning molecule with five extracellular immunoglobulin-like domains (of which domains 1-3 are believed to be involved in ligand-receptor binding), a transmembrane domain and an intracellular interrupted Src related tyrosine kinase domain. With reference to SEQ ID NO: 85, the aforementioned domains are located as follows: Ig domain 1: amino acids 27-102; Ig domain 2: amino acids 112-196; Ig domain 3: amino acids 215-285; Ig domain 4: amino acids 308-399; Ig domain 5: amino acids 410-492; transmembrane domain: amino acids 515-537; and kinase domain: amino acids 582-910. A "typical" immunoglobulin-like domain contains a loop structure usually anchored by a disulfide bond between two cysteines at the extremity of each loop. In M-CSF-R, these cysteines fonning the Ig-like loops are at the following amino acid positions: Domain 1: 42, 84; Domain 2: 127, 177; Domain 3: 224, 278; Domain 4: no cysteins involved; Domain 5: 419, 485.

The intact extracellular portion of M-GSFR or any fragment thereof that retains antigenicity, for example, one or more of the Ig-like loops, may be used to raise antibodies that would bind to the native receptor. Polyclonal, monoclonal, chimeric, CDRS grafted, humanized, fully human antibodies and antigen-binding fragments thereof may be prepared as described above for antibodies to M-CSF. The antibody products may be screened for activity as an MCSF antagonist and for suitability in the treatment methods-of the invention using assays as described in the section entitled "Screening Methods" herein or using any suitable assays known in the art.

One or more of the aformentioend Ig-like loops within the extracellular domain of the receptor may be sufficient to inhibit interaction between M-CSF and M-CSFR. Thus fragments of the extracellular domain of M-CSFR and muteins thereof may be easily prepared using recombinant or chemical synthetic means well known in the art. The products may be screened for activity as an MCSF antagonist and for suitability in the treatment methods of the invention using assays as described in the section entitled "Screening Methods" herein or using any suitable assays known in the art.

### D. Gene Therapy

Delivery of a therapeutic protein to appropriate cells can be effected via gene therapy ex vivo, in situ, or in vivo by use of any suitable approach known in the art, including by use of physical DNA transfer methods (e.g., liposomes or chemical treatments) or by use of viral vectors (e.g., adenovirus, adeno-associated virus, or a retrovirus). Antisense compounds and methods of using them are also provided by the present invention. The level of M-CSF or M-CSFR activity may be reduced by using well-known antisense, gene "knock-out," ribozyme, triple helix or RNAi methods to decrease the level gene expression. Techniques for the production and use of such molecules are well known to those of skill in the art.

As used herein, the term "peptidomimetic" is a non-peptide compound that comprises an assembly of amino acid side chains, or pharmacophores, or suitable derivatives thereof, that are supported on a scaffold such that the spatial orientation of the pharmacophores substantially mimic the bioactive conformation of a natural peptide. For example, a peptidomimetic may lack amino acids or peptide bonds but retain the particular three-dimensional arrangement of peptide chain groups from the parent peptide that is required for binding activity. The scaffold may comprise a bicyclic, tricyclic or higher polycyclic carbon or heteroatom skeleton, or may be based on one or more ring structures (e.g., pyridine, indazole, etc.) or amide bonds. This scaffold may be linked by spacers to an acidic group (e.g. a carboxylic acid functional group) at one end and a basic group (e.g. an N-containing moiety such as amidine or guanidine) at the other end of the core. Exemplary techniques for synthesizing peptidomimetics are described in U.S. patent application no. 20030199531 published October 23, 2003, U.S. Patent Application No. 20030139348 published July 24, 2003.

In addition to antibodies and other proteins, this invention also contemplates alternative M-CSF antagonists including, but not limited to, peptides or small organic molecules that are also effective in inhibiting the interaction between M-CSF and M-CSFR or the activation of M-CSFR.

### II. Combination Therapy

Concurrent administration of two therapeutic agents according to the present invention, such as an M-CSF antagonist and a second anti-osteoclast agent, does not require that the agents be administered at the same time or by the same route, as long as there is an overlap in the time period during which the agents are exerting their therapeutic effect. Simultaneous or sequential administration is contemplated, as is administration on different days or weeks.

The discovery of a significant time lag to observe therapeutic effect after commencing treatment with an M-CSF antibody (an exemplary M-CSF antagonist) makes desirable the co-administration of a second anti-osteoclast agent with quicker onset of action during this transition period. During the transition period, the two agents must be administered at a monotherapeutically effective amount. Subsequent to the transition period, the second anti-osteoclast agent may be discontinued or reduced in dosage. If the M-CSF antagonist and second anti-osteoclast agent exert synergistic effects, the dose of one or both may be lowered after the transition period.

Compositions of the invention are administered to a mammal already suffering from, or predisposed to, osteolytic disorder, including cancer metastasis and/or bone loss associated with cancer metastasis, or other bone loss related diseases, such as osteoporosis, in an amount sufficient to prevent or at least partially arrest the development of such disease. An amount of a therapeutic agent adequate to accomplish this when the therapeutic agent is given alone (not in combination with a second therapeutic agent) is defined as a "monotherapeutically effective dose."

In the combination therapy methods of the present invention, the M-CSF antagonist, such as the M-CSF antibody, and the second anti-osteoclast agent may be administered simultaneously or at different time. The two agents can be administered, for example, within 8 hours, 1 day, 1 4 days, 30 days, 3 months, 6 months, 9 months or 1 year of each other.

Exemplary second anti-osteoclast agents include bisphosphonates, including but not limited to zoledronate, pamidronate, clodronate, etidronate, tiludronate, alendronate, ibandronate or risedronate. Exemplary other anti-osteoclast agents include bisphosphonates, PTHrP neutralizing agents (e.g., antibody, antisense, siRNA), cathepsin K inhibitors, MIP-1-α antagonists, RANK/RANKL neutralizing agents (e.g., anti-RANK antibody, such as AMG-162, or antisense, soluble RANKL receptor or muteins thereof), RANKL vaccine, osteoprotegrin (OPG), platelet-derived growth factors (PDGF), src kinase inhibitors, gallium maltolate, and matrix metalloproteinase (MMP) inhibitors.

Exemplary doses of bisphosphonates include the intravenous administration of 4mg. Lesser dosages may also be administered including 3.5 mg, 3.3 mg or 3.0 mg. Other routes of administration are possible including subcutaneous and as described in WO 02/ 087555. Effective amounts of a M- CSF antibody will vary and depend on the severity of the disease and the weight and general state of the patient being treated, but generally range from about 1.0 mg/kg to about 100 mg/kg body weight, or about 10 mg/kg to about 30 mg/kg, with dosages of from about 0.1 mg/kg to about 10 mg/kg or about 1 mg/kg to about 10 mg/kg per application being more commonly used. For example, about 10 11g/kg to 5 mg/kg or about 30 Mg/kg to 1 mg/kg of antibody is an initial candidate dosage for administration to the patient, whether, for example, by one or more separate administrations, or by continuous infusion. Administration is daily, on alternating days, weekly or less frequently, as necessary depending on the response to the disease and the patient's tolerance of the therapy. Maintenance dosages over a longer period of time, such as 4, 5, 6, 7, 8, 10 or 12 weeks or longer may be needed until a desired suppression of disease symptoms occurs, and dosages may be adjusted as necessary. The progress of this therapy is easily monitored by conventional techniques and assays.

Although the methods of the present invention may be useful for all stages of cancers, they may be particularly appropriate in advanced or metastatic cancers. Combining the therapy method with a chemotherapeutic or radiation regimen may be preferred in patients that have not received chemotherapeutic treatment, whereas treatment with the therapy method of the present invention may be indicated for patients who have received one or more chemotherapies. Additionally, the therapy methods of the present invention can also enable the use of reduced dosages of concomitant chemotherapy, particularly in patients that do not tolerate the toxicity of the chemotherapeutic agent very well.

The method of the invention contemplate the administration of single anti-M-CSF antibodies, as well as combinations, or "cocktails", of different antibodies. Such antibody cocktails may have certain advantages inasmuch as they contain antibodies which exploit different effector mechanisms or combine directly cytotoxic antibodies with antibodies that rely on immune effector functionality. Such antibodies in combination may exhibit synergistic therapeutic effects.

The methods of the invention can be used in combination with yet other therapeutics, such as cancer therapeutics. Exemplary cancer therapeutic agents and/or procedures, include but are not limited to various chemotherapeutic agents, androgen-blockers, and immune modulators (e.g., IL-2, GM-CSF, SLC), Bisphosphonate(s) (e.g., Aredia (i.e., pamidronate, pamidronic acid, disodium pamidronate, pamidronate disodium pentahydrate); Zometa (i.e., Aclasta, zoledronic acid, zoledronate); Clondronate (i.e., Bonefos, Loron, clodronate disodium, sodium clondronate); Fosamax (i.e.,alendronate, alendronate sodium salt trihydrate, alendronic acid); Fosavance (i.e., Fosamax formulated with vitamin D); Bondronat or Bonviva or Boniva (i.e., ibandronate, ibandronic acid, ibandronate sodium); Actonel (i.e., risedronate, risedronate sodium, risendronic acid); Didronel or Didrocal (i.e., etidronate, etidronic acid, etidronate disodium); Nerixia (i.e., neridronate, neridronic acid); Skelid (i.e., tiludronate, tiludronic acid); dimethyl-APD (i.e., olpadronate, olpadronic acid); and medronic acid or medronate), surgery, radiation, cytotoxic chemotherapy, hormone therapy (e.g., Tamoxifen; anti-Androgen therapy), antibody therapy (e.g., antibodies to RANKL/RANK neutralizing; PTHrP neutralizing, anti-Her2, anti-CD20, anti-CD40, CD22, VEGF, IGFR-1, EphA2, HAAH, TMEFF2, CAIX antibodies), therapeutic protein therapy (e.g., soluble RANKL receptor; OPG, and PDGF and MMP inhibitors), small molecule drug therapy (e.g., Src-kinase inhibitor), kinase inhibitors of growth factor receptors, or RANKL inhibitors, oligonucleotides therapy (e.g., RANKL or RANK or PTHrP Anti-sense), gene therapy (e.g., RANKL or RANK inhibitors, such as anti-RANK antibodies), peptide therapy (e.g. muteins of RANKL) as well as those proteins, peptides, compounds, and small molecules described herein..

Cancer chemotherapeutic agents include, without limitation, alkylating agents, such as carboplatin and cisplatin; nitrogen mustard alkylating agents; nitrosourea alkylating agents, such as carmustine (BCNU); antimetabolites, such as methotrexate; folinic acid; purine analog antimetabolites, mercaptopurine; pyrimidine analog antimetabolites, such as fluorouracil (5-FU) and gemcitabine (Gernzar®); hormonal antineoplastics, such as goserelin, leuprolide, and tamoxifen; natural antineoplastics, such as aldesleukin, interleukin-2, docetaxel, etoposide (VP-16), interferon alfa, paclitaxel (Taxol®), and tretinoin (ATRA); antibiotic natural antineoplastics, such as bleomycin, dactinomycin, daunorubicin, doxorubicin, daunomycin and mitomycins including mitomycin C; and vinca alkaloid natural antineoplastics, such as vinblastine, vincristine, vindesine; hydroxyurea; aceglatone, adriamycin, ifosfamide, enocitabine, epitiostanol, aclarubicin, ancitabine, nimustine, procarbazine hydrochloride, carboquone, carboplatin, carmofur, chromomycin A3, antitumor polysaccharides, antitumor platelet factors, cyclophosphamide (Cytoxin®), Schizophyllan, cytarabine (cytosine arabinoside), dacarbazine, thioinosine, thiotepa, tegafur, dolastatins, dolastatin analogs such as auristatin, CPT-11 (irinotecan), mitozantrone, vinorelbine, teniposide, aminopterin, carminomycin, esperamicins (See, e.g., U:S. Patent No. 4,675,187), neocarzinostatin, OK-432, bleomycin, furtulon, broxuridine, busulfan, honvan, peplomycin, bestatin (Ubenimex®), interferon- β, mepitiostane, mitobronitol, melphalan, laminin peptides, lentinan, Coriolus versicolor extract, tegafur/uracil, estramustine (estrogen/mechlorethamine).

Further; additional agents used as therapy for cancer patients include EPO, G-CSF, ganciclovir; antibiotics, leuprolide; meperidine; zidovudine (AZT); interleukins 1 through 18, including mutants and analogues; interferons or cytokines, such as interferons α, β, and γ hormones, such as luteinizing hormone releasing hormone (LHRH) and analogues and, gonadotropin releasing hormone (GnRH); growth factors, such as transforming growth factor- β (TGF-β), fibroblast growth factor (FGF), nerve growth factor (NGF), growth hormone releasing factor (GHRF), epidermal growth factor (EGF), fibroblast growth factor homologous factor (FGFHF), hepatocyte growth factor (HGF), and insulin growth factor (IGF); tumor necrosis factor- α & β (TNF- α & β); invasion inhibiting factor-2 (IIF-2); bone morphogenetic proteins 1-7 (BMP 1-7); somatostatin; thymosin- a -1; γ-globulin; superoxide dismutase (SOD); complement factors; anti-angiogenesis factors; antigenic materials; and pro-drugs.

Prodrug refers to a precursor or derivative form of a pharmaceutically active substance that is less cytotoxic or non-cytotoxic to tumor cells compared to the parent drug and is capable of being enzymatically activated or converted into an active or the more active parent form. *See, e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). Prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use herein include, but are not limited to, those chemotherapeutic agents described above.

### III. Administration and preparation

Effective amounts of a M-CSF antagonist will vary and depend on the seventy of the disease and the weight and general state of the patient being treated, but generally range from about 1.0 µg/kg to about 100 mg/kg body weight, with dosages of from about 10 µg/kg to about 10 mg/kg per application being more commonly used. Determination of an effective amount of a composition of the invention can be accomplished through standard empirical methods which are well known in the art. For example, the in vivo neutralizing activity of sera from a subject treated with a given dosage of M-CSF antagonist may be evaluated using an assay that determines the ability of the sera to block M-CSF induced proliferation and survival of murine monocytes (CD11b+ cell, a subset of CD11 cells, which expresses high levels of receptor to M-CSF) in vitro as described in Cenci et al., J Clin. Invest. 1055: 1279-87, 2000.

Administration is daily, every two days, every 3 days, twice weekly, weekly or less frequently, as necessary depending on the response to the disease and the patient's tolerance of the therapy. Maintenance dosages over a prolonged period of time may be needed, and dosages may be adjusted as necessary.

Single or multiple administrations of the compositions can be carried out with the dose levels and pattern being selected by the treating physician.

The M-CSF antagonists, including anti-M-CSF antibodies used in the practice of a method of the invention may be formulated into pharmaceutical compositions comprising a carrier suitable for the desired delivery method. Suitable carriers include any material which, when combined with the M-CSF antagonist, retains the anti-tumor function of the antagonist and is nonreactive with the subject's immune systems. Examples include, but are not limited to, any of a number of standard pharmaceutical carriers such as sterile phosphate buffered saline solutions, bacteriostatic water, and the like. A variety of aqueous carriers may be used, e.g., water, buffered water, 0.4% saline, 0.3% glycine and the like, and may include other proteins for enhanced stability, such as albumin, lipoprotein, globulin, etc., subjected to mild chemical modifications or the like.

Therapeutic formulations of the antagonist are prepared for storage by mixing the antagonist having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The formulation herein may also contain more than one active compound as necessary for the particular indication being treated, preferably those with complementary activities that do not adversely affect each other. For example, it may be desirable to further provide an immunosuppressive agent. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

The antagonist is administered by any suitable means, including parenteral, subcutaneous, intraperitoneal, intrapulmonary, and intranasal, and, if desired for local treatment, intralesional administration. Parenteral infusions include intravenous, intraarterial, intraperitoneal, intramuscular, intradermal or subcutaneous administration. In addition, the antagonist is suitably administered by pulse infusion, particularly with declining doses of the antagonist. Preferably the dosing is given by injections, most preferably intravenous or subcutaneous injections, depending in part on whether the administration is brief or chronic. Other administration methods are contemplated, including topical, particularly transdermal, transmucosal, rectal, oral or local administration e.g. through a catheter placed close to the desired site.

Compositions of the present invention can be in the form of, for example, granules, powders, tablets, capsules, syrup, suppositories, injections, emulsions, elixirs, suspensions or solutions. The instant compositions can be formulated for various routes of administration, for example, by oral administration, by nasal administration, by rectal administration, subcutaneous injection, intravenous injection, intramuscular injections, or intraperitoneal injection.

Injectable dosage forms generally include aqueous suspensions or oil suspensions which may be prepared using a suitable dispersant or wetting agent and a suspending agent. Injectable forms may be in solution phase or in the form of a suspension, which is prepared with a solvent or diluent. Acceptable solvents or vehicles include sterilized water, Ringer's solution, or an isotonic aqueous saline solution. Alternatively, sterile oils maybe employed as solvents or suspending agents. Preferably, the oil or fatty acid is nonvolatile, including natural or synthetic oils, fatty acids, mono-, di- or tri-glycerides.

For injection, the pharmaceutical formulation and/or medicament may be a powder suitable for reconstitution with an appropriate solution as described above. Examples of these include, but are not limited to, freeze dried, rotary dried or spray dried powders, amorphous powders, granules, precipitates, or particulates. For injection, the formulations may optionally contain stabilizers, pH modifiers, surfactants, bioavailability modifiers and combinations of these.

Sustained-release preparations may be prepared. Suitable examples of sustained-release preparations include semipermeable matrices of solid hydrophobic polymers containing the antagonist, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and y ethyl-L-glutamate, non-degradable ethylene-vinyl acetate, degradable lactic acid-glycolic acid copolymers such as the Lupron Depot™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antagonists remain in the body for a long time, they may denature or aggregate as a result of exposure to moisture at 37°C., resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

The formulations of the invention may be designed to be short-acting, fast-releasing, long-acting, or sustained-releasing as described herein. Thus, the pharmaceutical formulations may also be formulated for controlled release or for slow release.

The instant compositions may also comprise, for example, micelles or liposomes, or some other encapsulated form, or may be administered in an extended release form to provide a prolonged storage and/or delivery effect. Therefore, the pharmaceutical formulations and medicaments may be compressed into pellets or cylinders and implanted intramuscularly or subcutaneously as depot injections or as implants such as stents. Such implants may employ known inert materials such as silicones and biodegradable polymers.

Besides those representative dosage forms described above, pharmaceutically acceptable excipients and carries are generally known to those skilled in the art and are thus included in the instant invention. Such excipients and carriers are described, for example, in "Remingtons Pharmaceutical Sciences" Mack Pub. Co., New Jersey (1991), which is incorporated herein by reference.

Specific dosages may be adjusted depending on conditions of disease, the age, body weight, general health conditions, sex, and diet of the subject, dose intervals, administration routes, excretion rate, and combinations of drugs. Any of the above dosage forms containing effective amounts are well within the bounds of routine experimentation and therefore, well within the scope of the instant invention.

M-CSF antagonists or antibodies useful as therapeutics according to the invention will often be prepared substantially free of other naturally occurring immunoglobulins or other biological molecules. Preferred M-CSF antagonists will also exhibit minimal toxicity when administered to a mammal afflicted with, or predisposed to suffer from, osteolytic disorders, including cancer metastasis and/or bone loss associated with cancer metastasis.

The compositions of the invention may be sterilized by conventional, well known sterilization techniques. The resulting solutions may be packaged for use or filtered under aseptic conditions and lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride and stabilizers (e.g., 1 20% maltose, etc.).

The M-CSF antagonists of the present invention may also be administered via liposomes, which are small vesicles composed of various types of lipids and/or phospholipids and/or surfactant which are useful for delivery of a drug (such as the antagonists disclosed herein and, optionally, a chemotherapeutic agent). Liposomes include emulsions, foams, micelles, insoluble monolayers, phospholipid dispersions, lamellar layers and the like, and can serve as vehicles to target the M-CSF antagonists to a particular tissue as well as to increase the half life of the composition. A variety of methods are available for preparing liposomes, as described in, e.g., U.S. Patent Nos. 4,837,028 and 5,019,369, which patents are incorporated herein by reference.

Liposomes containing the antagonist are prepared by methods known in the art, such as described in Epstein et al., Proc. Natal. Acad. Sci. USA 82: 3688 (1985); Hwang et al., Proc. Natl Acad. Sci. USA 77: 4030 (1980); and U.S. Patent Nos. 4,485,045 and 4,544,545. Liposomes with enhanced circulation time are disclosed in U.S. Patent No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the M-CSF antibody of the present invention can be conjugated to the liposomes as described in Martin et al., J. Biol. Chem. 257: 286-288 (1982) via a disulfide interchange reaction. A chemotherapeutic agent (such as Doxorubicin) is optionally contained within the liposome [*see, e.g.,* Gabizon et al.,J. National Cancer Inst. 81(19): 1484 (1989)].

The concentration of the M-CSF antagonist in these compositions can vary widely, i.e., from less than about 10%, usually at least about 25% to as much as 75% or 90% by weight and will be selected primarily by fluid volumes, viscosities, etc., in accordance with the particular mode of administration selected. Actual methods for preparing orally, topically and parenterally administrable compositions will be known or apparent to those skilled in the art and are described in detail in, for example, Remington's Pharmaceutical Science, 19th ed., Mack Publishing Co., Easton, PA (1995), which is incorporated herein by reference.

Determination of an effective amount of a composition of the invention can be accomplished through standard empirical methods which are well known in the art. For example, the in vivo neutralizing activity of sera from a subject treated with a given dosage of M-CSF antagonist may be evaluated using an assay that determines the ability of the sera to block M-CSF induced proliferation and survival of murine monocytes (CD11b+cell, a subset of CD11 cells, which expresses high levels of receptor to M-CSF) in vitro as described in Cenci et al., J Clin. Invest. 1055: 1279-87, 2000.

Compositions of the invention are administered to a mammal already suffering. from, or predisposed to, osteolytic disorder, including cancer metastasis and/or bone loss associated with cancer metastasis in an amount sufficient to prevent or at least partially arrest the development of such disease. Effective amounts of a M-CSF antagonist will vary and depend on the severity of the disease and the weight and general state of the patient being treated, but generally range from about 1.0 mg/kg to about 100 mg/kg body weight, or about 10 mg/kg to about 90 mg/kg, with dosages of from about 20 mg/kg to about 80 mg/kg or about 30 mg/kg to about 70 mg/kg or about 40 mg/kg to about 60mg/kg per application. For example, about 10 mg/kg to 50 mg/kg or about 20 mg/kg to 60 mg/kg of anti-MCSF antibody is an initial candidate dosage for administration to the patient, whether, for example, by one or more separate administrations, or by continuous infusion. Administration is daily, on alternating days, weekly or less frequently, as necessary depending on the response to the disease and the patient's tolerance of the therapy. Maintenance dosages over a longer period of time, such as 4, 5, 6, 7, 8, 10 or 12 weeks or longer may be needed until a desired suppression of disease symptoms occurs, and dosages may be adjusted as necessary. The progress of this therapy is easily monitored by conventional techniques and assays.

Single or multiple administrations of the compositions can be carried out with the dose levels and pattern being selected by the treating physician. For the prevention or treatment of disease, the appropriate dosage of M-CSF antagonist, including anti-M-CSF antibody will depend on the type of disease to be treated, as defined above, the severity and course of the disease, whether the antagonist is administered for preventive or therapeutic purposes, previous therapy, the patient's clinical history and response to the antagonist, and the discretion of the attending physician. The antagonist is suitably administered to the patient at one time or over a series of treatments.

The antagonist composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The therapeutically effective amount of the antagonist to be administered will be governed by such considerations, and is the minimum amount necessary to prevent, ameliorate, or treat the M-CSF mediated disease, condition or disorder, particularly to treat cancer cells, and most particularly to treat tumor cell metastases. Such amount is preferably below the amount that is toxic to the host or renders the host significantly more susceptible to infections.

In another embodiment of the invention, an article of manufacture containing materials useful for the treatment of the diseases, disorders or conditions described above is provided. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agent in the composition is the M-CSF antagonist or antibody of the invention. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further other materials desirable from a commercial and user standpoint, including other buffers, diluents, filters, needles, syringes, and package inserts with instructions for use.

The invention is illustrated by the following examples, which are not intended to be limiting in any way.

### EXAMPLES

### EXAMPLE 1

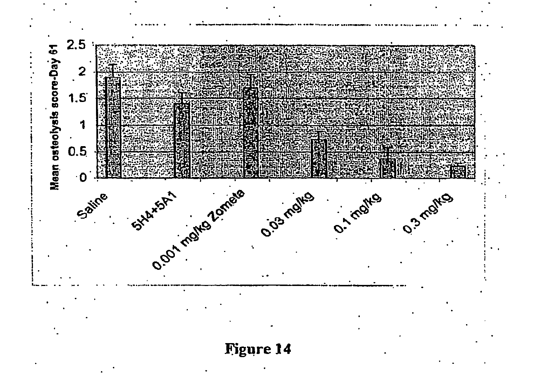

This Example establishes the dose-dependent, anti-resorptive effects of Zometa (zoledronate) in an animal model (Figure 14). Treatment with ≥ 0.03 mg/kg Zometa inhibited the osteolytic damage caused by tumor growth at the bony site. In addition, a dose response effect was observed when mice were treated with increasing concentrations of Zometa. Anti-mouse and anti-human M-CSF mAbs 5A1 and 5H4 combined also protected against bone damage. M-CSF antibody alone was more effective than 0.03 mg/kg Zometa in treating severe osteolytic damage. Osteolysis score based on x-ray image in last-day of study (Figure 14):
0= normal;
1= Equivocal or minimal lesion with normal cortex architecture;
2= Definite lytic lesion with minimal cortex/architecture disruption
3= Large lesion(s) with cortex/Architecture disruption
4= Gross destruction with no preserved architecture

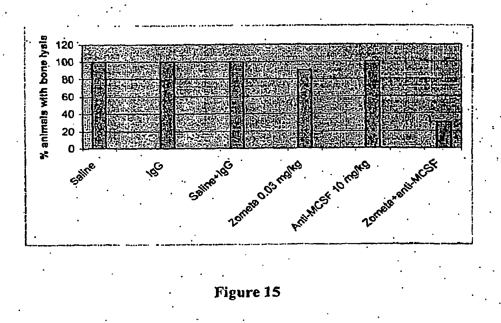

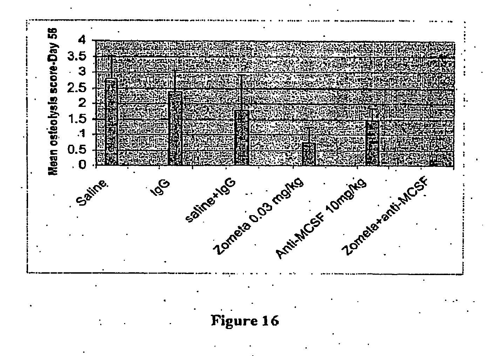

From this initial study, a combination study was performed using a sub-efficacious dose of 0.03 mg/kg Zometa. Two weeks after intratibial inoculation of 6x1.0⁵ MDA-MB-231 Luc cells, Nu/Nu mice were treated with 5A1/5H4 (10 mg/kg once weekly), Zometa (0.03 mg/kg twice weekly) or both antibody and bisphosphonate. Bone lesions were monitored weekly by Faxitron analysis (x-ray technology), and at the end of the study, all animals were subjected to a final x-ray, and the images collected and distributed for scoring of the severity of lesions. Analysis of the results showed that both anti-MCSF mAb and Zometa were effective in treating osteolysis, but that the combined treatment of Zometa and the M-CSF mAb inhibited incidence (Figure 15) and extent (Figure 16) of bone lysis to a greater extent than either treatment alone.

Figure 16 shows that treatment with 0.03 mg/kg Zometa or 10 mg/kg anti-MCSF antibody (5A1+5H4) inhibited the osteolytic damage caused by tumor growth at the bony site. A combination regimen of Zometa plus anti-MCSF antibody further inhibited bone lysis. Mean osteolysis scores were calculated from 1) an average of scores from 3 separate volunteer scorers and 2) a group average (originally 10 animals/group).

Osteolysis score based on x-ray image in last day of study:
0= normal;
1= Equivocal or minimal lesion with normal cortex architecture;
2= Definite lytic lesion with minimal cortex/architecture disruption
3= Large lesion(s) with cortex/Architecture disruption
4= Gross destruction with no preserved architecture

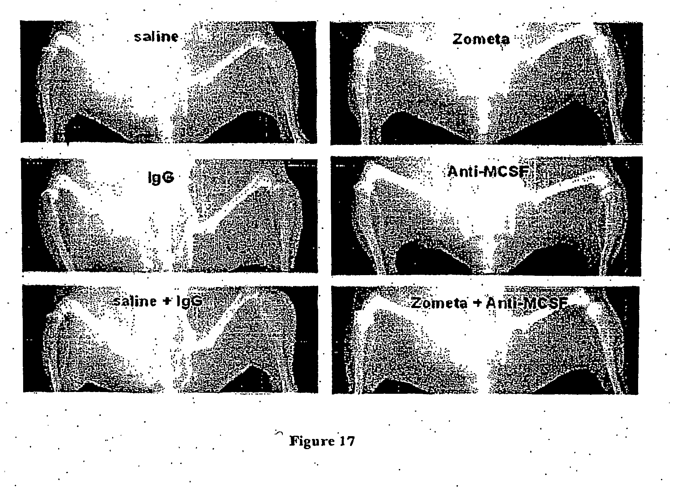

Examination of representative faxitron images further demonstrated the severity of lesions found in untreated animals compared to the relatively minor lesions in Zometa and anti-MCSF antibody-treated animals (Figure 17). There were no adverse interactions observed in the combination treatment group.

In conclusion, both anti-MCSF antibody and Zometa effectively inhibit osteolysis, and combining the two treatments results in an increased anti-resorptive effect compared with either treatment alone. This suggests that the combination may be a safe and effective option for patients with bone disease who are either bisphosphonate-intolerant, or who are already being treated with bisphosphonates.

### EXAMPLE 2

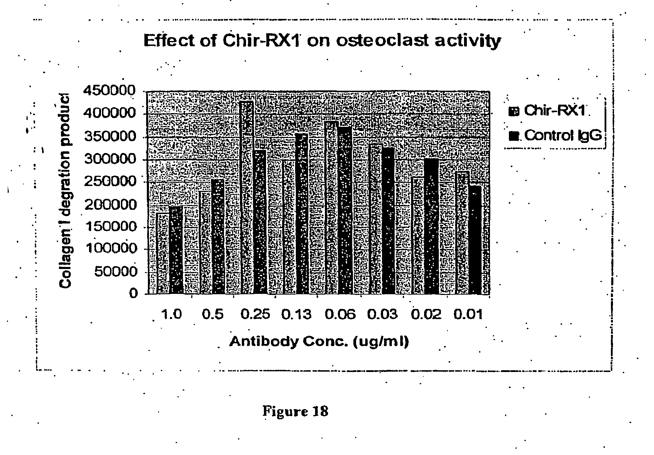

This Example shows that inhibition of M-CSf activity has no effect on differentiated osteoclasts activity (Figure 18). The effect of M-CSF-neutralizing antibodies and bisphosphonate on differentiated osteoclast activity was tested with humanized Chir-RX1 and Zometa.

The human bone marrow CD34+ cells (Biowhittaker catalog number 2M-101 A, 3x 1.0⁵ cells/ vial) were induced to differentiate into osteoclasts under the experimental conditions described here. On Day 1, CD34+ cells were thawed from one frozen vial into 10 ml of media (Alpha MEM with 10% FCS, 1 x Pen Strep and 1x fungizone). The cells were washed once and re-suspended in 2 ml of media and plate into onto the OsteoLyse plate (OsteoLyse™ Assay Kit (Human Collagen), Cambrex) at 100 ul per well.

On day 2, without removing the original media, add to each well 50 ul of 4x CSF-1 to 30 ng/ml final concentration and 50 ul of 4x RANKL (sRANKL, Chemicon catalog # GF091, 10 ug/package) to final concentration of 100 ng/ml. On day 7, add to each well 50 ul of 5x RANKL to final concentration of 100 ng/ml.

On day 15, antibodies (either Chir-RX1 or control antibody) or Zometa were added at the indicated concentrations. On day 17,10 ul of supernatant of the cell culture was sampled and mixed with 200 µl of Fluorophore Releasing Reagent in each well of the black 96-well assay plate (included in the OsteoLyse Assay Kit).

### EXAMPLE 3

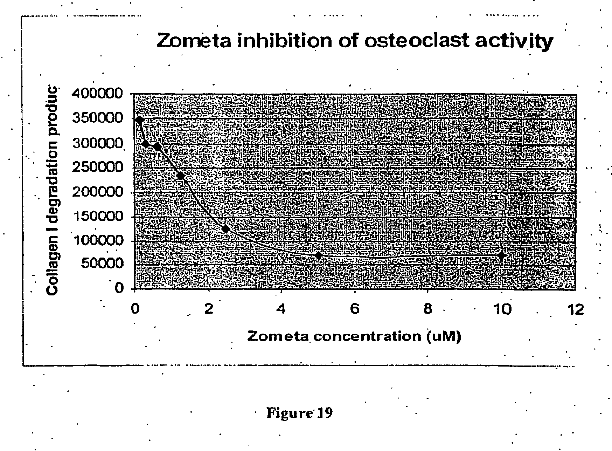

This Example shows that Zometa inhibits differentiated osteoclast activity in a dose-dependent manner (Figure 19). The effect of M-CSF-neutralizing antibodies and bisphosphonate on differentiated osteoclast activity was tested with humanized Chir-RX1 and Zometa.

The human bone marrow CD34+ cells (Biowhittaker catalog number 2M-101A, 3x 10⁵ cells / vial) were induced to differentiate into osteoclasts under the experimental conditions described here. On Day 1, CD34+ cells were thawed from one frozen vial into 10 ml of media (Alpha MEM with 10% FCS, 1 x Pen Strep and 1x fungizone). The cells were washed once and re-suspended in 2 ml of media and plate into onto the OsteoLyse plate (4steoLyse™ Assay Kit (Human Collagen), Cambrex) at 100 ul per well.

On day 2, without removing the original media, add to each well 50 ul of 4x CSF-1 to 30 ng/ml final concentration and 50 ul of 4x RANKL (sRANKL, Chemicon catalog # GF091, 10 ug/package) to final concentration of 100 ng/ml. On day 7, add to each well 50 ul of 5x RANKL to final concentration of 100 ng/ml.

On day 15, antibodies (either Chir-RX1 or control antibody) or Zometa were added at the indicated concentrations. On day 17,10 ul of supernatant of the cell culture was sampled and mixed with 200 µl of Fluorophore Releasing Reagent in each well of the black 96-well assay plate (included in the OsteoLyse Assay Kit).

### EXAMPLE 4

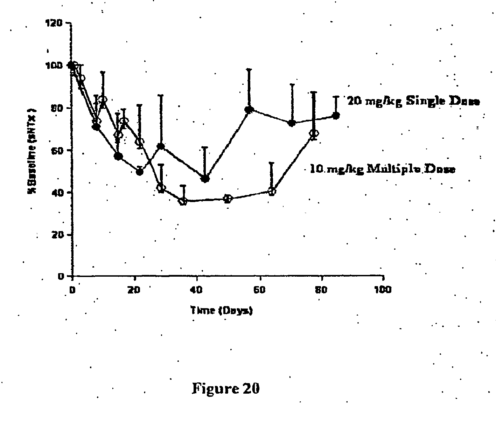

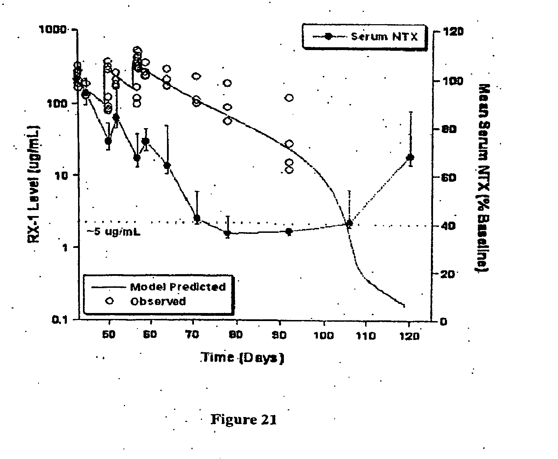

This Example shows the results of a pharmacokinetic and pharmacodynamic study using RX1 in primates (Figure 20 and Figure 21).

The purpose of this study was to investigate the pharmacodynamics and pharmacokinetics of heRX1-10.G1, a humanized anti-human M CSF antibody, when administered to cynomolgus monkeys either as a single slow bolus intravenous injection (Groups 2 and 3 on Day 1) followed by a 13-week observation period or as repeated doses (Group 1 on Days 1, 43, 50, and 57) followed by a 10-week observation period. Humanized anti-M-CSF IgG1 monoclonal antibody was adminsitered via slow bolus intravenous (IV) injection via a brachial or saphenous vein.

The use of animal is required by worldwide regulatory agencies for safety assessment of new drugs. The antibody is not cross-reactive in rodent species but has been shown to be active in cynomolgus monkeys. Therefore, the cynomolgus monkey was selected since it is an accepted non-rodent species for use in intravenous injection studies with biologics.

Animals were randomized into the following groups:

| Group No. | Dose Level | Dose Volume | No. of Animals | |
|---|---|---|---|---|
| Identification | (mg/kg) | (mL/kg) | Males | Females |
| 1 heRX1-10.G1 | 0.2/10^{a} | 4 | 2 | 2 |
| 2 heRX1-10.G1 | 2 | 4 | 2 | 2 |
| 3 heRX1-10.G1 | 20 | 4 | 2 | 2 |

| | | | | |
|---|---|---|---|---|
| ^{a} On Day 1, Group 1 will receive a 0.2 mg/kg/dose, and on Days 43, 50, and 57, the same animals will receive a 10 mg/kg/dose. | | | | |

Groups 2 and 3 were administered the test article formulation (2 and 20 mg/kg/dose, respectively) on Day 1 by intravenous slow bolus injection over an approximate 10-minute period. Formulations were administered via a saphenous vein using a catheter and an abbocath. The dose volume was 4 mL/kg and the actual dose was based on the most recent practical body weight of each animal. Group 1 was administered a dose of 0.2 mg/kg on Day 1 and subsequent doses of 10 mg/kg/dose on Days 43, 50 and 57. The formulation was administered by intravenous slow bolus injection (over an approximate 10 minute period) via a saphenous vein using a catheter and an abbocath. The dose volume ways 4 mL/kg and the actual dose was based on the most recent practical body weight of each animal.

Blood was collected from all animals for hematology and/or clinical biochemistry, as follows:

| **Occasion** | **Hematology** | | | **Biochemistry** | | |
|---|---|---|---|---|---|---|
| | **Group 1** | **Group 2** | **Group 3** | **Group 1** | **Group 2** | **Group 3** |
| Day -14^{a} | 0.5 mL | 0.5 mL | 0.5 mL | 1 mL | 1 mL | 1 mL |
| Day -7 | 0.5 mL | 0.5 mL | 0.5 mL | | | |
| Day 3^{a} | 0.5 mL | 0.5 mL | 0.5 mL | | | |
| Day 8 | 0.5 mL | 0.5 mL | 0.5 mL | | | |
| Day 15 | 0.5 mL | 0.5 mL | 0.5 mL | 1 mL | 1 mL | 1 mL |
| Day 22 | 0.5 mL | 0.5 mL | 0.5 mL | | | |
| Day 29 | 0.5 mL | 0.5 mL | 0.5 mL | 1 mL | 1 mL | 1 mL |
| Day 43 | 0.5 mL | 0.5 mL | 0.5 mL | 1 mL | 1 mL | 1 mL |
| Day 50 | 0.5 mL | 0.5 mL | 0.5 mL | 1 mL | | |
| Day 57 | 0.5 mL | 0.5 mL | 0.5 mL | | 1 mL | 1 mL |
| Day 64 | 0.5 mL | 0.5 mL | 0.5 mL | 1 mL | | |
| Day 71 | 0.5 mL | 0.5 mL | 0.5 mL | | 1 mL | 1 mL |
| Day 78 | 0.5 mL | 0.5 mL | 0.5 mL | 1 mL | | |
| Day 85 | | 0.5 mL | 0.5 mL | | 1 mL | 1 mL |
| Day 92 | 0.5 mL | | | 1 mL | | |
| Day 106 | 0.5 mL | | | 1 mL | | |
| Day 120 | 0.5 mL | | | 1 mL | | |
| ^{a} Only WBC count (total and differential) required to correlate with lymphocyte phenotyping | | | | | | |

The following parameters were examined:
Hematology: blood cell morphology; erythrocyte indices (MCV, MCH, MCHC and RDW); hematocrit; hemoglobin; mean platelet volume; platelet count; red blood cell count; reticulocytes (absolute and percent); and white blood cell count (total, absolute and percent differential).
Clinical biochemistry: A/G ratio (calculated); alanine aminotransferase; albumin; alkaline phosphatase; aspartate aminotransferase; blood urea nitrogen; calcium; chloride; cholesterol; creatinine; globulin (calculated); glucose; inorganic phosphorus; potassium; sodium; total, direct and indirect bilirubin; total protein; triglycerides; and C-reactive protein.

Biochemical markers of bone turnover were analyzed as follows (approximately 2 mL of blood was collected from all animals for determination of bone biomarkers):

| **Occasion** | **Markers of Bone Formation (BAP and Calcium** ^{#)} | | | **Markers of Bone Resorption (NTx and CTx)** | | |
|---|---|---|---|---|---|---|
| | **Group 1** | **Group 2** | **Group 3** | **Group 1** | **Group 2** | **Group 3** |
| Day-14 | 1 mL | 1 mL | 1 mL | 1 mL | 1 mL | 1 mL |
| Day -7 | 1 mL | 1 mL | 1 mL | 1 mL | 1 mL | 1 mL |
| Day 8 | | | | 1 mL | 1 mL | 1 mL |
| Day 15 | | | | 1 mL | mL | 1 mL |
| Day 22 | | | | 1 mL | 1 mL | 1 mL |
| Day 29 | 1 mL | 1 mL | 1 mL | 1 mL | 1 mL | 1 mL |
| Day 43 | | | | 1 mL | 1 mL | 1 mL |
| Day 45 | | | | 1 mL | | |
| Day 50 | 1 mL | | | 1 mL | | |
| Day 52 | | | | 1 mL | | |
| Day 57 | | 1 mL | 1 mL | 1 mL | 1 mL | 1 mL |
| Day 59 | | | | 1 mL | | |
| Day 64 | 1 mL | | | 1 mL | | |
| Day 71 | | | | 1 mL | 1 mL | 1 mL |
| Day 78 | | | | 1 mL | | |
| Day 85 | | 1 mL | 1 mL | | 1 mL | 1 mL |
| Day 92 | 1 mL | | | 1 mL | | |
| Day 106 | 1 mL | | | 1 mL | | |
| Day 120 | 1 mL | | | 1 mL | | |

| | | | | | | |
|---|---|---|---|---|---|---|
| (NTX: N-terminal cross-linking telopepide of bone collagen) (CTX: C-terminal cross-linking telopepide of bone collagen) | | | | | | |

### Pharmacokinetic evaluation and serum M-CSF activity:

For Groups 2 and 3, blood (1.5 mL each) was collected by venipuncture into SST tubes pre-dose, Day 1 (immediately after the end of the infusion and 4 hours after the end of the infusion), and Days 3, 8, 15, 22, 29, 43, 57, 71, and 85. For Group I animals, blood (1.5 mL each) was collected by venipuncture into SST tubes pre-dose, Day 1 (immediately after the end of the infusion and 4 hours after the end of the infusion), and Days 3, 8, 15, 22, 29, 43 (predose and 4 hours after the end of the infusion), 50 (predose and 4 hours after the end of the infusion), 57 (predose and 4 hours after the end of the infusion), 59, 64, 71, 78, 92, 106 and 120. Samples were analyzed for pharmacokinetic evaluation and for serum M-CSF activity and remaining heRX1-10-G1 activity.

Blood samples were allowed to clot at room temperature for approximately 30 minutes prior to centrifugation. The serum was obtained by centrifugation at approximately 2700 rpm for 10 minutes at approximately 4°C and the resultant serum were divided into 4 aliquots.

All of the above U.S. patents, U.S. patent application publications, U.S. patent applications, foreign patents, foreign patent applications and non patent publications referred to in this specification and/or listed in the Application Data Sheet, are incorporated herein by reference, in their entirety.

From the foregoing it will be appreciated that, although specific embodiments of the invention have been described herein for purposes of illustration, various modifications may be made without deviating from the spirit and scope of the invention.

### EMBODIMENTS OF THE INVENTION:

1. A method of treating a patient suffering from an osteolytic disorder comprising the steps of administering to said patient an anti-M-CSF antibody at a monotherapeutically effective dose and an osteoclast inhibitor at a monotherapeutically effective dose.
2. A method of treating a patient suffering from an osteolytic disorder comprising the steps of administering to said patient an anti-M-CSF antibody at a monotherapeutically effective dose and a bisphosphonate at a monotherapeutically effective dose.
3. The method of embodiment 1 wherein the osteoclast inhibitor is a RANKL inhibitor.
4. The method of embodiment 2 wherein the anti-MCSF antibody is RX1-derived antibody, an MC1-derived antibody, an MC3 derived antibody, or 5H4-derived antibody.
5. The method of embodiment 2 wherein the anti-MCSF antibody is heRX1.
6. The method of any of the preceding embodiments wherein the osteolytic disorder is osteoporosis, bone loss associated with cancer metastasis, Padget's disease, or periprosthetic bone loss.
7. The method of any of the preceding embodiments wherein the osteoclast inhibitor and the MCSF antibody are administered simultaneously
8. The method of embodiment wherein the RANKL inhibitor is selected from the group consisting of an anti-RANKL antibody, a soluble RANKL receptor, and a RANKL vaccine.
9. The method of embodiments 3, 4, 6, and 7 wherein the anti-RANKL antibody is AMG-162.
10. The method of embodiments 2, 4, 6 and 7 wherein the bisphosphonate is selected from the group consisting of is zoledronate, pamidronate, clodronate, etidronate, tiludronate, alendronate, ibandronate, and risedronate.
11. The method of embodiment 10 wherein the bisphosphonate is zoledronate.
12. A method of treating a patient suffering from an osteolytic disorder comprising the steps of administering to said patient an anti-M-CSF antibody at a monotherapeutically effective dose and a bisphosphonate at a monotherapeutically effective dose for a transition period.
13. The method of embodiment 12 wherein the osteoclast inhibitor is a bisphosphonate or a RANKL inhibitor.
14. The method of embodiment 13 wherein the transition period is approximately 1-7 days.
15. The method of embodiment 13 wherein the transition period is 1 week to 1 month.
16. The method of embodiment 13 wherein the transition period is 1 month to 3 months.
17. The method of embodiment 13 wherein the transition period is 3 to 6 months.
18. The method of embodiment 13 wherein the transition period is 6 to 12 months.
19. The method of embodiment 13 further comprising the step of discontinuing the bisphosphonate therapy after the transition period.
20. The method of embodiment 13 further comprising the step of reducing the dose of bisphosphonate after the transition period.
21. The method of embodiment 13 wherein the dose of bisphosphonate is reduced immediately after the transition period.
22. The method of embodiment 13 further comprising the step of reducing the dose of anti-MCSF antibody after the transition period.
24. The method of embodiment 13 wherein the dose of anti-MCSF antibody is reduced immediately after the transition period.
25. The method of embodiment 13 wherein the bisphosphonate is administered at least one time after the transition period.
26. The method of any of the preceding embodiments wherein the anti-MCSF antibody comprises at least two of the CDRs from murine RX1 antibody of SEQ ID NO: 5 and SEQ ID NO: 6.
27. The method of any of the preceding embodiments wherein the anti-MCSF antibody comprises at least three of the CDRs from murine RX1 antibody of SEQ ID NO: 5 and SEQ ID NO: 6.
28. The method of any of the preceding embodiments wherein the anti-MCSF antibody competes with murine RX 1 antibody for binding to M-CSF of SEQ ID NO: 5 and SEQ ID NO: 6 by at least 75%.
29. A method of embodiments 6, 13, and 20, wherein the osteoclast inhibitor is zoledronate and the MCSF antibody is an RX1-derived antibody.
30. The method of embodiment 29 wherein the zoledrorate is administered at a dose of between 0.5 mg and 4mg.
31. The method according to embodiments 4, 6, 8, 10, 11, or 12 wherein the anti-MCSF antibody binds to the same epitope as RX1, MC1, MC3, or 5H4 antibodies.

## Claims

1. An anti-M-CSF antibody and an osteoclast inhibitor for use in a method of treating a patient suffering from an osteolytic disorder comprising the steps of administering to said patient the anti-M-CSF antibody at a monotherapeutically effective dose and the osteoclast inhibitor at a monotherapeutically effective dose.

2. An anti-M-CSF antibody and a bisphosphonate for use in a method of treating a patient suffering from an osteolytic disorder comprising the steps of administering to said patient the anti-M-CSF antibody at a monotherapeutically effective dose and the bisphosphonate at a monotherapeutically effective dose.

3. The anti-M-CSF antibody and osteoclast inhibitor of claim 1 wherein the osteoclast inhibitor is a RANKL inhibitor.

4. The anti-M-CSF antibody and bisphosphonate of claim 2 wherein the anti-MCSF antibody is RX1-derived antibody, an MC1-derived antibody, an MC3-derived antibody, or 5H4-derived antibody.

5. The anti-M-CSF antibody and osteoclast inhibitor of claim 1 or claim 3; or the anti-M-CSF antibody and bisphosphonate of claim 2 or claim 4 wherein the osteolytic disorder is osteoporosis, bone loss associated with cancer metastasis, Padget's disease, or periprosthetic bone loss.

6. The anti-M-CSF antibody and osteoclast inhibitor of any one of claims 1, 3 or 5, wherein the osteoclast inhibitor and the MCSF antibody are administered simultaneously.

7. The anti-M-CSF antibody and osteoclast inhibitor of claim 3 wherein the RANKL inhibitor is selected from the group consisting of an anti-RANKL antibody, a soluble RANKL receptor, and a RANKL vaccine.

8. The anti-M-CSF antibody and osteoclast inhibitorof claim 7 wherein the anti-RANKL antibody is AMG-162.

9. The anti-M-CSF antibody and a bisphosphonate of any one of claims 2, 4, or 5 wherein the bisphosphonate is selected from the group consisting of is zoledronate, pamidronate, clodronate, etidronate, tiludronate, alendronate, ibandronate, and risedronate.

10. The anti-M-CSF antibody and a bisphosphonate of claim 9 wherein the bisphosphonate is zoledronate.

11. An anti-M-CSF antibody and an osteoclast inhibitor for use in a method of treating a patient suffering from an osteolytic disorder comprising the steps of administering to said patient the anti-M-CSF antibody at a monotherapeutically effective dose and the osteoclast inhibitor at a monotherapeutically effective dose for a transition period.

12. The anti-M-CSF antibody and an osteoclast inhibitor of claim 11 wherein the osteoclast inhibitor is a bisphosphonate or a RANKL inhibitor.

13. The anti-M-CSF antibody and an osteoclast inhibitor of claim 12 wherein the transition period is approximately 1 -7 days; 1 week to 1 month; 1 month to 3 months; 3 to 6 months; or 6 to 12 months.

14. The anti-M-CSF antibody and osteoclast inhibitor of claim 5 or claim 12, wherein the osteoclast inhibitor is zoledronate and the MCSF antibody is an RX1 -derived antibody.

15. The anti-M-CSF antibody and osteoclast inhibitor of claim 14 wherein the zoledrorate is administered at a dose of between 0.5 mg and 4mg.

16. The anti-M-CSF antibody and osteoclast inhibitor of any one of claims 1, 3, 5-8 or 11-15, or the anti-MCSF antibody and bisphosphonate of any one of claims 2, 4, 5, 9 or 10, wherein the anti-M-CSF antibody comprises all three CDRs of SEQ ID NO: 43 and all three CDRs of SEQ ID NO: 53.
